(19) Europäisches Patentamt
European Patent Office
Office européen des brevets

(11) EP 4 371 980 A1

(12) EUROPEAN PATENT APPLICATION
published in accordance with Art. 153(4) EPC

(43) Date of publication:
**22.05.2024 Bulletin 2024/21**

(21) Application number: **22841471.0**

(22) Date of filing: **14.07.2022**

(51) International Patent Classification (IPC):
***C07D 401/04** (2006.01)* ***A61K 31/444** (2006.01)*
***A61P 25/00** (2006.01)*

(52) Cooperative Patent Classification (CPC):
**A61K 31/444; A61P 25/00; C07D 401/04**

(86) International application number:
**PCT/CN2022/105793**

(87) International publication number:
**WO 2023/284838 (19.01.2023 Gazette 2023/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.07.2021 CN 202110801701**
**22.09.2021 CN 202111105950**
**10.11.2021 CN 202111324545**
**03.12.2021 CN 202111465224**

(71) Applicant: **Xizang Haisco Pharmaceutical Co., Ltd.**
**Lhoka, Tibet 856099 (CN)**

(72) Inventors:
- **LI, Yao**
**Chengdu, Sichuan 611130 (CN)**
- **WANG, Wenjing**
**Chengdu, Sichuan 611130 (CN)**
- **SHI, Zongjun**
**Chengdu, Sichuan 611130 (CN)**
- **ZHANG, Haoliang**
**Chengdu, Sichuan 611130 (CN)**
- **DU, Chenglong**
**Chengdu, Sichuan 611130 (CN)**
- **CHENG, Fengkai**
**Chengdu, Sichuan 611130 (CN)**
- **LIU, Xin**
**Chengdu, Sichuan 611130 (CN)**
- **ZHANG, Xiaozhuan**
**Chengdu, Sichuan 611130 (CN)**
- **WANG, Long**
**Chengdu, Sichuan 611130 (CN)**
- **TANG, Pingming**
**Chengdu, Sichuan 611130 (CN)**
- **YU, Yan**
**Chengdu, Sichuan 611130 (CN)**
- **ZHANG, Chen**
**Chengdu, Sichuan 611130 (CN)**
- **YAN, Pangke**
**Chengdu, Sichuan 611130 (CN)**

(74) Representative: **Hoffmann Eitle**
**Patent- und Rechtsanwälte PartmbB**
**Arabellastraße 30**
**81925 München (DE)**

# (54) AAK1 INHIBITOR AND USE THEREOF

(57) A compound of formula (I) and a stereoisomer, deuterated compound, solvate, prodrug, metabolite, pharmaceutically acceptable salt or co-crystal thereof, or a pharmaceutical composition containing same, and the use thereof as an AAK1 inhibitor in the preparation of a drug for treating related diseases.

EP 4 371 980 A1

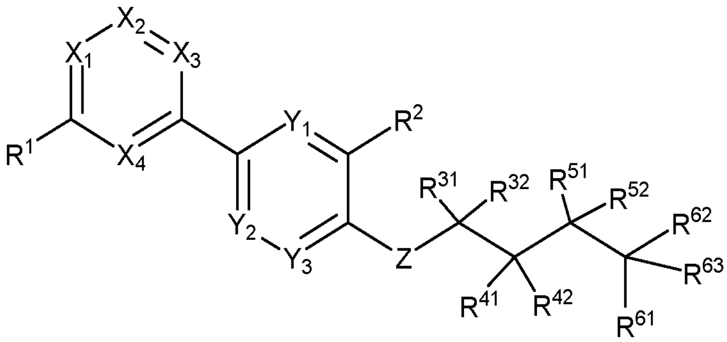

(I)

## Description

### Technical Field

The present invention belongs to the field of drugs, and in particular relates to a derivative of a protein arginine methyltransferase inhibitor, or a stereoisomer, pharmaceutically acceptable salt, solvate, co-crystal or deuterated compound thereof, and the use thereof in the preparation of a drug for treating related diseases mediated by AAK1.

### Background Art

Adaptor-associated kinase 1 (AAK1) is a member of the Ark1/Prk1 family of serine/threonine kinases. AAK1 mRNA exists in two splice forms known as the short form and the long form. The long form is predominant and is highly expressed in the brain and heart (Henderson and Conner, Mol. Biol. Cell. 2007, 18, 2698-2706). AAK1 is enriched in the synaptosomal preparation and is co-localized with endocytic structures in cultured cells. AAK1 regulates the endocytosis involving clathrin coats, a key process in synaptic vesicle recycling and receptor-mediated endocytosis. AAK1 binds to an AP2 complex, which is a heterotetramer connecting the receptor cargo with the clathrin coat. Clathrin-AAK1 binding stimulates AAK1 activity (Conner et al., Traffic 2003, 4, 885-890; Jackson et al., J. Cell. Biol. 2003, 163, 231-236). AAK1 phosphorylates the mu-2 subunit of AP-2, which enhances the binding of mu-2 to tyrosine-containing sorting motifs on the cargo receptor (Ricotta et al., J. Cell Bio. 2002, 156, 791-795; Conner and Schmid, J. Cell Bio. 2002, 156, 921-929). Phosphorylation of Mu2 is not required by receptor uptake, but improves the internalization efficiency (Motely et al., Mol. Biol. Cell. 2006, 17,5298-5308).

AAK1 has been identified as an inhibitor of Neuregulin-1/ErbB4 signalling in PC12 cells. Loss of AAK1 expression via RNA interference-mediated gene silencing or by treatment with the kinase inhibitor K252a (which inhibits AAK1 activity) potentiates Nrg1-induced neurite outgrowth. Such treatments result in increased ErbB4 expression and increased ErbB4 accumulation in the plasma membrane or in close proximity to the plasma membrane (Kuai et al., Chemistry and Biology 2011, 18, 891-906). NRG1 and ErbB4 are putative susceptibility genes of schizophrenia (Buonanno, Brain Res. Bull. 2010, 83, 122-131). The SNPs in both genes are associated with multiple endophenotypes of schizophrenia (Greenwood et al., Am. J. Psychiatry 2011, 168, 930-946). Mouse models for NRG1 and ErbB4KO have displayed morphological changes and behavioural manifestations related to schizophrenia (Jaaro-Peled et al., Schizophrenia Bulletin 2010, 36, 301-313; Wen et al., Proc. Natl. Acad. Sci. USA. 2010, 107, 1211-1216). Furthermore, the single nucleotide polymorphism in the introns of the AAK1 gene has been implicated with the onset age in Parkinson's disease (Latourelle et al., BMC Med. Genet. 2009, 10, 98). These results show that the inhibition of AAK1 activity is useful in treating schizophrenia, cognitive deficit in schizophrenia, Parkinson's disease, neuropathic pain, bipolar disorder and Alzheimer's disease.

### Summary of the Invention

The compound and the stereoisomer, deuterated compound, solvate, prodrug, metabolite, pharmaceutically acceptable salt or co-crystal thereof provided in the present invention have an inhibitory effect on AAK1, can inhibit cell proliferation, possess good pharmacokinetic characteristics, high bioavailability, good safety, high selectivity and low toxicity and side effects, can be administered orally, and have fast absorption, high clearance rate and other advantages. Moreover, it has surprisingly been found that the compound of the present invention has a good brain penetrability.

The present invention relates to a compound of formula (I), (Ia), (Ib) or (II), or a stereoisomer, deuterated compound, solvate, prodrug, metabolite, pharmaceutically acceptable salt or co-crystal thereof,

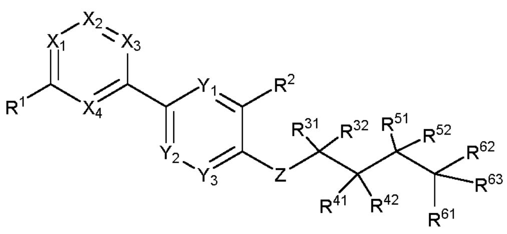

(I)

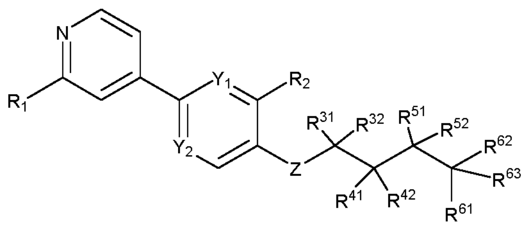

(Ia)

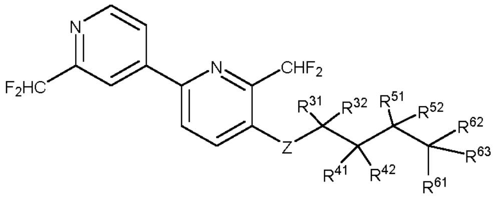

(II)

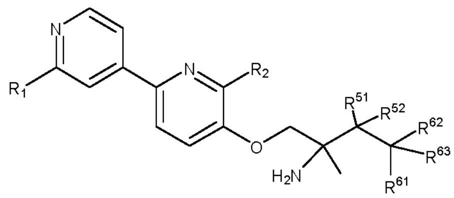

(Ib)

wherein

$X_1$, $X_2$, $X_3$ and $X_4$ are each independently selected from N or $CR^x$; in certain embodiments, $X_1$ is selected from N, and $X_2$, $X_3$ and $X_4$ are each independently selected from N or $CR^x$; in certain embodiments, $X_1$ is selected from N, and $X_2$, $X_3$ and $X_4$ are each independently selected from $CR^x$;

$Y_1$, $Y_2$ and $Y_3$ are each independently selected from N or $CR^y$; in certain embodiments, $Y_1$ is selected from N, and $Y_2$ and $Y_3$ are each independently selected from N or $CR^y$; in certain embodiments, $Y_1$ is selected from N, and $Y_2$ and $Y_3$ are each independently selected from $CR^y$;

Z is selected from $NR^z$ or O; in certain embodiments, Z is selected from O; in certain embodiments, Z is selected from $NR^z$;

$R^z$ is selected from H, deuterium, halogen, $C_{1-6}$ alkyl, halo $C_{1-6}$ alkyl, or deuterated $C_{1-6}$ alkyl; in certain embodiments, $R^z$ is selected from H, deuterium, halogen, $C_{1-4}$ alkyl, halo $C_{1-4}$ alkyl, or deuterated $C_{1-4}$ alkyl; in certain embodiments, $R^z$ is selected from H, deuterium, F, Cl, $C_{1-2}$ alkyl, halo $C_{1-2}$ alkyl, or deuterated $C_{1-2}$ alkyl; in certain embodiments, $R^z$ is selected from H, deuterium, F, Cl, methyl, ethyl, $-CH_2F$, $-CHF_2$, $-CF_3$, $-CH_2CH_2F$, $-CH_2CHF_2$, $-CH_2CF_3$, $-CHFCH_2F$, $-CHFCHF_2$, $-CHFCF_3$, $-CF_2CH_2F$, $-CF_2CHF_2$, $-CF_2CF_3$, $-CH_2D$, $-CHD_2$, $-CD_3$, $-CH_2CH_2D$, $-CH_2CHD_2$, $-CH_2CD_3$, $-CHDCH_2D$, $-CHDCHD_2$, $-CHDCD_3$, $-CD_2CH_2D$, $-CD_2CHD_2$, or $-CD_2CD_3$,

$R^x$ and $R^y$ are each independently selected from H, deuterium, halogen, amino, nitro, cyano, hydroxyl, sulphonyl, $C_{1-6}$ alkyl, halo $C_{1-6}$ alkyl, deuterated $C_{1-6}$ alkyl, $C_{1-6}$ alkoxy, halo $C_{1-6}$ alkoxy, deuterated $C_{1-6}$ alkoxy, hydroxy $C_{1-6}$ alkyl, $C_{3-6}$ cycloalkyl, or $C_{4-6}$ heterocycloalkyl; in certain embodiments, $R^x$ and $R^y$ are each independently selected from H, deuterium, halogen, amino, nitro, cyano, hydroxyl, sulphonyl, $C_{1-4}$ alkyl, halo $C_{1-4}$ alkyl, deuterated $C_{1-4}$ alkyl, $C_{1-4}$ alkoxy, halo $C_{1-4}$ alkoxy, deuterated $C_{1-4}$ alkoxy, hydroxy $C_{1-4}$ alkyl, 3-membered cycloalkyl, 4-membered cycloalkyl, 5-membered cycloalkyl, 6-membered cycloalkyl, 4-membered heterocycloalkyl, 5-membered heterocycloalkyl, or 6-membered heterocycloalkyl; in certain embodiments, $R^x$ and $R^y$ are each independently selected from H, deuterium, F, Cl, amino, nitro, cyano, hydroxyl, sulphonyl, $C_{1-2}$ alkyl, halo $C_{1-2}$ alkyl, deuterated $C_{1-2}$ alkyl, $C_{1-2}$ alkoxy, halo $C_{1-2}$ alkoxy, deuterated $C_{1-2}$ alkoxy, hydroxy $C_{1-2}$ alkyl, 3-membered cycloalkyl, 4-membered cycloalkyl, 5-membered cycloalkyl, 6-membered cycloalkyl, 4-membered heterocycloalkyl, 5-membered heterocycloalkyl, or 6-membered heterocycloalkyl; unless otherwise specified, the heterocycloalkyl contains 1-3 heteroatoms selected from N, S and O, and the alkyl, cycloalkyl and heterocycloalkyl are optionally further substituted with 1-3 $R^A$;

$R^1$ and $R^2$ are each independently selected from H, deuterium, halogen, amino, -COOH, cyano, sulphonyl, aminoacyl, $C_{1-6}$ alkyl, halo $C_{1-6}$ alkyl, deuterated $C_{1-6}$ alkyl, $C_{1-6}$ alkoxy, halo $C_{1-6}$ alkoxy, deuterated $C_{1-6}$ alkoxy, hydroxy $C_{1-6}$ alkyl, $C_{3-6}$ cycloalkyl, 4- to 6-membered heterocycloalkyl containing 1-3 heteroatoms selected from N, S and O, $-NHC(O)C_{1-6}$ alkyl, $-NHC(O)C_{3-6}$ cycloalkyl, $-NHC(O)C_{4-6}$ heterocycloalkyl, $-NHC(O)NHC_{1-6}$ alkyl, or $-NHC(O)OC_{1-6}$ alkyl, wherein the alkyl, cycloalkyl and heterocycloalkyl are optionally further substituted with 1-3 $R^A$ substituents; in certain embodiments, $R^1$ and $R^2$ are each independently selected from H, deuterium, F, Cl, amino, -COOH, cyano, sulphonyl, aminoacyl, $C_{1-4}$ alkyl, halo $C_{1-4}$ alkyl, deuterated $C_{1-4}$ alkyl, $C_{1-4}$ alkoxy, halo $C_{1-4}$ alkoxy, deuterated $C_{1-4}$ alkoxy, hydroxy $C_{1-4}$ alkyl, 3-membered cycloalkyl, 4-membered cycloalkyl, 5-membered cycloalkyl, 6-membered cycloalkyl, 4-membered heterocycloalkyl, 5-membered heterocycloalkyl, 6-membered heterocycloalkyl, $-NHC(O)C_{1-4}$ alkyl, $-NHC(O)C_{3-6}$ cycloalkyl, $-NHC(O)C_{4-6}$ heterocycloalkyl, $-NHC(O)NHC_{1-4}$ alkyl, or $-NHC(O)OC_{1-4}$ alkyl; in certain embodiments, $R^1$ and $R^2$ are each independently selected from H, deuterium, F, Cl, amino, -COOH, cyano, sulphonyl, aminoacyl, $C_{1-2}$ alkyl, halo $C_{1-2}$ alkyl, deuterated $C_{1-2}$ alkyl, $C_{1-2}$ alkoxy, halo $C_{1-2}$ alkoxy, deuterated $C_{1-2}$ alkoxy, hydroxy $C_{1-2}$ alkyl, 3-membered cycloalkyl, 4-membered cycloalkyl, 5-membered cycloalkyl, 6-membered cycloalkyl, 4-membered heterocycloalkyl, 5-membered heterocycloalkyl, 6-membered heterocycloalkyl, $-NHC(O)C_{1-2}$ alkyl, $-NHC(O)C_{3-4}$ cycloalkyl, $-NHC(O)C_{4-5}$ heterocycloalkyl, $-NHC(O)NHC_{1-2}$ alkyl, or $-NHC(O)OC_{1-2}$ alkyl; in certain embodiments, $R^1$ and $R^2$ are each independently selected from H, deuterium, cyano, halo $C_{1-2}$ alkyl, halo $C_{1-2}$ alkoxy, $-NHC(O)C_{1-2}$ alkyl, $-NHC(O)OC_{1-2}$ alkyl, or $-NHC(O)C_{3-4}$ cycloalkyl; in certain embodiments, $R^1$ and $R^2$ are each independently selected from cyano, halo $C_{1-2}$ alkyl, $-NHC(O)C_{1-2}$ alkyl, $-NHC(O)OC_{1-2}$ alkyl, or $-NHC(O)C_{3-4}$ cycloalkyl; in certain embodiments, $R^1$ and $R^2$ are each independently selected from cyano, $-CH_2F$,

- $CHF_2$, -$CF_3$, -$CH_2CH_2F$, -$CH_2CHF_2$, -$CH_2CF_3$, -$CHFCH_2F$, -$CHFCHF_2$, -$CHFCF_3$, -$CF_2CH_2F$, -$CF_2CHF_2$, -$CF_2CF_3$, -NHC(O)$CH_3$, -NHC(O)O$CH_3$

, 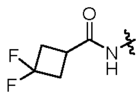 ,

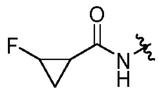 ,

or

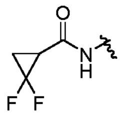 ;

in certain embodiments, $R^1$ and $R^2$ are each independently selected from H, deuterium, halogen, amino, -COOH, cyano, sulphonyl, $C_{1-6}$ alkyl, halo $C_{1-6}$ alkyl, deuterated $C_{1-6}$ alkyl, $C_{1-6}$ alkoxy, halo $C_{1-6}$ alkoxy, deuterated $C_{1-6}$ alkoxy, hydroxy $C_{1-6}$ alkyl, $C_{3-6}$ cycloalkyl, $C_{4-6}$ heterocycloalkyl, -NHC(O)$C_{1-6}$ alkyl, or -NHC(O)O$C_{1-6}$ alkyl; in certain embodiments, $R^1$ and $R^2$ are each independently selected from H, deuterium, F, Cl, amino, -COOH, cyano, sulphonyl, $C_{1-4}$ alkyl, halo $C_{1-4}$ alkyl, deuterated $C_{1-4}$ alkyl, $C_{1-4}$ alkoxy, halo $C_{1-4}$ alkoxy, deuterated $C_{1-4}$ alkoxy, hydroxy $C_{1-4}$ alkyl, 3-membered cycloalkyl, 4-membered cycloalkyl, 5-membered cycloalkyl, 6-membered cycloalkyl, 4-membered heterocycloalkyl, 5-membered heterocycloalkyl, 6-membered heterocycloalkyl, -NHC(O)$C_{1-4}$ alkyl, or - NHC(O)O$C_{1-4}$ alkyl; in certain embodiments, $R^1$ and $R^2$ are each independently selected from H, deuterium, F, Cl, amino, -COOH, cyano, sulphonyl, $C_{1-2}$ alkyl, halo $C_{1-2}$ alkyl, deuterated $C_{1-2}$ alkyl, $C_{1-2}$ alkoxy, halo $C_{1-2}$ alkoxy, deuterated $C_{1-2}$ alkoxy, hydroxy $C_{1-2}$ alkyl, 3-membered cycloalkyl, 4-membered cycloalkyl, 5-membered cycloalkyl, 6-membered cycloalkyl, 4-membered heterocycloalkyl, 5-membered heterocycloalkyl, 6-membered heterocycloalkyl, -NHC(O)$C_{1-2}$ alkyl, or -NHC(O)O$C_{1-2}$ alkyl; in certain embodiments, $R^1$ and $R^2$ are each independently selected from H, deuterium, halo $C_{1-2}$ alkyl, or halo $C_{1-2}$ alkoxy; in certain embodiments, $R^1$ and $R^2$ are each independently selected from halo $C_{1-2}$ alkyl; in certain embodiments, $R^1$ and $R^2$ are each independently selected from -$CH_2F$, - $CHF_2$, -$CF_3$, -$CH_2CH_2F$, -$CH_2CHF_2$, -$CH_2CF_3$, -$CHFCH_2F$, -$CHFCHF_2$, -$CHFCF_3$, -$CF_2CH_2F$, -$CF_2CHF_2$, or -$CF_2CF_3$; unless otherwise specified, the heterocycloalkyl contains 1-3 heteroatoms selected from N, S and O, and the alkyl, cycloalkyl and heterocycloalkyl are optionally further substituted with 1-3 $R^A$; in certain embodiments, $R^1$ is selected from sulphonyl, aminoacyl, halo $C_{1-3}$ alkyl, - NHC(O)$C_{1-4}$ alkyl, -NHC(O)$C_{3-6}$ cycloalkyl, -NHC(O)$C_{4-6}$ heterocycloalkyl, - NHC(O)NH$C_{1-4}$ alkyl, or -NHC(O)O$C_{1-4}$ alkyl, wherein the alkyl, cycloalkyl and heterocycloalkyl are optionally further substituted with 1-3 $R^A$ substituents; $R^2$ is selected from cyano, $C_{1-3}$ alkyl, halo $C_{1-3}$ alkyl, or deuterated $C_{1-3}$ alkyl;

$R^{31}$ and $R^{32}$ are each independently selected from H, deuterium, halogen, cyano, hydroxyl, $C_{1-6}$ alkyl, halo $C_{1-6}$ alkyl, deuterated $C_{1-6}$ alkyl, $C_{1-6}$ alkoxy, halo $C_{1-6}$ alkoxy, deuterated $C_{1-6}$ alkoxy, or hydroxy $C_{1-6}$ alkyl; or $R^{31}$ and $R^{32}$ together with the carbon atom to which they are attached form $C_{3-6}$ cycloalkyl or 4- to 6-membered heterocycloalkyl, wherein the cycloalkyl and heterocycloalkyl are optionally further substituted with 1-3 $R^A$ substituents; in certain embodiments, $R^{31}$ and $R^{32}$ are each independently selected from H, deuterium, F, Cl, cyano, hydroxyl, $C_{1-4}$ alkyl, halo $C_{1-4}$ alkyl, deuterated $C_{1-4}$ alkyl, $C_{1-4}$ alkoxy, halo $C_{1-4}$ alkoxy, deuterated $C_{1-4}$ alkoxy, or hydroxy $C_{1-4}$ alkyl, or $R^{31}$ and $R^{32}$ together with the carbon atom to which they are attached form 3-membered cycloalkyl, 4-membered cycloalkyl, 5-membered cycloalkyl, 6-membered cycloalkyl, 4-membered heterocycloalkyl, 5-membered heterocycloalkyl, or 6-membered heterocycloalkyl, wherein the cycloalkyl and heterocycloalkyl are optionally further substituted with 1, 2 or 3 $R^A$ substituents; in certain embodiments, $R^{31}$ and $R^{32}$ are each independently selected from H, deuterium, F, Cl, hydroxyl, $C_{1-2}$ alkyl, halo $C_{1-2}$ alkyl, deuterated $C_{1-2}$ alkyl, $C_{1-2}$ alkoxy, halo $C_{1-2}$ alkoxy, deuterated $C_{1-2}$ alkoxy, or hydroxy $C_{1-2}$ alkyl, or $R^{31}$ and $R^{32}$ together with the carbon atom to which they are attached form 3-membered cycloalkyl, 4-membered cycloalkyl, or 4-membered heterocycloalkyl, wherein the cycloalkyl and heterocycloalkyl are optionally further substituted with 1, 2 or 3 $R^A$ substituents; unless otherwise specified, the heterocycloalkyl contains 1-3 heteroatoms selected from N, S and O;

$R^{41}$ and $R^{42}$ are each independently selected from H, deuterium, amino, $C_{1-6}$ alkyl, halogen, cyano, hydroxyl, halo

$C_{1-6}$ alkyl, deuterated $C_{1-6}$ alkyl, $C_{1-6}$ alkoxy, halo $C_{1-6}$ alkoxy, -$C_{1-6}$ alkyl-$C_{3-6}$ cycloalkyl, $C_{3-6}$ cycloalkyl, $C_{4-6}$ heterocycloalkyl, deuterated $C_{1-6}$ alkoxy, or hydroxy $C_{1-6}$ alkyl; in certain embodiments, $R^{41}$ and $R^{42}$ are each independently selected from H, deuterium, amino, $C_{1-4}$ alkyl, halogen, cyano, hydroxyl, halo $C_{1-4}$ alkyl, deuterated $C_{1-6}$ alkyl, $C_{1-4}$ alkoxy, halo $C_{1-4}$ alkoxy, -$C_{1-4}$ alkyl-3-membered cycloalkyl, -$C_{1-4}$ alkyl-4-membered cycloalkyl, -$C_{1-4}$ alkyl-5-membered cycloalkyl, 3-membered cycloalkyl, 4-membered cycloalkyl, 5-membered cycloalkyl, 6-membered cycloalkyl, 4-membered heterocycloalkyl, 5-membered heterocycloalkyl, 6-membered heterocycloalkyl, deuterated $C_{1-4}$ alkoxy, or hydroxy $C_{1-4}$ alkyl;

in certain embodiments, $R^{41}$ is selected from H, deuterium, amino, $C_{1-4}$ alkyl, halogen, cyano, hydroxyl, halo $C_{1-4}$ alkyl, deuterated $C_{1-6}$ alkyl, $C_{1-4}$ alkoxy, halo $C_{1-4}$ alkoxy, -$C_{1-4}$ alkyl-3-membered cycloalkyl, -$C_{1-4}$ alkyl-4-membered cycloalkyl, -$C_{1-4}$ alkyl-5-membered cycloalkyl, 3-membered cycloalkyl, 4-membered cycloalkyl, 5-membered cycloalkyl, 6-membered cycloalkyl, 4-membered heterocycloalkyl, 5-membered heterocycloalkyl, 6-membered heterocycloalkyl, deuterated $C_{1-4}$ alkoxy, or hydroxy $C_{1-4}$ alkyl; in certain embodiments, $R^{41}$ is selected from H, deuterium, amino, $C_{1-4}$ alkyl, halogen, cyano, hydroxyl, halo $C_{1-2}$ alkyl, deuterated $C_{1-2}$ alkyl, $C_{1-2}$ alkoxy, halo $C_{1-2}$ alkoxy, -$C_{1-2}$ alkyl-3-membered cycloalkyl, -$C_{1-2}$ alkyl-4-membered cycloalkyl, - $C_{1-2}$ alkyl-5-membered cycloalkyl, 3-membered cycloalkyl, 4-membered cycloalkyl, 5-membered cycloalkyl, 6-membered cycloalkyl, 4-membered heterocycloalkyl, 5-membered heterocycloalkyl, 6-membered heterocycloalkyl, deuterated $C_{1-2}$ alkoxy, or hydroxy $C_{1-2}$ alkyl;

in certain embodiments, $R^{42}$ is selected from H, hydroxyl, amino, $C_{1-2}$ alkyl, deuterated $C_{1-2}$ alkyl, or $C_{1-2}$ alkoxy;

in certain embodiments, $R^{42}$ is selected from amino; unless otherwise specified, the heterocycloalkyl contains 1-3 heteroatoms selected from N, S and O;

or $R^{41}$ and $R^{42}$ together form $C_{3-6}$ cycloalkyl or 4- to 6-membered heterocycloalkyl containing 1 heteroatom selected from O or S, wherein the cycloalkyl and heterocycloalkyl are optionally further substituted with 1-3 $R^A$ substituents;

in certain embodiments, $R^{41}$ and $R^{42}$ together form 3-membered cycloalkyl, 4-membered cycloalkyl, 5-membered cycloalkyl, 6-membered cycloalkyl, or 4-membered heterocycloalkyl, 5-membered heterocycloalkyl, or 6-membered heterocycloalkyl containing 1 heteroatom selected from O or S, wherein the cycloalkyl and heterocycloalkyl are optionally further substituted with 1, 2 or 3 $R^A$ substituents;

$R^{51}$ and $R^{52}$ are each independently selected from H, deuterium, amino, halogen, $C_{1-6}$ alkyl, cyano, hydroxyl, halo $C_{1-6}$ alkyl, deuterated $C_{1-6}$ alkyl, $C_{1-6}$ alkoxy, halo $C_{1-6}$ alkoxy, -$C_{1-6}$ alkyl-$C_{3-6}$ cycloalkyl, $C_{3-6}$ cycloalkyl, $C_{4-6}$ heterocycloalkyl, deuterated $C_{1-6}$ alkoxy, or hydroxy $C_{1-6}$ alkyl; or $R^{51}$ and $R^{52}$ together with the carbon atom to which they are attached form $C_{3-6}$ cycloalkyl or 4-to 6-membered heterocycloalkyl, wherein the cycloalkyl and heterocycloalkyl are optionally further substituted with 1-3 $R^A$ substituents; in certain embodiments, $R^{51}$ and $R^{52}$ are each independently selected from H, deuterium, amino, F, Cl, $C_{1-4}$ alkyl, cyano, hydroxyl, halo $C_{1-4}$ alkyl, deuterated $C_{1-4}$ alkyl, $C_{1-4}$ alkoxy, halo $C_{1-4}$ alkoxy, -$C_{1-4}$ alkyl-3-membered cycloalkyl, -$C_{1-4}$ alkyl-4-membered cycloalkyl, - $C_{1-4}$ alkyl-5-membered cycloalkyl, 3-membered cycloalkyl, 4-membered cycloalkyl, 5-membered cycloalkyl, 6-membered cycloalkyl, 4-membered heterocycloalkyl, 5-membered heterocycloalkyl, 6-membered heterocycloalkyl, deuterated $C_{1-4}$ alkoxy, or hydroxy $C_{1-4}$ alkyl; or $R^{51}$ and $R^{52}$ together with the carbon atom to which they are attached form 3-membered cycloalkyl, 4-membered cycloalkyl, 5-membered cycloalkyl, 6-membered cycloalkyl, 4-membered heterocycloalkyl, 5-membered heterocycloalkyl, or 6-membered heterocycloalkyl, wherein the cycloalkyl and heterocycloalkyl are optionally further substituted with 1, 2 or 3 $R^A$ substituents; in certain embodiments, $R^{51}$ and $R^{52}$ are each independently selected from H, deuterium, amino, F, Cl, $C_{1-4}$ alkyl, cyano, hydroxyl, halo $C_{1-2}$ alkyl, deuterated $C_{1-2}$ alkyl, $C_{1-2}$ alkoxy, halo $C_{1-2}$ alkoxy, -$C_{1-2}$ alkyl -3-membered cycloalkyl, -$C_{1-2}$ alkyl-4-membered cycloalkyl, -$C_{1-2}$ alkyl-5-membered cycloalkyl, 3-membered cycloalkyl, 4-membered cycloalkyl, 5-membered cycloalkyl, 6-membered cycloalkyl, 4-membered heterocycloalkyl, 5-membered heterocycloalkyl, 6-membered heterocycloalkyl, deuterated $C_{1-2}$ alkoxy, or hydroxy $C_{1-2}$ alkyl, or $R^{51}$ and $R^{52}$ together with the carbon atom to which they are attached form 3-membered cycloalkyl, 4-membered cycloalkyl, 4-membered heterocycloalkyl, or 5-membered heterocycloalkyl, wherein the cycloalkyl and heterocycloalkyl are optionally further substituted with 1, 2 or 3 $R^A$ substituents; in certain embodiments, $R^{51}$ and $R^{52}$ are each independently selected from H, deuterium, amino, F, Cl, $C_{1-4}$ alkyl, cyano, hydroxyl, halo $C_{1-2}$ alkyl, or $R^{51}$ and $R^{52}$ together with the carbon atom to which they are attached form 3-membered cycloalkyl; unless otherwise specified, the heterocycloalkyl contains 1-3 heteroatoms selected from N, S and O;

$R^{61}$, $R^{62}$ and $R^{63}$ are each independently selected from H, deuterium, halogen, amino, cyano, hydroxyl, $C_{1-6}$ alkyl, halo $C_{1-6}$ alkyl, deuterated $C_{1-6}$ alkyl, $C_{1-6}$ alkoxy, halo $C_{1-6}$ alkoxy, -$C_{1-6}$ alkyl-$C_{3-6}$ cycloalkyl, $C_{3-6}$ cycloalkyl, $C_{4-6}$ heterocycloalkyl, deuterated $C_{1-6}$ alkoxy, or hydroxy $C_{1-6}$ alkyl; in certain embodiments, $R^{61}$, $R^{62}$ and $R^{63}$ are each independently selected from H, deuterium, F, Cl, amino, cyano, hydroxyl, $C_{1-4}$ alkyl, halo $C_{1-4}$ alkyl, deuterated $C_{1-4}$ alkyl, $C_{1-4}$ alkoxy, halo $C_{1-4}$ alkoxy, -$C_{1-4}$ alkyl-3-membered cycloalkyl, -$C_{1-4}$ alkyl-4-membered cycloalkyl, -$C_{1-4}$ alkyl-5-membered cycloalkyl, 3-membered cycloalkyl, 4-membered cycloalkyl, 5-membered cycloalkyl, 6-membered cycloalkyl, 4-membered heterocycloalkyl, 5-membered heterocycloalkyl, 6-membered heterocycloalkyl, deuterated $C_{1-4}$ alkoxy, or hydroxy $C_{1-4}$ alkyl; in certain embodiments, $R^{61}$, $R^{62}$ and $R^{63}$ are each independently selected from H, deuterium, F, Cl, amino, cyano, hydroxyl, $C_{1-2}$ alkyl, halo $C_{1-2}$ alkyl, deuterated $C_{1-2}$ alkyl, $C_{1-2}$ alkoxy, halo $C_{1-2}$

alkoxy, -$C_{1-2}$ alkyl-3-membered cycloalkyl, -$C_{1-2}$ alkyl-4-membered cycloalkyl, -$C_{1-2}$ alkyl-5-membered cycloalkyl, 3-membered cycloalkyl, 4-membered cycloalkyl, 5-membered cycloalkyl, 6-membered cycloalkyl, 4-membered heterocycloalkyl, 5-membered heterocycloalkyl, 6-membered heterocycloalkyl, deuterated $C_{1-2}$ alkoxy, or hydroxy $C_{1-2}$ alkyl; in certain embodiments, $R^{61}$, $R^{62}$ and $R^{63}$ are each independently selected from H, deuterium, F, Cl, amino, cyano, hydroxyl, methyl, ethyl, -$CH_2F$, -$CHF_2$, -$CF_3$, -$CH_2CH_2F$, - $CH_2CHF_2$, -$CH_2CF_3$, -$CHFCH_2F$, -$CHFCHF_2$, -$CHFCF_3$, -$CF_2CH_2F$, -$CF_2CHF_2$, - $CF_2CF_3$, -$CH_2D$, -$CHD_2$, -$CD_3$, -$CH_2CH_2D$, -$CH_2CHD_2$, -$CH_2CD_3$, -$CHDCH_2D$, - $CHDCHD_2$, -$CHDCD_3$, -$CD_2CH_2D$, -$CD_2CHD_2$, -$CD_2CD_3$, methoxy, ethoxy, - $OCHF_2$, -$OCH_2F$, -$OCF_3$, -$OCH_2CH_2F$, -$OCH_2CHF_2$, -$OCH_2CF_3$, -$OCHFCH_2F$, - $OCHFCHF_2$, -$OCHFCF_3$, -$OCF_2CH_2F$, -$OCF_2CHF_2$, -$OCF_2CF_3$, -methyl-3-membered cycloalkyl, -ethyl-3-membered cycloalkyl, -methyl-4-membered cycloalkyl, -ethyl-4-membered cycloalkyl, -methyl-5-membered cycloalkyl, -ethyl-5-membered cycloalkyl, 3-membered cycloalkyl, 4-membered cycloalkyl, 5-membered cycloalkyl, 6-membered cycloalkyl, 4-membered heterocycloalkyl, 5-membered heterocycloalkyl, 6-membered heterocycloalkyl, -$OCHD_2$, -$OCH_2D$, - $OCD_3$, -$OCH_2CH_2D$, -$OCH_2CHD_2$, -$OCH_2CD_3$, -$OCHDCH_2D$, -$OCHDCHD_2$, - $OCHDCD_3$, -$OCD_2CH_2D$, -$OCD_2CHD_2$, -$OCD_2CD_3$, -$CH_2OH$, or -$CH_2CH_2OH$; unless otherwise specified, the heterocycloalkyl contains 1-3 heteroatoms selected from N, S and O;

or, $R^{31}$ and $R^{41}$, or $R^{41}$ and $R^{51}$ together with the carbon atoms to which they are each attached form $C_{3-6}$ cycloalkyl or 4- to 6-membered heterocycloalkyl, wherein the cycloalkyl and heterocycloalkyl are optionally further substituted with 1-3 $R^A$ substituents; in certain embodiments, $R^{31}$ and $R^{41}$, or $R^{41}$ and $R^{51}$ together with the carbon atoms to which they are each attached form 3-membered cycloalkyl, 4-membered cycloalkyl, 5-membered cycloalkyl, 6-membered cycloalkyl, 4-membered heterocycloalkyl, 5-membered heterocycloalkyl, or 6-membered heterocycloalkyl, wherein the cycloalkyl and heterocycloalkyl are optionally further substituted with 1, 2 or 3 $R^A$ substituents; unless otherwise specified, the heterocycloalkyl contains 1-3 heteroatoms selected from N, S and O;

or, $R^{41}$ and $R^{61}$, or $R^z$ and $R^{41}$ together with the atoms to which they are each attached form $C_{4-6}$ cycloalkyl or 4- to 6-membered heterocycloalkyl, wherein the cycloalkyl and heterocycloalkyl are optionally further substituted with 1-3 $R^A$ substituents; in certain embodiments, $R^{41}$ and $R^{61}$, or $R^z$ and $R^{41}$ together with the atoms to which they are each attached form 4-membered cycloalkyl, 5-membered cycloalkyl, 6-membered cycloalkyl, 4-membered heterocycloalkyl, 5-membered heterocycloalkyl, or 6-membered heterocycloalkyl, wherein the cycloalkyl and heterocycloalkyl are optionally further substituted with 1, 2 or 3 $R^A$ substituents; unless otherwise specified, the heterocycloalkyl contains 1-3 heteroatoms selected from N, S and O;

or, $R^{51}$ and $R^{61}$, or $R^{61}$ and $R^{62}$ together with the carbon atom(s) to which they are each attached form $C_{3-6}$ cycloalkyl, 4- to 6-membered heterocycloalkyl, or a double bond, wherein the cycloalkyl and heterocycloalkyl are optionally further substituted with 1-3 $R^A$ substituents; in certain embodiments, $R^{51}$ and $R^{61}$, or $R^{61}$ and $R^{62}$ together with the carbon atom(s) to which they are each attached form 3-membered cycloalkyl, 4-membered cycloalkyl, 5-membered cycloalkyl, 6-membered cycloalkyl, 4-membered heterocycloalkyl, 5-membered heterocycloalkyl, 6-membered heterocycloalkyl, or a double bond, wherein the cycloalkyl and heterocycloalkyl are optionally further substituted with 1, 2 or 3 $R^A$ substituents; unless otherwise specified, the heterocycloalkyl contains 1-3 heteroatoms selected from N, S and O;

or, $R^{61}$, $R^{62}$ and $R^{63}$ together with the carbon atom to which they are attached form $C_{5-10}$ bridged ring, or $C_{5-11}$ spiro ring, wherein the bridged ring and spiro ring are optionally further substituted with 1-3 $R^A$ substituents; in certain embodiments, $R^{61}$, $R^{62}$ and $R^{63}$ together with the carbon atom to which they are attached form 5-membered bridged ring, 6-membered bridged ring, 7-membered bridged ring, 8-membered bridged ring, 5-membered spiro ring, 6-membered spiro ring, 7-membered spiro ring, 8-membered spiro ring, 9-membered spiro ring, 10-membered spiro ring, or 11-membered spiro ring, wherein the bridged ring and spiro ring are optionally further substituted with 1, 2 or 3 $R^A$ substituents; in certain embodiments, $R^{61}$, $R^{62}$ and $R^{63}$ together with the carbon atom to which they are attached form 5-membered saturated carbocyclic bridged ring, 6-membered saturated carbocyclic bridged ring, 7-membered saturated carbocyclic bridged ring, 8-membered saturated carbocyclic bridged ring, 3-membered carbocycle-spiro-3-membered carbocyclyl, 3-membered carbocycle-spiro-4-membered carbocyclyl, 3-membered carbocycle-spiro-5-membered carbocyclyl, 3-membered carbocycle-spiro-6-membered carbocyclyl, 4-membered carbocycle-spiro-3-membered carbocyclyl, 4-membered carbocycle-spiro-4-membered carbocyclyl, 4-membered carbocycle-spiro-5-membered carbocyclyl, 4-membered carbocycle-spiro-6-membered carbocyclyl, 5-membered carbocycle-spiro-3-membered carbocyclyl, 5-membered carbocycle-spiro-4-membered carbocyclyl, 5-membered carbocycle-spiro-5-membered carbocyclyl, 5-membered carbocycle-spiro-6-membered carbocyclyl, 6-membered carbocycle-spiro-3-membered carbocyclyl, 6-membered carbocycle-spiro-4-membered carbocyclyl, 6-membered carbocycle-spiro-5-membered carbocyclyl, 6-membered carbocycle-spiro-6-membered carbocyclyl, 3-membered carbocycle-spiro-3-membered heterocyclyl, 3-membered carbocycle-spiro-4-membered heterocyclyl, 3-membered carbocycle-spiro-5-membered heterocyclyl, 3-membered carbocycle-spiro-6-membered heterocyclyl, 4-membered carbocycle-spiro-3-membered heterocyclyl, 4-membered carbocycle-spiro-4-membered heterocyclyl, 4-membered carbocycle-spiro-5-membered heterocyclyl, 4-membered carbocycle-spiro-6-membered heterocyclyl, 5-membered carbocycle-spiro-3-membered heterocyclyl, 5-membered carbocycle-spiro-4-membered heterocyclyl, 5-membered

carbocycle-spiro-5-membered heterocyclyl, 5-membered carbocycle-spiro-6-membered heterocyclyl, 6-membered carbocycle-spiro-3-membered heterocyclyl, 6-membered carbocycle-spiro-4-membered heterocyclyl, 6-membered carbocycle-spiro-5-membered heterocyclyl, 6-membered carbocycle-spiro-6-membered heterocyclyl, 3-membered heterocycle-spiro-3-membered carbocyclyl, 3-membered heterocycle-spiro-4-membered carbocyclyl, 3-membered heterocycle-spiro-5-membered carbocyclyl, 3-membered heterocycle-spiro-6-membered carbocyclyl, 4-membered heterocycle-spiro-3-membered carbocyclyl, 4-membered heterocycle-spiro-4-membered carbocyclyl, 4-membered heterocycle-spiro-5-membered carbocyclyl, 4-membered heterocycle-spiro-6-membered carbocyclyl, 5-membered heterocycle-spiro-3-membered carbocyclyl, 5-membered heterocycle-spiro-4-membered carbocyclyl, 5-membered heterocycle-spiro-5-membered carbocyclyl, 5-membered heterocycle-spiro-6-membered carbocyclyl, 6-membered heterocycle-spiro-3-membered carbocyclyl, 6-membered heterocycle-spiro-4-membered carbocyclyl, 6-membered heterocycle-spiro-5-membered carbocyclyl, 6-membered heterocycle-spiro-6-membered carbocyclyl, 3-membered heterocycle-spiro-3-membered heterocyclyl, 3-membered heterocycle-spiro-4-membered heterocyclyl, 3-membered heterocycle-spiro-5-membered heterocyclyl, 3-membered heterocycle-spiro-6-membered heterocyclyl, 4-membered heterocycle-spiro-3-membered heterocyclyl, 4-membered heterocycle-spiro-4-membered heterocyclyl, 4-membered heterocycle-spiro-5-membered heterocyclyl, 4-membered heterocycle-spiro-6-membered heterocyclyl, 5-membered heterocycle-spiro-3-membered heterocyclyl, 5-membered heterocycle-spiro-4-membered heterocyclyl, 5-membered heterocycle-spiro-5-membered heterocyclyl, 5-membered heterocycle-spiro-6-membered heterocyclyl, 6-membered heterocycle-spiro-3-membered heterocyclyl, 6-membered heterocycle-spiro-4-membered heterocyclyl, 6-membered heterocycle-spiro-5-membered heterocyclyl, or 6-membered heterocycle-spiro-6-membered heterocyclyl; the carbocyclyl and heterocyclyl are optionally further substituted with 1, 2 or 3 $R^A$ substituents; unless otherwise specified, the heterocycloalkyl contains 1-3 heteroatoms selected from N, S and O;

$R^A$ is selected from deuterium, halogen, amino, cyano, hydroxyl, $C_{1-6}$ alkyl, halo $C_{1-6}$ alkyl, deuterated $C_{1-6}$ alkyl, $C_{1-6}$ alkoxy, halo $C_{1-6}$ alkoxy, deuterated $C_{1-6}$ alkoxy, or hydroxy $C_{1-6}$ alkyl; in certain embodiments, $R^A$ is selected from deuterium, F, Cl, amino, cyano, hydroxyl, $C_{1-4}$ alkyl, halo $C_{1-4}$ alkyl, deuterated $C_{1-4}$ alkyl, $C_{1-4}$ alkoxy, halo $C_{1-4}$ alkoxy, deuterated $C_{1-4}$ alkoxy, or hydroxy $C_{1-4}$ alkyl; in certain embodiments, $R^A$ is selected from deuterium, F, Cl, amino, cyano, hydroxyl, $C_{1-2}$ alkyl, halo $C_{1-2}$ alkyl, deuterated $C_{1-2}$ alkyl, $C_{1-2}$ alkoxy, halo $C_{1-2}$ alkoxy, deuterated $C_{1-2}$ alkoxy, or hydroxy $C_{1-2}$ alkyl; unless otherwise specified, the heterocycloalkyl contains 1-3 heteroatoms selected from N, S and O,

provided that when Z is selected from O,

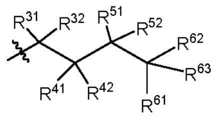

does not form the following structures:

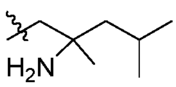

and

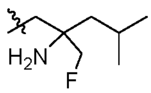

.

In particular, as a first technical solution of the present invention, the present invention relates to a compound of formula (I), or a stereoisomer, deuterated compound, solvate, prodrug, metabolite, pharmaceutically acceptable salt or co-crystal thereof,

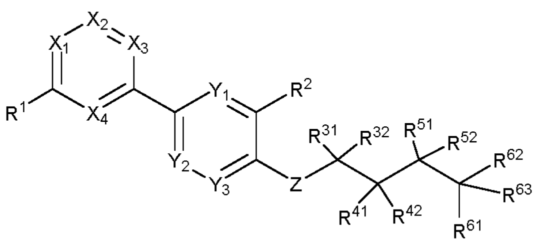

(I)

wherein

$X_1$, $X_2$, $X_3$ and $X_4$ are each independently selected from N or $CR^x$;

$Y_1$, $Y_2$ and $Y_3$ are each independently selected from N or $CR^7$;
Z is selected from $NR^z$ or O;
$R^z$ is selected from H, deuterium, halogen, $C_{1-6}$ alkyl, halo $C_{1-6}$ alkyl, or deuterated $C_{1-6}$ alkyl;
$R^x$ and $R^y$ are each independently selected from H, deuterium, halogen, amino, nitro, cyano, hydroxyl, sulphonyl, $C_{1-6}$ alkyl, halo $C_{1-6}$ alkyl, deuterated $C_{1-6}$ alkyl, $C_{1-6}$ alkoxy, halo $C_{1-6}$ alkoxy, deuterated $C_{1-6}$ alkoxy, hydroxy $C_{1-6}$ alkyl, $C_{3-6}$ cycloalkyl, or 4- to 6-membered heterocycloalkyl containing 1-3 heteroatoms selected from N, S and O, wherein the alkyl, cycloalkyl and heterocycloalkyl are optionally further substituted with 1-3 $R^A$;
$R^1$ and $R^2$ are each independently selected from H, deuterium, halogen, amino, -COOH, cyano, sulphonyl, aminoacyl, $C_{1-6}$ alkyl, halo $C_{1-6}$ alkyl, deuterated $C_{1-6}$ alkyl, $C_{1-6}$ alkoxy, halo $C_{1-6}$ alkoxy, deuterated $C_{1-6}$ alkoxy, hydroxy $C_{1-6}$ alkyl, $C_{3-6}$ cycloalkyl, 4- to 6-membered heterocycloalkyl containing 1-3 heteroatoms selected from N, S and O, $-NHC(O)C_{1-6}$ alkyl, $-NHC(O)C_{3-6}$ cycloalkyl, $-NHC(O)C_{4-6}$ heterocycloalkyl, $-NHC(O)NHC_{1-6}$ alkyl, or $-NHC(O)OC_{1-6}$ alkyl, wherein the alkyl, cycloalkyl and heterocycloalkyl are optionally further substituted with 1-3 $R^A$;
$R^{31}$ and $R^{32}$ are each independently selected from H, deuterium, halogen, cyano, hydroxyl, $C_{1-6}$ alkyl, halo $C_{1-6}$ alkyl, deuterated $C_{1-6}$ alkyl, $C_{1-6}$ alkoxy, halo $C_{1-6}$ alkoxy, deuterated $C_{1-6}$ alkoxy, or hydroxy $C_{1-6}$ alkyl; or $R^{31}$ and $R^{32}$ together with the carbon atom to which they are attached form $C_{3-6}$ cycloalkyl or 4- to 6-membered heterocycloalkyl containing 1-3 heteroatoms selected from N, S and O, wherein the cycloalkyl and heterocycloalkyl are optionally further substituted with 1-3 $R^A$ substituents;
$R^{41}$ and $R^{42}$ are each independently selected from H, deuterium, amino, $C_{1-6}$ alkyl, halogen, cyano, hydroxyl, halo $C_{1-6}$ alkyl, deuterated $C_{1-6}$ alkyl, $C_{1-6}$ alkoxy, halo $C_{1-6}$ alkoxy, $-C_{1-6}$ alkyl-$C_{3-6}$ cycloalkyl, $C_{3-6}$ cycloalkyl, 4- to 6-membered heterocycloalkyl containing 1-3 heteroatoms selected from N, S and O, deuterated $C_{1-6}$ alkoxy, or hydroxy $C_{1-6}$ alkyl;
or $R^{41}$ and $R^{42}$ together form $C_{3-6}$ cycloalkyl or 4- to 6-membered heterocycloalkyl containing 1 heteroatom selected from O or S, wherein the cycloalkyl and heterocycloalkyl are optionally further substituted with 1-3 $R^A$ substituents;
$R^{51}$ and $R^{52}$ are each independently selected from H, deuterium, amino, halogen, $C_{1-6}$ alkyl, cyano, hydroxyl, halo $C_{1-6}$ alkyl, deuterated $C_{1-6}$ alkyl, $C_{1-6}$ alkoxy, halo $C_{1-6}$ alkoxy, $-C_{1-6}$ alkyl-$C_{3-6}$ cycloalkyl, $C_{3-6}$ cycloalkyl, 4- to 6-membered heterocycloalkyl containing 1-3 heteroatoms selected from N, S and O, deuterated $C_{1-6}$ alkoxy, or hydroxy $C_{1-6}$ alkyl; or $R^{51}$ and $R^{52}$ together with the carbon atom to which they are attached form $C_{3-6}$ cycloalkyl or 4- to 6-membered heterocycloalkyl containing 1-3 heteroatoms selected from N, S and O, wherein the cycloalkyl and heterocycloalkyl are optionally further substituted with 1-3 $R^A$ substituents;
$R^{61}$, $R^{62}$ and $R^{63}$ are each independently selected from H, deuterium, halogen, amino, cyano, hydroxyl, $C_{1-6}$ alkyl, halo $C_{1-6}$ alkyl, deuterated $C_{1-6}$ alkyl, $C_{1-6}$ alkoxy, halo $C_{1-6}$ alkoxy, $-C_{1-6}$ alkyl-$C_{3-6}$ cycloalkyl, $C_{3-6}$ cycloalkyl, 4- to 6-membered heterocycloalkyl containing 1-3 heteroatoms selected from N, S and O, deuterated $C_{1-6}$ alkoxy, or hydroxy $C_{1-6}$ alkyl;
or, $R^{31}$ and $R^{41}$, or $R^{41}$ and $R^{51}$ together with the carbon atoms to which they are each attached form $C_{3-6}$ cycloalkyl or 4- to 6-membered heterocycloalkyl containing 1-3 heteroatoms selected from N, S and O, wherein the cycloalkyl and heterocycloalkyl are optionally further substituted with 1-3 $R^A$ substituents;
or, $R^{41}$ and $R^{61}$, or $R^z$ and $R^{41}$ together with the atoms to which they are each attached form $C_{4-6}$ cycloalkyl or 4- to 6-membered heterocycloalkyl containing 1-3 heteroatoms selected from N, S and O, wherein the cycloalkyl and heterocycloalkyl are optionally further substituted with 1-3 $R^A$ substituents;
or, $R^{51}$ and $R^{61}$, or $R^{61}$ and $R^{62}$ together with the carbon atom(s) to which they are each attached form $C_{3-6}$ cycloalkyl, 4- to 6-membered heterocycloalkyl containing 1-3 heteroatoms selected from N, S and O, or a double bond, wherein the cycloalkyl and heterocycloalkyl are optionally further substituted with 1-3 $R^A$ substituents;
or, $R^{61}$, $R^{62}$ and $R^{63}$ together with the carbon atom to which they are attached form $C_{5-10}$ bridged ring, or $C_{5-11}$ spiro ring, wherein the bridged ring and spiro ring are optionally further substituted with 1-3 $R^A$ substituents;
$R^A$ is selected from deuterium, halogen, amino, cyano, hydroxyl, $C_{1-6}$ alkyl, halo $C_{1-6}$ alkyl, deuterated $C_{1-6}$ alkyl, $C_{1-6}$ alkoxy, halo $C_{1-6}$ alkoxy, deuterated $C_{1-6}$ alkoxy, or hydroxy $C_{1-6}$ alkyl,
provided that when Z is selected from O,

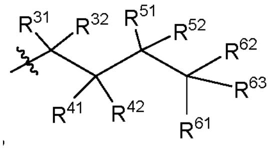

,

does not form the following structures:

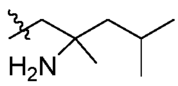

and

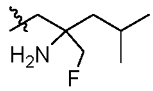

.

As a second technical solution of the present invention, the present invention relates to a compound of formula (Ia), or a stereoisomer, deuterated compound, solvate, prodrug, metabolite, pharmaceutically acceptable salt or co-crystal thereof

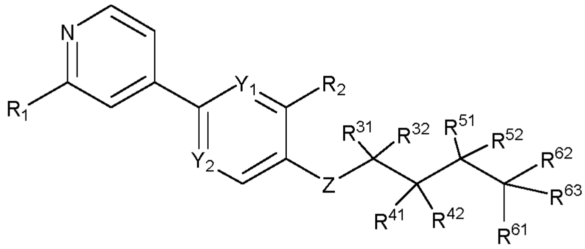

(Ia).

As a third technical solution of the present invention, the present invention relates to the compound, or the stereoisomer, deuterated compound, solvate, prodrug, metabolite, pharmaceutically acceptable salt or co-crystal thereof according to the present invention, wherein

$R^1$ is selected from sulphonyl, aminoacyl, halo $C_{1-3}$ alkyl, -NHC(O)$C_{1-4}$ alkyl, -NHC(O)$C_{3-6}$ cycloalkyl, -NHC(O)$C_{4-6}$ heterocycloalkyl, -NHC(O)NH$C_{1-4}$ alkyl, or -NHC(O)O$C_{1-4}$ alkyl, wherein the alkyl, cycloalkyl and heterocycloalkyl are optionally further substituted with 1-3 $R^A$ substituents;
$R^2$ is selected from cyano, $C_{1-3}$ alkyl, halo $C_{1-3}$ alkyl, or deuterated $C_{1-3}$ alkyl;
$R^A$ is selected from deuterium, F, Cl, amino, cyano, hydroxyl, $C_{1-3}$ alkyl, halo $C_{1-3}$ alkyl, deuterated $C_{1-3}$ alkyl, $C_{1-3}$ alkoxy, halo $C_{1-3}$ alkoxy, deuterated $C_{1-3}$ alkoxy, or hydroxy $C_{1-3}$ alkyl;
other groups have the same definitions as those in the preceding technical solutions.

As a fourth technical solution of the present invention, the present invention relates to the compound, or the stereoisomer, deuterated compound, solvate, prodrug, metabolite, pharmaceutically acceptable salt or co-crystal thereof according to the present invention, having a structure of formula (II):

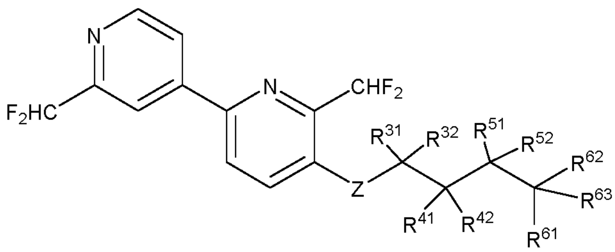

(II)

other groups have the same definitions as those in the technical solutions described above.

As a fifth technical solution of the present invention, the present invention relates to the compound, or the stereoisomer, deuterated compound, solvate, prodrug, metabolite, pharmaceutically acceptable salt or co-crystal thereof according to the present invention, wherein

Z is selected from $NR^z$ or O;
$R^z$ is selected from H, deuterium or $C_{1-4}$ alkyl;
$R^{31}$ and $R^{32}$ are each independently selected from H, deuterium, F, Cl, cyano, hydroxyl, $C_{1-4}$ alkyl, halo $C_{1-4}$ alkyl, deuterated $C_{1-4}$ alkyl, $C_{1-4}$ alkoxy, halo $C_{1-4}$ alkoxy, deuterated $C_{1-4}$ alkoxy, or hydroxy $C_{1-4}$ alkyl; or $R^{31}$ and $R^{32}$ together with the carbon atom to which they are attached form 3-membered cycloalkyl, 4-membered cycloalkyl, 5-membered cycloalkyl, 4-membered heterocycloalkyl, or 5-membered heterocycloalkyl, wherein the cycloalkyl and heterocycloalkyl are optionally further substituted with 1, 2 or 3 substituents selected from $R^A$, and the heterocycloalkyl contains 1-3 heteroatoms selected from N, S and O;

$R^{41}$ and $R^{42}$ are each independently selected from H, deuterium, amino, $C_{1-4}$ alkyl, halogen, cyano, hydroxyl, halo $C_{1-4}$ alkyl, deuterated $C_{1-4}$ alkyl, $C_{1-4}$ alkoxy, halo $C_{1-4}$ alkoxy, $-C_{1-4}$ alkyl-3-membered cycloalkyl, $-C_{1-4}$ alkyl-4-membered cycloalkyl, $-C_{1-4}$ alkyl-5-membered cycloalkyl, 3-membered cycloalkyl, 4-membered cycloalkyl, 5-membered cycloalkyl, 4-membered heterocycloalkyl, 5-membered heterocycloalkyl, 6-membered heterocycloalkyl, deuterated $C_{1-4}$ alkoxy, or hydroxy $C_{1-4}$ alkyl; or $R^{41}$ and $R^{42}$ together form 3-membered cycloalkyl, 4-membered cycloalkyl, 5-membered cycloalkyl, 4-membered heterocycloalkyl, or 5-membered heterocycloalkyl, wherein the cycloalkyl and heterocycloalkyl are optionally further substituted with 1, 2 or 3 substituents selected from $R^A$, and the heterocycloalkyl contains 1-3 heteroatoms selected from N, S and O;

$R^{51}$ and $R^{52}$ are each independently selected from H, deuterium, amino, halogen, $C_{1-4}$ alkyl, cyano, hydroxyl, halo $C_{1-4}$alkyl, deuterated $C_{1-4}$ alkyl, $C_{1-4}$ alkoxy, halo $C_{1-4}$ alkoxy, $-C_{1-4}$ alkyl-3-membered cycloalkyl, $-C_{1-4}$ alkyl-4-membered cycloalkyl, $-C_{1-4}$ alkyl-5-membered cycloalkyl, 3-membered cycloalkyl, 4-membered cycloalkyl, 5-membered cycloalkyl, 4-membered heterocycloalkyl, 5-membered heterocycloalkyl, 6-membered heterocycloalkyl, deuterated $C_{1-4}$ alkoxy, or hydroxy $C_{1-4}$ alkyl; or $R^{51}$ and $R^{52}$ together with the carbon atom to which they are attached form 3-membered cycloalkyl, 4-membered cycloalkyl, 5-membered cycloalkyl, 4-membered heterocycloalkyl, 5-membered heterocycloalkyl, or 6-membered heterocycloalkyl, wherein the cycloalkyl and heterocycloalkyl are optionally further substituted with 1, 2 or 3 substituents selected from $R^A$, and the heterocycloalkyl contains 1-3 heteroatoms selected from N, S and O;

$R^{61}$, $R^{62}$ and $R^{63}$ are each independently selected from H, deuterium, halogen, amino, cyano, hydroxyl, $C_{1-4}$ alkyl, halo $C_{1-4}$ alkyl, deuterated $C_{1-4}$ alkyl, $C_{1-4}$ alkoxy, halo $C_{1-4}$ alkoxy, $-C_{1-4}$ alkyl-3-membered cycloalkyl, $-C_{1-4}$ alkyl-4-membered cycloalkyl, $-C_{1-4}$ alkyl-5-membered cycloalkyl, 3-membered cycloalkyl, 4-membered cycloalkyl, 5-membered cycloalkyl, 4-membered heterocycloalkyl, 5-membered heterocycloalkyl, 6-membered heterocycloalkyl, deuterated $C_{1-4}$ alkoxy, or hydroxy $C_{1-4}$alkyl, wherein the heterocycloalkyl contains 1-3 heteroatoms selected from N, S and O;

or, $R^{31}$ and $R^{41}$, or $R^{41}$ and $R^{51}$ together with the carbon atoms to which they are each attached form 3-membered cycloalkyl, 4-membered cycloalkyl, 5-membered cycloalkyl, 4-membered heterocycloalkyl, 5-membered heterocycloalkyl, or 6-membered heterocycloalkyl, wherein the cycloalkyl and heterocycloalkyl are optionally further substituted with 1, 2 or 3 substituents selected from $R^A$, and the heterocycloalkyl contains 1-3 heteroatoms selected from N, S and O;

or, $R^{41}$ and $R^{61}$ together with the carbon atom(s) to which they are each attached form 4-membered cycloalkyl, 5-membered cycloalkyl, 6-membered cycloalkyl, 4-membered heterocycloalkyl, 5-membered heterocycloalkyl, or 6-membered heterocycloalkyl, wherein the cycloalkyl and heterocycloalkyl are optionally further substituted with 1, 2 or 3 substituents selected from $R^A$, and the heterocycloalkyl contains 1-3 heteroatoms selected from N, S and O;

or, $R^z$ and $R^{41}$ together with the carbon atom(s) to which they are each attached form 4-membered heterocycloalkyl, 5-membered heterocycloalkyl, or 6-membered heterocycloalkyl, wherein the heterocycloalkyl is optionally further substituted with 1, 2 or 3 substituents selected from $R^A$ and contains 1-3 heteroatoms selected from N, S and O;

or, $R^{51}$ and $R^{61}$, or $R^{61}$ and $R^{62}$ together with the carbon atom(s) to which they are each attached form 3-membered cycloalkyl, 4-membered cycloalkyl, 5-membered cycloalkyl, 4-membered heterocycloalkyl, 5-membered heterocycloalkyl, 6-membered heterocycloalkyl, or a double bond, wherein the cycloalkyl and heterocycloalkyl are optionally further substituted with 1, 2 or 3 $R^A$ substituents, and the heterocycloalkyl contains 1-3 heteroatoms selected from N, S and O;

or, $R^{61}$, $R^{62}$ and $R^{63}$ together with the carbon atom to which they are attached form 5-membered bicyclic bridged ring, 6-membered bicyclic bridged ring, 7-membered bicyclic bridged ring, 8-membered bicyclic bridged ring, 5-membered spiro ring, 6-membered spiro ring, 7-membered spiro ring, 8-membered spiro ring, 9-membered spiro ring, or 10-membered spiro ring, wherein the bridged ring and spiro ring are optionally further substituted with 1, 2 or 3 substituents selected from $R^A$, and the heterocycloalkyl contains 1-3 heteroatoms selected from N, S and O;

$R^A$ is selected from deuterium, F, Cl, amino, cyano, hydroxyl, $C_{1-4}$ alkyl, halo $C_{1-4}$ alkyl, deuterated $C_{1-4}$ alkyl, $C_{1-4}$ alkoxy, halo $C_{1-4}$ alkoxy, deuterated $C_{1-4}$ alkoxy, or hydroxy $C_{1-4}$ alkyl;

other groups have the same definitions as those in the preceding technical solutions.

As a sixth technical solution of the present invention, the present invention relates to the compound, or the stereoisomer, deuterated compound, solvate, prodrug, metabolite, pharmaceutically acceptable salt or co-crystal thereof according to the present invention, having a structure of formula (Ib):

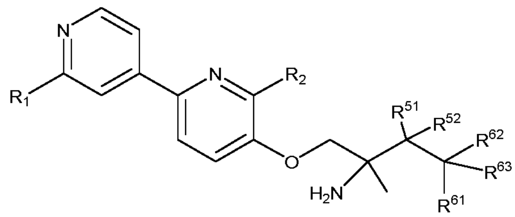 (Ib)

wherein $R^1$ is selected from halo $C_{1-3}$ alkyl, $-NHC(O)C_{1-4}$ alkyl, $-NHC(O)C_{3-6}$ cycloalkyl, $-NHC(O)C_{4-6}$ heterocycloalkyl, $-NHC(O)NHC_{1-4}$ alkyl, or - $NHC(O)OC_{1-4}$ alkyl, wherein the alkyl, cycloalkyl and heterocycloalkyl are optionally further substituted with 1-3 substituents selected from deuterium, F, Cl, amino, cyano, or hydroxyl;

$R^2$ is selected from cyano, halo $C_{1-3}$ alkyl, or deuterated $C_{1-3}$ alkyl;

$R^{51}$ and $R^{52}$ are each independently selected from H or deuterium;

$R^{61}$ is independently selected from H, deuterium, halogen, amino, cyano, hydroxyl, $C_{1-6}$ alkyl, halo $C_{1-6}$ alkyl, deuterated $C_{1-6}$ alkyl, $C_{1-6}$ alkoxy, halo $C_{1-6}$ alkoxy, $-C_{1-6}$ alkyl-$C_{3-6}$ cycloalkyl, $C_{3-6}$ cycloalkyl, 4- to 6-membered heterocycloalkyl containing 1-3 heteroatoms selected from N, S and O, deuterated $C_{1-6}$ alkoxy or hydroxy $C_{1-6}$ alkyl;

$R^{62}$ and $R^{63}$ are each independently selected from halogen, amino, cyano, hydroxyl, $C_{1-6}$ alkyl, halo $C_{1-6}$ alkyl, deuterated $C_{1-6}$ alkyl, $C_{1-6}$ alkoxy, halo $C_{1-6}$ alkoxy, $-C_{1-6}$ alkyl-$C_{3-6}$ cycloalkyl, $C_{3-6}$ cycloalkyl, 4- to 6-membered heterocycloalkyl containing 1-3 heteroatoms selected from N, S and O, deuterated $C_{1-6}$ alkoxy, or hydroxy $C_{1-6}$ alkyl;

or, $R^{61}$ and $R^{62}$ together with the carbon atom(s) to which they are each attached form $C_{3-6}$ cycloalkyl, 4- to 6-membered heterocycloalkyl containing 1-3 heteroatoms selected from N, S and O, or a double bond, wherein the cycloalkyl and heterocycloalkyl are optionally further substituted with 1-3 substituents selected from deuterium, F, Cl, amino, cyano, or hydroxyl.

As a seventh technical solution of the present invention, the present invention relates to the compound of formula (I), (Ia), (Ib) or (II), or the stereoisomer, deuterated compound, solvate, prodrug, metabolite, pharmaceutically acceptable salt or co-crystal thereof according to the present invention, wherein

$R^1$ is selected from halo $C_{1-3}$ alkyl, $-NHC(O)C_{1-4}$ alkyl, $-NHC(O)C_{3-6}$ cycloalkyl, or $-NHC(O)OC_{1-4}$ alkyl, wherein the alkyl, cycloalkyl and heterocycloalkyl are optionally further substituted with 1-3 substituents selected from deuterium, F, Cl, amino, cyano, or hydroxyl; in certain embodiments, $R^1$ is selected from halo $C_{1-3}$ alkyl, or $-NHC(O)OC_{1-4}$ alkyl; in certain embodiments, $R^1$ is selected from $-CH_2F$, $-CHF_2$, $-CF_3$, $-CH_2CH_2F$, $-CH_2CHF_2$, $-CH_2CF_3$, - $CHFCH_2F$, $-CHFCHF_2$, $-CHFCF_3$, $-CF_2CH_2F$, $-CF_2CHF_2$, $-CF_2CF_3$, $-NHC(O)CH_3$, $-NHC(O)OCH_3$

, 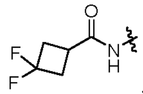 , 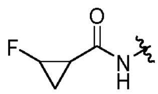 , or 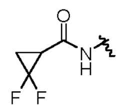 ;

$R^2$ is selected from cyano or halo $C_{1-3}$ alkyl; in certain embodiments, $R^2$ is selected from cyano, $-CH_2F$, $-CHF_2$, $-CF_3$, $-CH_2CH_2F$, $-CH_2CHF_2$, $-CH_2CF_3$, - $CHFCH_2F$, $-CHFCHF_2$, $-CHFCF_3$, $-CF_2CH_2F$, $-CF_2CHF_2$, or $-CF_2CF_3$; in certain embodiments, $R^2$ is selected from cyano or $-CHF_2$; in certain embodiments, $R^2$ is selected from $-CHF_2$;

$R^{51}$ and $R^{52}$ are each independently selected from H or deuterium;

$R^{61}$ is independently selected from H, deuterium, halogen, amino, cyano, hydroxyl, $C_{1-3}$ alkyl, or hydroxy $C_{1-3}$ alkyl; in certain embodiments, $R^{61}$ is independently selected from H, deuterium, F, Cl, amino, cyano, hydroxyl, methyl, ethyl, $-CH_2OH$, or $-CH_2CH_2OH$;

$R^{62}$ and $R^{63}$ are each independently selected from halogen, amino, cyano, hydroxyl, $C_{1-3}$ alkyl, halo $C_{1-3}$ alkyl, deuterated $C_{1-3}$ alkyl, or hydroxy $C_{1-3}$ alkyl; in certain embodiments, $R^{62}$ and $R^{63}$ are each independently selected from F, Cl, amino, cyano, hydroxyl, methyl, ethyl, $-CH_2F$, $-CHF_2$, $-CF_3$, $-CH_2CH_2F$, - $CH_2CHF_2$, $-CH_2CF_3$, $-CHFCH_2F$, $-CHFCHF_2$, $-CHFCF_3$, $-CF_2CH_2F$, $-CF_2CHF_2$, - $CF_2CF_3$, $-CH_2D$, $-CHD_2$, $-CD_3$, $-CH_2CH_2D$, $-CH_2CHD_2$, $-CH_2CD_3$, $-CHDCH_2D$, - $CHDCHD_2$, $-CHDCD_3$, $-CD_2CH_2D$, $-CD_2CHD_2$, $-CD_2CD_3$, $-CH_2OH$, or - $CH_2CH_2OH$;

or, $R^{61}$ and $R^{62}$ together with the carbon atom(s) to which they are each attached form $C_3$, $C_4$, $C_5$, or $C_6$ cycloalkyl or a double bond, wherein the cycloalkyl is optionally further substituted with 1, 2 or 3 substituents selected from deuterium, F, Cl, amino, cyano, or hydroxyl.

As an eighth technical solution of the present invention, the present invention relates to the compound, or the stereoisomer, deuterated compound, solvate, prodrug, metabolite, pharmaceutically acceptable salt or co-crystal thereof

according to the present invention, wherein

Z is selected from $NR^z$ or O;

$R^z$ is selected from H, deuterium, methyl, ethyl, n-propyl, or isopropyl;

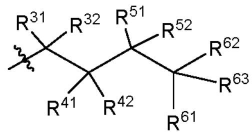

is selected from the following groups:

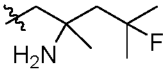 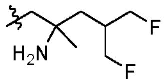 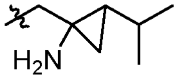 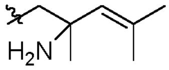 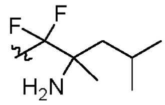

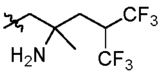 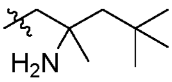 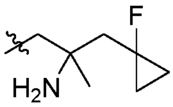 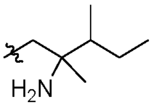 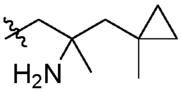

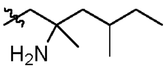 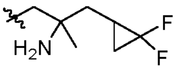 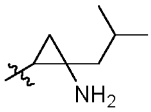 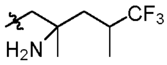 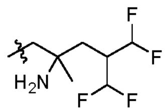

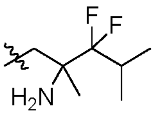 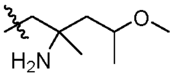 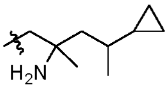 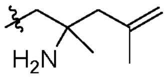 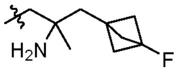

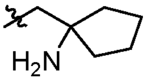 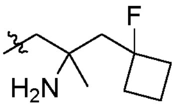 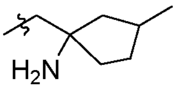 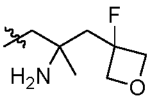 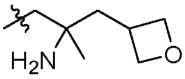

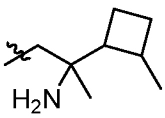 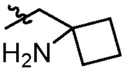 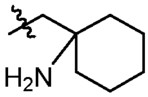 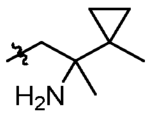 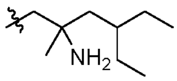

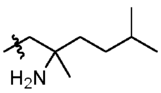 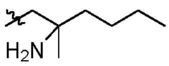 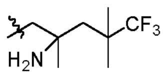 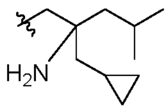 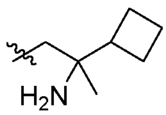

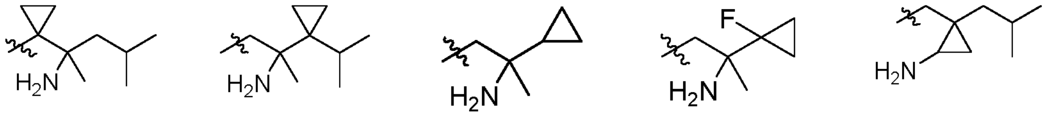

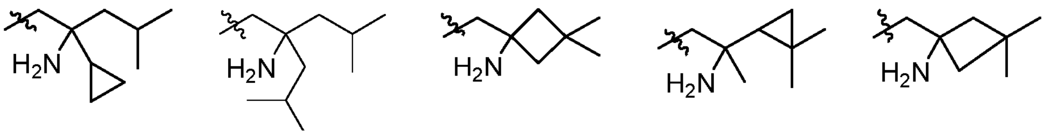

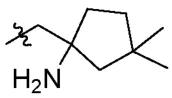 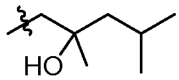 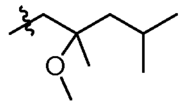 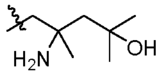 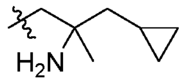

or

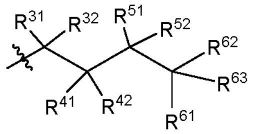

is selected from

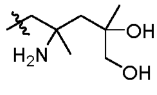 ;

or,

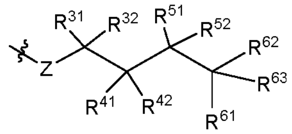

is selected from:

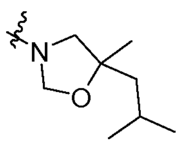 .

For the compound of formula (I) according to the present invention, $X_1$, $X_2$, $X_3$ and $X_4$ are each independently selected from N or $CR^x$; in certain embodiments, $X_1$ is selected from N, and $X_2$, $X_3$ and $X_4$ are each independently selected from N or $CR^x$; in certain embodiments, $X_1$ is selected from N, and $X_2$, $X_3$ and $X_4$ are each independently selected from $CR^x$; in certain embodiments, $X_1$ is selected from $CR^x$, and $X_2$, $X_3$ and $X_4$ are each independently selected from N or $CR^x$; in certain embodiments, $X_2$ is selected from N, and $X_1$, $X_3$ and $X_4$ are each independently selected from N or $CR^x$; in certain embodiments, $X_3$ is selected from N, and $X_1$, $X_2$ and $X_4$ are each independently selected from N or $CR^x$.

For the compound of formula (I) or (Ia) according to the present invention, $Y_1$, $Y_2$ and $Y_3$ are each independently selected from N or $CR^y$; in certain embodiments, $Y_1$ is selected from N, and $Y_2$ and $Y_3$ are each independently selected from N or $CR^y$; in certain embodiments, $Y_1$ is selected from N, and $Y_2$ and $Y_3$ are each independently selected from $CR^y$; in certain embodiments, $Y_2$ is selected from N, and $Y_1$ and $Y_3$ are each independently selected from $CR^y$; in certain embodiments, $Y_3$ is selected from N, and $Y_1$ and $Y_2$ are each independently selected from $CR^y$.

For the compound of formula (I), (Ia) or (II) according to the present invention, Z is selected from $NR^z$ or O; in certain embodiments, Z is selected from O; in certain embodiments, Z is selected from $NR^z$.

For the compound of formula (I), (Ia) or (II) according to the present invention, $R^z$ is selected from H, deuterium, halogen, $C_{1-6}$ alkyl, halo $C_{1-6}$ alkyl, or deuterated $C_{1-6}$ alkyl; in certain embodiments, $R^z$ is selected from H, deuterium, halogen, $C_{1-4}$ alkyl, halo $C_{1-4}$ alkyl, or deuterated $C_{1-4}$ alkyl; in certain embodiments, $R^z$ is selected from H, deuterium, F, Cl, $C_{1-2}$ alkyl, halo $C_{1-2}$ alkyl, or deuterated $C_{1-2}$ alkyl; in certain embodiments, $R^z$ is selected from H, deuterium, F, Cl, methyl, ethyl, $-CH_2F$, $-CHF_2$, $-CF_3$, $-CH_2CH_2F$, $-CH_2CHF_2$, $-CH_2CF_3$, $-CHFCH_2F$, $-CHFCHF_2$, $-CHFCF_3$, $-CF_2CH_2F$, $-CF_2CHF_2$, $-CF_2CF_3$, $-CH_2D$, $-CHD_2$, $-CD_3$, $-CH_2CH_2D$, $-CH_2CHD_2$, $-CH_2CD_3$, $-CHDCH_2D$, $-CHDCHD_2$, $-CHDCD_3$, $-CD_2CH_2D$, $-CD_2CHD_2$, or $-CD_2CD_3$.

For the compound of formula (I) or (Ia) according to the present invention, $R^x$ and $R^y$ are each independently

selected from H, deuterium, halogen, amino, nitro, cyano, hydroxyl, sulphonyl, $C_{1-6}$ alkyl, halo $C_{1-6}$ alkyl, deuterated $C_{1-6}$ alkyl, $C_{1-6}$ alkoxy, halo $C_{1-6}$ alkoxy, deuterated $C_{1-6}$ alkoxy, hydroxy $C_{1-6}$ alkyl, $C_{3-6}$ cycloalkyl, or $C_{4-6}$ heterocycloalkyl; in certain embodiments, $R^x$ and $R^y$ are each independently selected from H, deuterium, halogen, amino, nitro, cyano, hydroxyl, sulphonyl, $C_{1-4}$ alkyl, halo $C_{1-4}$ alkyl, deuterated $C_{1-4}$ alkyl, $C_{1-4}$ alkoxy, halo $C_{1-4}$ alkoxy, deuterated $C_{1-4}$ alkoxy, hydroxy $C_{1-4}$ alkyl, 3-membered cycloalkyl, 4-membered cycloalkyl, 5-membered cycloalkyl, 6-membered cycloalkyl, 4-membered heterocycloalkyl, 5-membered heterocycloalkyl, or 6-membered heterocycloalkyl; in certain embodiments, $R^x$ and $R^y$ are each independently selected from H, deuterium, F, Cl, amino, nitro, cyano, hydroxyl, sulphonyl, $C_{1-2}$ alkyl, halo $C_{1-2}$ alkyl, deuterated $C_{1-2}$ alkyl, $C_{1-2}$ alkoxy, halo $C_{1-2}$ alkoxy, deuterated $C_{1-2}$ alkoxy, hydroxy $C_{1-2}$ alkyl, 3-membered cycloalkyl, 4-membered cycloalkyl, 5-membered cycloalkyl, 6-membered cycloalkyl, 4-membered heterocycloalkyl, 5-membered heterocycloalkyl, or 6-membered heterocycloalkyl, wherein the heterocycloalkyl contains 1-3 heteroatoms selected from N, S and O, and the alky, cycloalkyl and heterocycloalkyl are optionally further substituted with 1-3 $R^A$.

For the compound of formula (I), (Ia), (Ib) or (II) according to the present invention, $R^1$ and $R^2$ are each independently selected from H, deuterium, halogen, amino, -COOH, cyano, sulphonyl, aminoacyl, $C_{1-6}$ alkyl, halo $C_{1-6}$ alkyl, deuterated $C_{1-6}$ alkyl, $C_{1-6}$ alkoxy, halo $C_{1-6}$ alkoxy, deuterated $C_{1-6}$ alkoxy, hydroxy $C_{1-6}$ alkyl, $C_{3-6}$ cycloalkyl, 4- to 6-membered heterocycloalkyl containing 1-3 heteroatoms selected from N, S and O, $-NHC(O)C_{1-6}$ alkyl, $-NHC(O)C_{3-6}$ cycloalkyl, $-NHC(O)C_{4-6}$ heterocycloalkyl, $-NHC(O)NHC_{1-6}$ alkyl, or$-NHC(O)OC_{1-6}$ alkyl, wherein the alkyl, cycloalkyl and heterocycloalkyl are optionally further substituted with 1-3 $R^A$ substituents; in certain embodiments, $R^1$ and $R^2$ are each independently selected from H, deuterium, F, Cl, amino, -COOH, cyano, sulphonyl, aminoacyl, $C_{1-4}$ alkyl, halo $C_{1-4}$ alkyl, deuterated $C_{1-4}$ alkyl, $C_{1-4}$ alkoxy, halo $C_{1-4}$ alkoxy, deuterated $C_{1-4}$ alkoxy, hydroxy $C_{1-4}$ alkyl, 3-membered cycloalkyl, 4-membered cycloalkyl, 5-membered cycloalkyl, 6-membered cycloalkyl, 4-membered heterocycloalkyl, 5-membered heterocycloalkyl, 6-membered heterocycloalkyl, $-NHC(O)C_{1-4}$ alkyl, $-NHC(O)C_{3-6}$ cycloalkyl,$-NHC(O)C_{4-6}$ heterocycloalkyl, $-NHC(O)NHC_{1-4}$ alkyl, or $-NHC(O)OC_{1-4}$ alkyl; in certain embodiments, $R^1$ and $R^2$ are each independently selected from H, deuterium, F, Cl, amino, -COOH, cyano, sulphonyl, aminoacyl, $C_{1-2}$ alkyl, halo $C_{1-2}$ alkyl, deuterated $C_{1-2}$ alkyl, $C_{1-2}$ alkoxy, halo $C_{1-2}$ alkoxy, deuterated $C_{1-2}$ alkoxy, hydroxy $C_{1-2}$ alkyl, 3-membered cycloalkyl, 4-membered cycloalkyl, 5-membered cycloalkyl, 6-membered cycloalkyl, 4-membered heterocycloalkyl, 5-membered heterocycloalkyl, 6-membered heterocycloalkyl, $-NHC(O)C_{1-2}$ alkyl, $-NHC(O)C_{3-4}$ cycloalkyl, $-NHC(O)C_{4-5}$ heterocycloalkyl, $-NHC(O)NHC_{1-2}$ alkyl, or$-NHC(O)OC_{1-2}$ alkyl; in certain embodiments, $R^1$ and $R^2$ are each independently selected from H, deuterium, cyano, halo $C_{1-2}$ alkyl, halo $C_{1-2}$ alkoxy, $-NHC(O)C_{1-2}$ alkyl, $-NHC(O)OC_{1-2}$ alkyl, or $-NHC(O)C_{3-4}$ cycloalkyl; in certain embodiments, $R^1$ and $R^2$ are each independently selected from cyano, halo $C_{1-2}$ alkyl, - $NHC(O)C_{1-2}$ alkyl, $-NHC(O)OC_{1-2}$ alkyl, or $-NHC(O)C_{3-4}$ cycloalkyl; in certain embodiments, $R^1$ and $R^2$ are each independently selected from cyano, $-CH_2F$, - $CHF_2$, $-CF_3$, $-CH_2CH_2F$, $-CH_2CHF_2$, $-CH_2CF_3$, $-CHFCH_2F$, $-CHFCHF_2$, $-CHFCF_3$, $-CF_2CH_2F$, $-CF_2CHF_2$, $-CF_2CF_3$, $-NHC(O)CH_3$, $-NHC(O)OCH_3$,

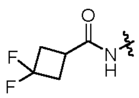

,

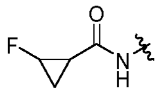

, or

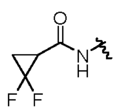

;

in certain embodiments, $R^1$ and $R^2$ are each independently selected from H, deuterium, halogen, amino, -COOH, cyano, sulphonyl, $C_{1-6}$ alkyl, halo $C_{1-6}$ alkyl, deuterated $C_{1-6}$ alkyl, $C_{1-6}$ alkoxy, halo $C_{1-6}$ alkoxy, deuterated $C_{1-6}$ alkoxy, hydroxy $C_{1-6}$ alkyl, $C_{3-6}$ cycloalkyl, $C_{4-6}$ heterocycloalkyl, $-NHC(O)C_{1-6}$ alkyl, or $-NHC(O)OC_{1-6}$ alkyl; in certain embodiments, $R^1$ and $R^2$ are each independently selected from H, deuterium, F, Cl, amino, -COOH, cyano, sulphonyl, $C_{1-4}$ alkyl, halo $C_{1-4}$ alkyl, deuterated $C_{1-4}$ alkyl, $C_{1-4}$ alkoxy, halo $C_{1-4}$ alkoxy, deuterated $C_{1-4}$ alkoxy, hydroxy $C_{1-4}$ alkyl, 3-membered cycloalkyl, 4-membered cycloalkyl, 5-membered cycloalkyl, 6-membered cycloalkyl, 4-membered heterocycloalkyl, 5-membered heterocycloalkyl, 6-membered heterocycloalkyl, $-NHC(O)C_{1-4}$ alkyl, or$-NHC(O)OC_{1-4}$ alkyl; in certain embodiments, $R^1$ and $R^2$ are each independently selected from H, deuterium, F, Cl, amino, -COOH, cyano, sulphonyl, $C_{1-2}$

alkyl, halo $C_{1-2}$ alkyl, deuterated $C_{1-2}$ alkyl, $C_{1-2}$ alkoxy, halo $C_{1-2}$ alkoxy, deuterated $C_{1-2}$ alkoxy, hydroxy $C_{1-2}$ alkyl, 3-membered cycloalkyl, 4-membered cycloalkyl, 5-membered cycloalkyl, 6-membered cycloalkyl, 4-membered heterocycloalkyl, 5-membered heterocycloalkyl, 6-membered heterocycloalkyl, -NHC(O)$C_{1-2}$ alkyl, or -NHC(O)O$C_{1-2}$ alkyl; in certain embodiments, $R^1$ and $R^2$ are each independently selected from H, deuterium, halo $C_{1-2}$ alkyl, or halo $C_{1-2}$ alkoxy; in certain embodiments, $R^1$ and $R^2$ are each independently selected from halo $C_{1-2}$ alkyl; in certain embodiments, $R^1$ and $R^2$ are each independently selected from $-CH_2F$,$-CHF_2$, $-CF_3$, $-CH_2CH_2F$, $-CH_2CHF_2$, $-CH_2CF_3$, $-CHFCH_2F$, $-CHFCHF_2$, $-CHFCF_3$, $-CF_2CH_2F$, $-CF_2CHF_2$, or $-CF_2CF_3$, wherein the heterocycloalkyl contains 1-3 heteroatoms selected from N, S and O, wherein the alkyl, cycloalkyl and heterocycloalkyl are optionally further substituted with 1-3 $R^A$.

For the compound of (I), (Ia), (Ib) or (II) according to the present invention, $R^1$ is selected from sulphonyl, aminoacyl, halo $C_{1-6}$ alkyl, -NHC(O)$C_{1-6}$ alkyl, - NHC(O)$C_{3-6}$ cycloalkyl, -NHC(O)$C_{4-6}$ heterocycloalkyl, -NHC(O)NH$C_{1-6}$ alkyl, or -NHC(O)O$C_{1-6}$ alkyl, wherein the alkyl, cycloalkyl and heterocycloalkyl are optionally further substituted with 1-3 $R^A$ substituents; in certain embodiments, $R^1$ is selected from sulphonyl, aminoacyl, halo $C_{1-3}$ alkyl, -NHC(O)$C_{1-4}$ alkyl,-NHC(O)$C_{3-6}$ cycloalkyl, -NHC(O)$C_{4-6}$ heterocycloalkyl, -NHC(O)NH$C_{1-4}$ alkyl, or -NHC(O)O$C_{1-4}$ alkyl, wherein the alkyl, cycloalkyl and heterocycloalkyl are optionally further substituted with 1, 2 or 3 $R^A$ substituents; in certain embodiments, $R^1$ is selected from sulphonyl, aminoacyl, halo $C_{1-2}$ alkyl,-NHC(O)$C_{1-2}$ alkyl, -NHC(O)$C_{3-4}$ cycloalkyl, -NHC(O)$C_{4-5}$ heterocycloalkyl,-NHC(O)NH$C_{1-2}$ alkyl, or -NHC(O)O$C_{1-2}$ alkyl; in certain embodiments, $R^1$ is selected from halo $C_{1-2}$ alkyl, -NHC(O)$C_{1-2}$ alkyl, -NHC(O)O$C_{1-2}$ alkyl, or-NHC(O)$C_{3-4}$ cycloalkyl; in certain embodiments, $R^1$ is selected from $-CH_2F$,$-CHF_2$, $-CF_3$, $-CH_2CH_2F$, $-CH_2CHF_2$, $-CH_2CF_3$, $-CHFCH_2F$, $-CHFCHF_2$, $-CHFCF_3$, $-CF_2CH_2F$, $-CF_2CHF_2$, $-CF_2CF_3$, $-NHC(O)CH_3$, $-NHC(O)OCH_3$,

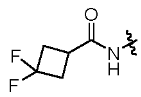

,

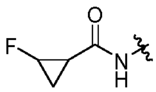

,

or

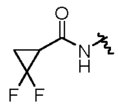

.

For the compound of formula (I), (Ia), (Ib), or (II) according to the present invention, $R^2$ is selected from cyano, $C_{1-6}$ alkyl, halo $C_{1-6}$ alkyl, deuterated $C_{1-6}$ alkyl, $C_{1-6}$ alkoxy, halo $C_{1-6}$ alkoxy, deuterated $C_{1-6}$ alkoxy, hydroxy $C_{1-6}$ alkyl, $C_{3-6}$ cycloalkyl, 4- to 6-membered heterocycloalkyl containing 1-3 heteroatoms selected from N, S and O, wherein the alkyl, cycloalkyl and heterocycloalkyl are optionally further substituted with 1-3 $R^A$ substituents; in certain embodiments, $R^2$ is selected from cyano, $C_{1-4}$ alkyl, halo $C_{1-4}$ alkyl, or deuterated $C_{1-4}$ alkyl; in certain embodiments, $R^2$ is selected from cyano, $C_{1-2}$ alkyl, halo $C_{1-2}$ alkyl, or deuterated $C_{1-2}$ alkyl; in certain embodiments, $R^2$ is selected from cyano, $C_{1-2}$ alkyl, or halo $C_{1-2}$ alkyl; in certain embodiments, $R^2$ is selected from cyano, $-CH_3$,$-CH_2CH_3$, $-CH_2F$, $-CHF_2$, $-CF_3$, $-CH_2CH_2F$, $-CH_2CHF_2$, $-CH_2CF_3$, $-CHFCH_2F$, - $CHFCHF_2$, $-CHFCF_3$, $-CF_2CH_2F$, $-CF_2CHF_2$, or $-CF_2CF_3$.

For the compound of formula (I), (Ia) or (II) according to the present invention, $R^{31}$ and $R^{32}$ are each independently selected from H, deuterium, halogen, cyano, hydroxyl, $C_{1-6}$ alkyl, halo $C_{1-6}$ alkyl, deuterated $C_{1-6}$ alkyl, $C_{1-6}$ alkoxy, halo $C_{1-6}$ alkoxy, deuterated $C_{1-6}$ alkoxy, or hydroxy $C_{1-6}$ alkyl; or $R^{31}$ and $R^{32}$ together with the carbon atom to which they are attached form $C_{3-6}$ cycloalkyl or 4- to 6-membered heterocycloalkyl, wherein the cycloalkyl and heterocycloalkyl are optionally further substituted with 1-3 $R^A$ substituents; in certain embodiments, $R^{31}$ and $R^{32}$ are each independently selected from H, deuterium, F, Cl, cyano, hydroxyl, $C_{1-4}$ alkyl, halo $C_{1-4}$ alkyl, deuterated $C_{1-4}$ alkyl, $C_{1-4}$ alkoxy, halo $C_{1-4}$ alkoxy, deuterated $C_{1-4}$ alkoxy, or hydroxy $C_{1-4}$ alkyl, or $R^{31}$ and $R^{32}$ together with the carbon atom to which they are attached form 3-membered cycloalkyl, 4-membered cycloalkyl, 5-membered cycloalkyl, 6-membered cycloalkyl, 4-membered heterocycloalkyl, 5-membered heterocycloalkyl, or 6-membered heterocycloalkyl, wherein the cycloalkyl and heterocycloalkyl are optionally further substituted with 1, 2 or 3 $R^A$ substituents; in certain embodiments, $R^{31}$ and $R^{32}$ are each independently selected from H, deuterium, F, Cl, hydroxyl, $C_{1-2}$ alkyl, halo $C_{1-2}$ alkyl, deuterated $C_{1-2}$ alkyl, $C_{1-2}$ alkoxy, halo $C_{1-2}$ alkoxy, deuterated $C_{1-2}$ alkoxy, or hydroxy $C_{1-2}$ alkyl, or $R^{31}$ and $R^{32}$ together with the carbon atom to which they are attached form 3-membered cycloalkyl, 4-membered cycloalkyl, or 4-membered heterocycloalkyl,

wherein the cycloalkyl and heterocycloalkyl are optionally further substituted with 1, 2 or 3 $R^A$ substituents, and the heterocycloalkyl contains 1-3 heteroatoms selected from N, S and O.

For the compound of formula (I), (Ia) or (II) according to the present invention, $R^{41}$ and $R^{42}$ are each independently selected from H, deuterium, amino, $C_{1-6}$ alkyl, halogen, cyano, hydroxyl, halo $C_{1-6}$ alkyl, deuterated $C_{1-6}$ alkyl, $C_{1-6}$ alkoxy, halo $C_{1-6}$ alkoxy, -$C_{1-6}$ alkyl-$C_{3-6}$ cycloalkyl, $C_{3-6}$ cycloalkyl, $C_{4-6}$ heterocycloalkyl, deuterated $C_{1-6}$ alkoxy, or hydroxy $C_{1-6}$ alkyl; in certain embodiments, $R^{41}$ and $R^{42}$ are each independently selected from H, deuterium, amino, $C_{1-4}$ alkyl, halogen, cyano, hydroxyl, halo $C_{1-4}$ alkyl, deuterated $C_{1-6}$ alkyl, $C_{1-4}$ alkoxy, halo $C_{1-4}$ alkoxy, -$C_{1-4}$ alkyl-3-membered cycloalkyl, -$C_{1-4}$ alkyl-4-membered cycloalkyl, -$C_{1-4}$ alkyl-5-membered cycloalkyl, 3-membered cycloalkyl, 4-membered cycloalkyl, 5-membered cycloalkyl, 6-membered cycloalkyl, 4-membered heterocycloalkyl, 5-membered heterocycloalkyl, 6-membered heterocycloalkyl, deuterated $C_{1-4}$ alkoxy, or hydroxy $C_{1-4}$ alkyl; in certain embodiments, $R^{41}$ is selected from H, deuterium, amino, $C_{1-4}$ alkyl, halogen, cyano, hydroxyl, halo $C_{1-4}$ alkyl, deuterated $C_{1-6}$ alkyl, $C_{1-4}$ alkoxy, halo $C_{1-4}$ alkoxy, -$C_{1-4}$ alkyl-3-membered cycloalkyl, -$C_{1-4}$ alkyl-4-membered cycloalkyl,-$C_{1-4}$ alkyl-5-membered cycloalkyl, 3-membered cycloalkyl, 4-membered cycloalkyl, 5-membered cycloalkyl, 6-membered cycloalkyl, 4-membered heterocycloalkyl, 5-membered heterocycloalkyl, 6-membered heterocycloalkyl, deuterated $C_{1-4}$ alkoxy, or hydroxy $C_{1-4}$ alkyl; in certain embodiments, $R^{41}$ is selected from H, deuterium, amino, $C_{1-4}$ alkyl, halogen, cyano, hydroxyl, halo $C_{1-2}$ alkyl, deuterated $C_{1-2}$ alkyl, $C_{1-2}$ alkoxy, halo $C_{1-2}$ alkoxy, -$C_{1-2}$ alkyl-3-membered cycloalkyl, -$C_{1-2}$ alkyl-4-membered cycloalkyl, -$C_{1-2}$ alkyl-5-membered cycloalkyl, 3-membered cycloalkyl, 4-membered cycloalkyl, 5-membered cycloalkyl, 6-membered cycloalkyl, 4-membered heterocycloalkyl, 5-membered heterocycloalkyl, 6-membered heterocycloalkyl, deuterated $C_{1-2}$ alkoxy, or hydroxy $C_{1-2}$ alkyl; in certain embodiments, $R^{42}$ is selected from H, hydroxyl, amino, $C_{1-2}$ alkyl, deuterated $C_{1-2}$ alkyl, or $C_{1-2}$ alkoxy; in certain embodiments, $R^{42}$ is selected from amino, and the heterocycloalkyl contains 1-3 heteroatoms selected from N, S and O.

For the compound of formula (I), (Ia) or (II) according to the present invention, $R^{41}$ and $R^{42}$ together form $C_{3-6}$ cycloalkyl or 4- to 6-membered heterocycloalkyl containing 1 heteroatom selected from O or S, wherein the cycloalkyl and heterocycloalkyl are optionally further substituted with 1-3 $R^A$ substituents; in certain embodiments, $R^{41}$ and $R^{42}$ together form 3-membered cycloalkyl, 4-membered cycloalkyl, 5-membered cycloalkyl, 6-membered cycloalkyl, or 4-membered heterocycloalkyl, 5-membered heterocycloalkyl, or 6-membered heterocycloalkyl containing 1 heteroatom selected from O or S, wherein the cycloalkyl and heterocycloalkyl are optionally further substituted with 1, 2 or 3 $R^A$ substituents.

For the compound of formula (I), (Ia), (Ib), or (II) according to the present invention, $R^{51}$ and $R^{52}$ are each independently selected from H, deuterium, amino, halogen, $C_{1-6}$ alkyl, cyano, hydroxyl, halo $C_{1-6}$ alkyl, deuterated $C_{1-6}$ alkyl, $C_{1-6}$ alkoxy, halo $C_{1-6}$ alkoxy, -$C_{1-6}$ alkyl-$C_{3-6}$ cycloalkyl, $C_{3-6}$ cycloalkyl, $C_{4-6}$ heterocycloalkyl, deuterated $C_{1-6}$ alkoxy, or hydroxy $C_{1-6}$ alkyl; or $R^{51}$ and $R^{52}$ together with the carbon atom to which they are attached form $C_{3-6}$ cycloalkyl or 4-to 6-membered heterocycloalkyl, wherein the cycloalkyl and heterocycloalkyl are optionally further substituted with 1-3 $R^A$ substituents; in certain embodiments, $R^{51}$ and $R^{52}$ are each independently selected from H, deuterium, amino, F, Cl, $C_{1-4}$ alkyl, cyano, hydroxyl, halo $C_{1-4}$ alkyl, deuterated $C_{1-4}$ alkyl, $C_{1-4}$ alkoxy, halo $C_{1-4}$ alkoxy, -$C_{1-4}$ alkyl-3-membered cycloalkyl, -$C_{1-4}$ alkyl-4-membered cycloalkyl,-$C_{1-4}$ alkyl-5-membered cycloalkyl, 3-membered cycloalkyl, 4-membered cycloalkyl, 5-membered cycloalkyl, 6-membered cycloalkyl, 4-membered heterocycloalkyl, 5-membered heterocycloalkyl, 6-membered heterocycloalkyl, deuterated $C_{1-4}$ alkoxy, or hydroxy $C_{1-4}$ alkyl; or $R^{51}$ and $R^{52}$ together with the carbon atom to which they are attached form 3-membered cycloalkyl, 4-membered cycloalkyl, 5-membered cycloalkyl, 6-membered cycloalkyl, 4-membered heterocycloalkyl, 5-membered heterocycloalkyl, or 6-membered heterocycloalkyl, wherein the cycloalkyl and heterocycloalkyl are optionally further substituted with 1, 2 or 3 $R^A$ substituents; in certain embodiments, $R^{51}$ and $R^{52}$ are each independently selected from H, deuterium, amino, F, Cl, $C_{1-4}$ alkyl, cyano, hydroxyl, halo $C_{1-2}$ alkyl, deuterated $C_{1-2}$ alkyl, $C_{1-2}$ alkoxy, halo $C_{1-2}$ alkoxy, -$C_{1-2}$ alkyl -3-membered cycloalkyl, -$C_{1-2}$ alkyl-4-membered cycloalkyl, -$C_{1-2}$ alkyl-5-membered cycloalkyl, 3-membered cycloalkyl, 4-membered cycloalkyl, 5-membered cycloalkyl, 6-membered cycloalkyl, 4-membered heterocycloalkyl, 5-membered heterocycloalkyl, 6-membered heterocycloalkyl, deuterated $C_{1-2}$ alkoxy, or hydroxy $C_{1-2}$ alkyl, or $R^{51}$ and $R^{52}$ together with the carbon atom to which they are attached form 3-membered cycloalkyl, 4-membered cycloalkyl, 4-membered heterocycloalkyl, or 5-membered heterocycloalkyl, wherein the cycloalkyl and heterocycloalkyl are optionally further substituted with 1, 2 or 3 $R^A$ substituents; in certain embodiments, $R^{51}$ and $R^{52}$ are each independently selected from H, deuterium, amino, F, Cl, $C_{1-4}$ alkyl, cyano, hydroxyl, halo $C_{1-2}$ alkyl, or $R^{51}$ and $R^{52}$ together with the carbon atom to which they are attached form 3-membered cycloalkyl.

For the compound of formula (I), (Ia), (Ib), or (II) according to the present invention, $R^{61}$, $R^{62}$ and $R^{63}$ are each independently selected from H, deuterium, halogen, amino, cyano, hydroxyl, $C_{1-6}$ alkyl, halo $C_{1-6}$ alkyl, deuterated $C_{1-6}$ alkyl, $C_{1-6}$ alkoxy, halo $C_{1-6}$ alkoxy, -$C_{1-6}$ alkyl-$C_{3-6}$ cycloalkyl, $C_{3-6}$ cycloalkyl, $C_{4-6}$ heterocycloalkyl, deuterated $C_{1-6}$ alkoxy, or hydroxy $C_{1-6}$ alkyl; in certain embodiments, $R^{61}$, $R^{62}$ and $R^{63}$ are each independently selected from H, deuterium, F, Cl, amino, cyano, hydroxyl, $C_{1-4}$ alkyl, halo $C_{1-4}$ alkyl, deuterated $C_{1-4}$ alkyl, $C_{1-4}$ alkoxy, halo $C_{1-4}$ alkoxy, -$C_{1-4}$ alkyl-3-membered cycloalkyl, -$C_{1-4}$ alkyl-4-membered cycloalkyl, -$C_{1-4}$ alkyl-5-membered cycloalkyl, 3-membered cycloalkyl, 4-membered cycloalkyl, 5-membered cycloalkyl, 6-membered cycloalkyl, 4-membered heterocycloalkyl, 5-membered heterocycloalkyl, 6-membered heterocycloalkyl, deuterated $C_{1-4}$ alkoxy, or hydroxy $C_{1-4}$ alkyl; in certain embodiments, $R^{61}$, $R^{62}$ and $R^{63}$ are each independently selected from H, deuterium, F, Cl, amino, cyano, hydroxyl, $C_{1-2}$ alkyl, halo $C_{1-2}$ alkyl, deuterated $C_{1-2}$ alkyl, $C_{1-2}$ alkoxy, halo $C_{1-2}$ alkoxy, -$C_{1-2}$ alkyl-3-membered cycloalkyl, -$C_{1-2}$ alkyl-4-membered cycloalkyl, -$C_{1-2}$ alkyl-5-membered cycloalkyl, 3-membered cycloalkyl, 4-membered cycloalkyl, 5-membered cycloalkyl, 6-membered cycloalkyl, 4-membered heterocycloalkyl, 5-membered heterocycloalkyl, 6-membered heterocycloalkyl, deuterated $C_{1-2}$ alkoxy, or hydroxy $C_{1-2}$ alkyl; in certain embodiments, $R^{61}$, $R^{62}$ and $R^{63}$ are each independently selected from H, deuterium, F, Cl, amino, cyano, hydroxyl, methyl, ethyl, $-CH_2F$, $-CHF_2$, $-CF_3$, $-CH_2CH_2F$,$-CH_2CHF_2$, $-CH_2CF_3$, $-CHFCH_2F$, $-CHFCHF_2$, $-CHFCF_3$, $-CF_2CH_2F$, $-CF_2CHF_2$,$-CF_2CF_3$, $-CH_2D$, $-CHD_2$, $-CD_3$, $-CH_2CH_2D$, $-CH_2CHD_2$, $-CH_2CD_3$, $-CHDCH_2D$,$-CHDCHD_2$, $-CHDCD_3$, $-CD_2CH_2D$, $-CD_2CHD_2$, $-CD_2CD_3$, methoxy, ethoxy,$-OCHF_2$, $-OCH_2F$, $-OCF_3$, $-OCH_2CH_2F$, $-OCH_2CHF_2$, $-OCH_2CF_3$, $-OCHFCH_2F$,$-OCHFCHF_2$, $-OCHFCF_3$, $-OCF_2CH_2F$, $-OCF_2CHF_2$, $-OCF_2CF_3$, -methyl-3-membered cycloalkyl, -ethyl-3-membered cycloalkyl, -methyl-4-membered cycloalkyl, -ethyl-4-membered cycloalkyl, -methyl-5-membered cycloalkyl, -ethyl-5-membered cycloalkyl, 3-membered cycloalkyl, 4-membered cycloalkyl, 5-membered cycloalkyl, 6-membered cycloalkyl, 4-membered heterocycloalkyl, 5-membered heterocycloalkyl, 6-membered heterocycloalkyl, $-OCHD_2$, $-OCH_2D$,$-OCD_3$, $-OCH_2CH_2D$, $-OCH_2CHD_2$, $-OCH_2CD_3$, $-OCHDCH_2D$, $-OCHDCHD_2$,$-OCHDCD_3$, $-OCD_2CH_2D$, $-OCD_2CHD_2$, $-OCD_2CD_3$, $-CH_2OH$, or $-CH_2CH_2OH$, wherein the heterocycloalkyl contains 1-3 heteroatoms selected from N, S and O.

For the compound of (I), (Ia) or (II) according to the present invention, $R^{31}$ and $R^{41}$, or $R^{41}$ and $R^{51}$ together with the carbon atoms to which they are each attached form $C_{3-6}$ cycloalkyl or 4- to 6-membered heterocycloalkyl, wherein the cycloalkyl and heterocycloalkyl are optionally further substituted with 1-3 $R^A$ substituents; in certain embodiments, $R^{31}$ and $R^{41}$, or $R^{41}$ and $R^{51}$ together with the carbon atoms to which they are each attached form 3-membered cycloalkyl, 4-membered cycloalkyl, 5-membered cycloalkyl, 6-membered cycloalkyl, 4-membered heterocycloalkyl, 5-membered heterocycloalkyl, or 6-membered heterocycloalkyl, wherein the cycloalkyl and heterocycloalkyl are optionally further substituted with 1, 2 or 3 $R^A$ substituents, and the heterocycloalkyl contains 1-3 heteroatoms selected from N, S and O.

For the compound of (I), (Ia) or (II) according to the present invention, $R^{41}$ and $R^{61}$, or $R^z$ and $R^{41}$ together with the atoms to which they are each attached form $C_{4-6}$ cycloalkyl or 4- to 6-membered heterocycloalkyl, wherein the cycloalkyl and heterocycloalkyl are optionally further substituted with 1-3 $R^A$ substituents; in certain embodiments, $R^{41}$ and $R^{61}$, or $R^z$ and $R^{41}$ together with the atoms to which they are each attached form 4-membered cycloalkyl, 5-membered cycloalkyl, 6-membered cycloalkyl, 4-membered heterocycloalkyl, 5-membered heterocycloalkyl, or 6-membered heterocycloalkyl, wherein the cycloalkyl and heterocycloalkyl are optionally further substituted with 1, 2 or 3 $R^A$ substituents, and the heterocycloalkyl contains 1-3 heteroatoms selected from N, S and O.

For the compound of formula (I), (Ia), (Ib), or (II) according to the present invention, $R^{51}$ and $R^{61}$, or $R^{61}$ and $R^{62}$ together with the carbon atom(s) to which they are each attached form $C_{3-6}$ cycloalkyl, 4- to 6-membered heterocycloalkyl, or a double bond, wherein the cycloalkyl and heterocycloalkyl are optionally further substituted with 1-3 $R^A$ substituents; in certain embodiments, $R^{51}$ and $R^{61}$, or $R^{61}$ and $R^{62}$ together with the carbon atom(s) to which they are each attached form 3-membered cycloalkyl, 4-membered cycloalkyl, 5-membered cycloalkyl, 6-membered cycloalkyl, 4-membered heterocycloalkyl, 5-membered heterocycloalkyl, 6-membered heterocycloalkyl, or a double bond, wherein the cycloalkyl and heterocycloalkyl are optionally further substituted with 1, 2 or 3 $R^A$ substituents, and the heterocycloalkyl contains 1-3 heteroatoms selected from N, S and O.

For the compound of formula (I), (Ia), (Ib), or (II) according to the present invention, $R^{61}$, $R^{62}$ and $R^{63}$ together with the carbon atom to which they are attached form $C_{5-10}$ bridged ring, or $C_{5-11}$ spiro ring, wherein the bridged ring and spiro ring are optionally further substituted with 1-3 $R^A$ substituents; in certain embodiments, $R^{61}$, $R^{62}$ and $R^{63}$ together with the carbon atom to which they are attached form 5-membered bridged ring, 6-membered bridged ring, 7-membered bridged ring, 8-membered bridged ring, 5-membered spiro ring, 6-membered spiro ring, 7-membered spiro ring, 8-membered spiro ring, 9-membered spiro ring, 10-membered spiro ring, or 11-membered spiro ring, wherein the bridged ring and spiro ring are optionally further substituted with 1, 2 or 3 $R^A$ substituents; in certain embodiments, $R^{61}$, $R^{62}$ and $R^{63}$ together with the carbon atom to which they are attached form 5-membered saturated carbocyclic bridged ring, 6-membered saturated carbocyclic bridged ring, 7-membered saturated carbocyclic bridged ring, 8-membered saturated carbocyclic bridged ring, 3-membered carbocycle-spiro-3-membered carbocyclyl, 3-membered carbocycle-spiro-4-membered carbocyclyl, 3-membered carbocycle-spiro-5-membered carbocyclyl, 3-membered carbocycle-spiro-6-membered carbocyclyl, 4-membered carbocycle-spiro-3-membered carbocyclyl, 4-membered carbocycle-spiro-4-membered carbocyclyl, 4-membered carbocycle-spiro-5-membered carbocyclyl, 4-membered carbocycle-spiro-6-membered carbocyclyl, 5-membered carbocycle-spiro-3-membered carbocyclyl, 5-membered carbocycle-spiro-4-membered carbocyclyl, 5-membered carbocycle-spiro-5-membered carbocyclyl, 5-membered carbocycle-spiro-6-membered carbocyclyl, 6-membered carbocycle-spiro-3-membered carbocyclyl, 6-membered carbocycle-spiro-4-membered carbocyclyl, 6-membered carbocycle-spiro-5-membered carbocyclyl, 6-membered carbocycle-spiro-6-membered carbocyclyl, 3-membered carbocycle-spiro-3-membered heterocyclyl, 3-membered carbocycle-spiro-4-membered heterocyclyl, 3-membered carbocycle-spiro-5-membered heterocyclyl, 3-membered carbocycle-spiro-6-membered heterocyclyl, 4-membered carbocycle-spiro-3-membered heterocyclyl, 4-membered carbocycle-spiro-4-membered heterocyclyl, 4-membered carbocycle-spiro-5-membered heterocyclyl, 4-membered carbocycle-spiro-6-membered heterocyclyl, 5-membered carbocycle-spiro-3-membered heterocyclyl, 5-membered carbocycle-spiro-4-membered heterocyclyl, 5-membered carbocycle-spiro-5-membered heterocyclyl, 5-membered carbocycle-spiro-6-membered heterocyclyl, 6-membered carbocycle-spiro-3-membered heterocyclyl, 6-membered carbocycle-spiro-4-membered heterocyclyl, 6-membered carbocycle-spiro-5-membered heterocyclyl, 6-membered carbocycle-spiro-6-membered heterocyclyl, 3-membered heterocycle-spiro-3-membered carbocyclyl, 3-membered heterocycle-spiro-4-membered carbocyclyl, 3-membered heterocycle-spiro-5-membered carbocyclyl, 3-membered heterocycle-spiro-6-membered carbocyclyl, 4-membered heterocycle-spiro-3-membered carbocyclyl, 4-membered heterocycle-spiro-4-membered carbocyclyl, 4-membered heterocycle-spiro-5-membered carbocyclyl, 4-membered heterocycle-spiro-6-membered carbocyclyl, 5-membered heterocycle-spiro-3-membered carbocyclyl, 5-membered heterocycle-spiro-4-membered carbocyclyl, 5-membered heterocycle-spiro-5-membered carbocyclyl, 5-membered heterocycle-spiro-6-membered carbocyclyl, 6-membered heterocycle-spiro-3-membered carbocyclyl, 6-membered heterocycle-spiro-4-membered carbocyclyl, 6-membered heterocycle-spiro-5-membered carbocyclyl, 6-membered heterocycle-spiro-6-membered carbocyclyl, 3-membered heterocycle-spiro-3-membered heterocyclyl, 3-membered heterocycle-spiro-4-membered heterocyclyl, 3-membered heterocycle-spiro-5-membered heterocyclyl, 3-membered heterocycle-spiro-6-membered heterocyclyl, 4-membered heterocycle-spiro-3-membered heterocyclyl, 4-membered heterocycle-spiro-4-membered heterocyclyl, 4-membered heterocycle-spiro-5-membered heterocyclyl, 4-membered heterocycle-spiro-6-membered heterocyclyl, 5-membered heterocycle-spiro-3-membered heterocyclyl, 5-membered heterocycle-spiro-4-membered heterocyclyl, 5-membered heterocycle-spiro-5-membered heterocyclyl, 5-membered heterocycle-spiro-6-membered heterocyclyl, 6-membered heterocycle-spiro-3-membered heterocyclyl, 6-membered heterocycle-spiro-4-membered heterocyclyl, 6-membered heterocycle-spiro-5-membered heterocyclyl, or 6-membered heterocycle-spiro-6-membered heterocyclyl; the carbocyclyl and heterocyclyl are optionally further substituted with 1, 2 or 3 $R^A$ substituents, and the heterocycloalkyl contains 1-3 heteroatoms selected from N, S and O.

For the compound of formula (I), (Ia), (Ib), or (II) according to the present invention, $R^A$ is selected from deuterium, halogen, amino, cyano, hydroxyl, $C_{1-6}$ alkyl, halo $C_{1-6}$ alkyl, deuterated $C_{1-6}$ alkyl, $C_{1-6}$ alkoxy, halo $C_{1-6}$ alkoxy, deuterated $C_{1-6}$ alkoxy, or hydroxy $C_{1-6}$ alkyl, in certain embodiments, $R^A$ is selected from deuterium, F, Cl, amino, cyano, hydroxyl, $C_{1-4}$ alkyl, halo $C_{1-4}$ alkyl, deuterated $C_{1-4}$ alkyl, $C_{1-4}$ alkoxy, halo $C_{1-4}$ alkoxy, deuterated $C_{1-4}$ alkoxy, or hydroxy $C_{1-4}$alkyl; in certain embodiments, $R^A$ is selected from deuterium, F, Cl, amino, cyano, hydroxyl, $C_{1-2}$ alkyl, halo $C_{1-2}$ alkyl, deuterated $C_{1-2}$ alkyl, $C_{1-2}$ alkoxy, halo $C_{1-2}$ alkoxy, deuterated $C_{1-2}$ alkoxy, or hydroxy $C_{1-2}$alkyl.

For the compound of formula (I), (Ia) or (II) according to the present invention, when Z is selected from O

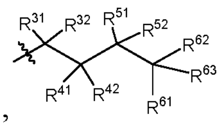
,

does not form the following structures:

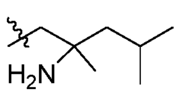
and
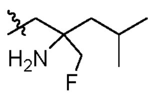
.

The present invention provides the compound of formula (I), (Ia), (Ib), or (II), or the stereoisomer, deuterated compound, solvate, prodrug, metabolite, pharmaceutically acceptable salt or co-crystal thereof, wherein the compound has a structure selected from one of the following:

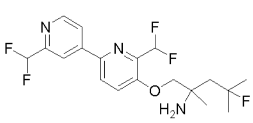
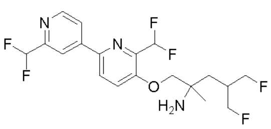
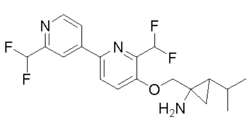
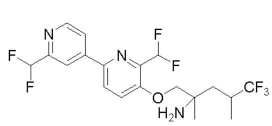

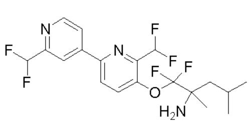
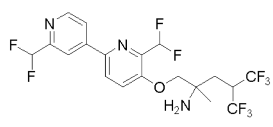
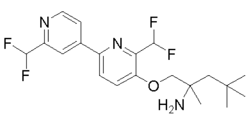
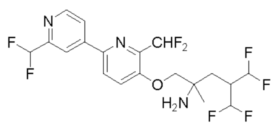

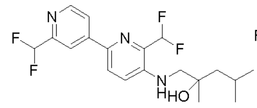
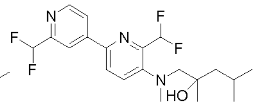
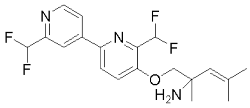
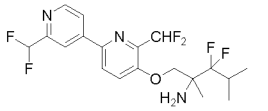

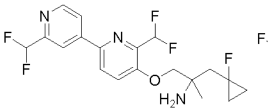
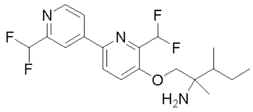
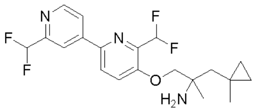
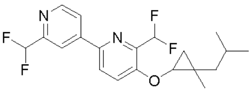

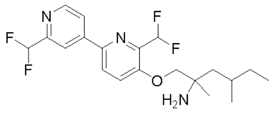
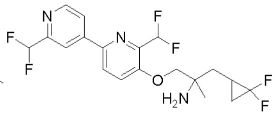
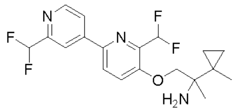
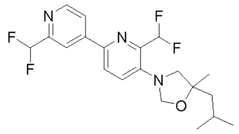

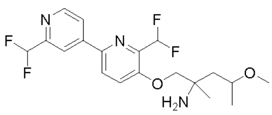
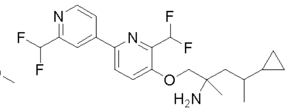
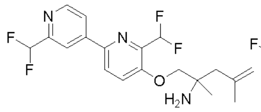
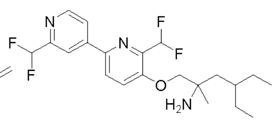

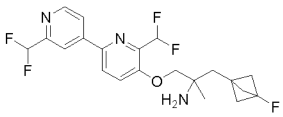
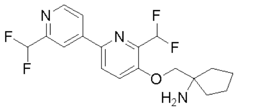
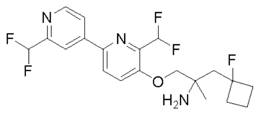
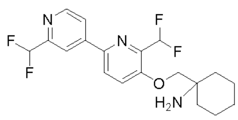

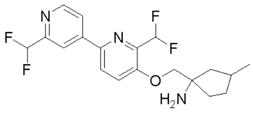
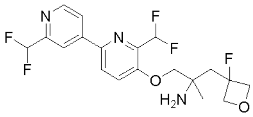
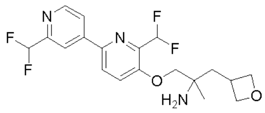
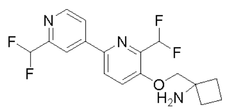

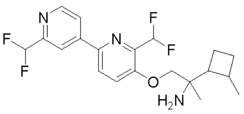
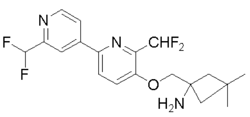
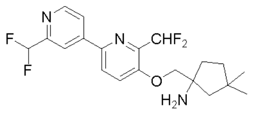
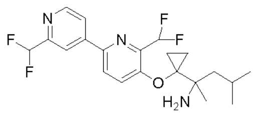

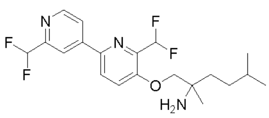
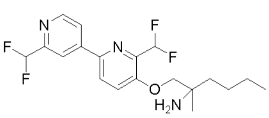
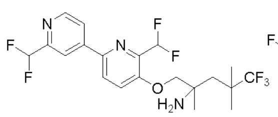
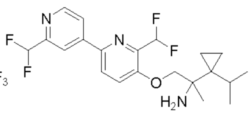

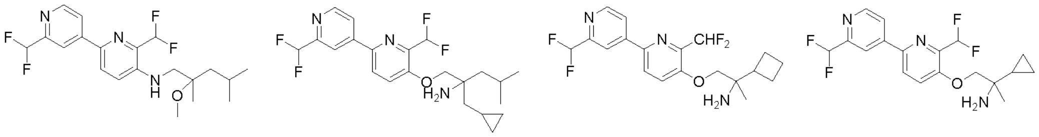

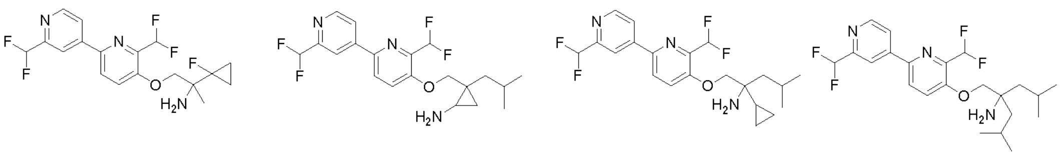

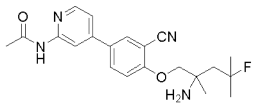
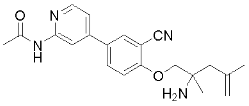
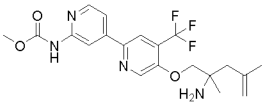
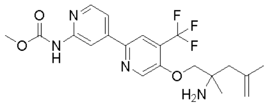
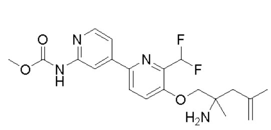
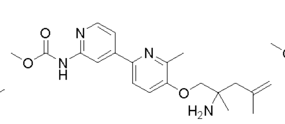
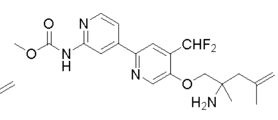
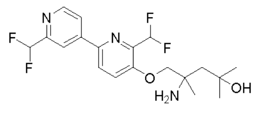
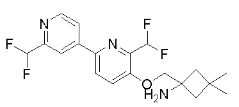
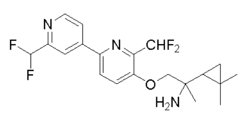
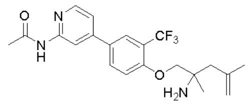
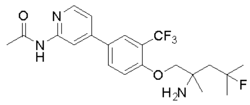
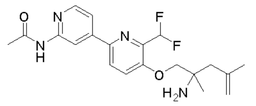
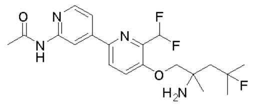
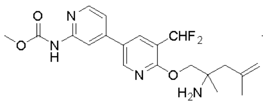
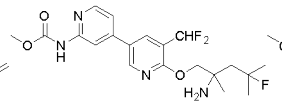
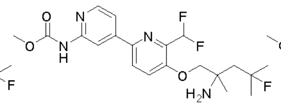
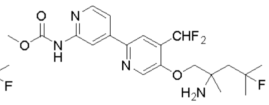
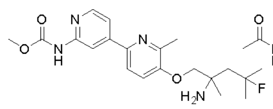
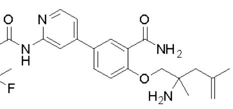
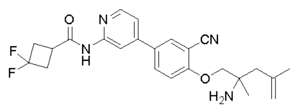
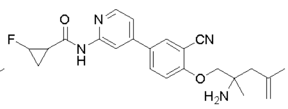
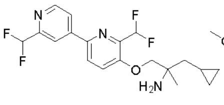
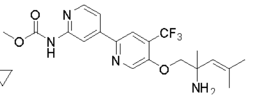
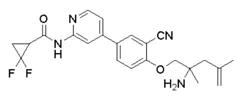
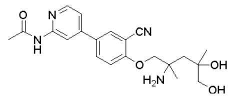
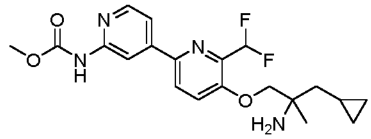.

The compound has a structure further selected from:

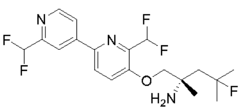
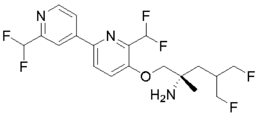
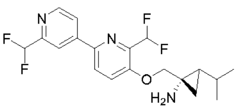
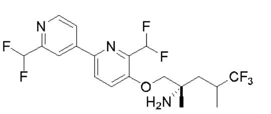
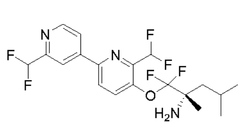
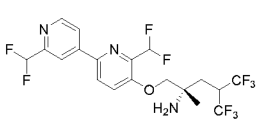
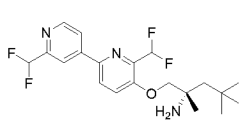
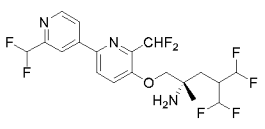
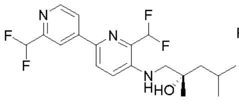
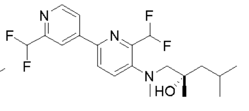
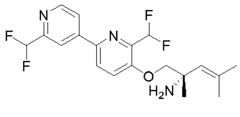
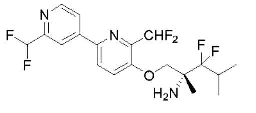

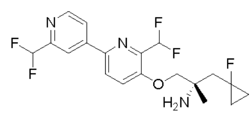 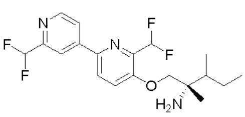 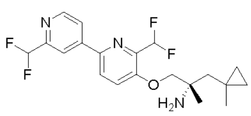 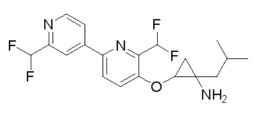

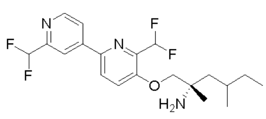 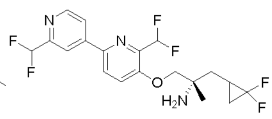 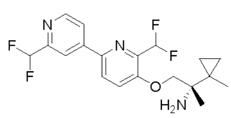 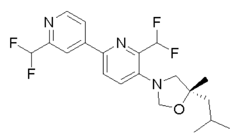

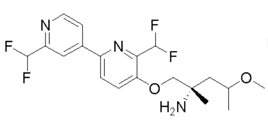 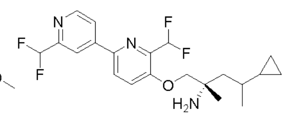 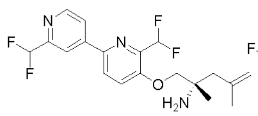 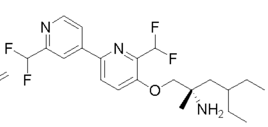

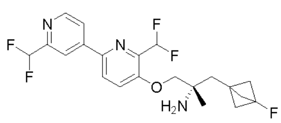 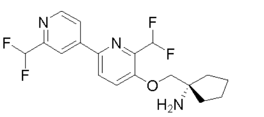 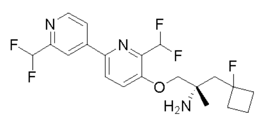 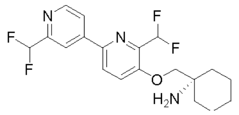

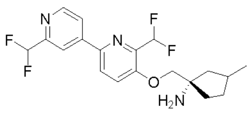 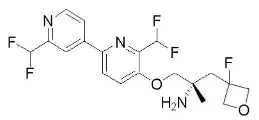 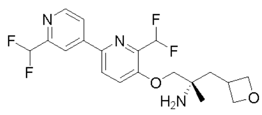 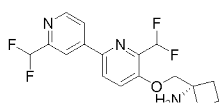

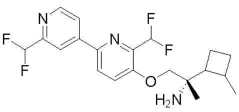 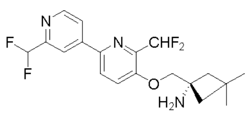 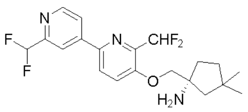 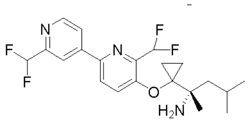

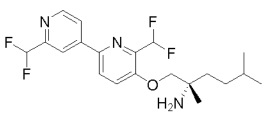 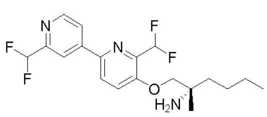 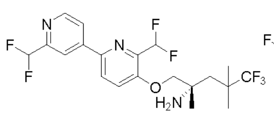 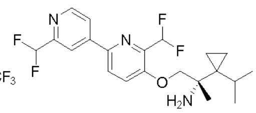

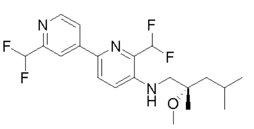 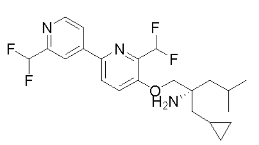 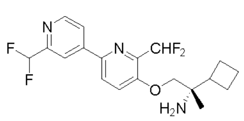 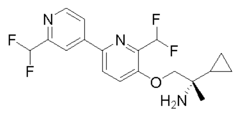

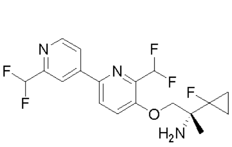 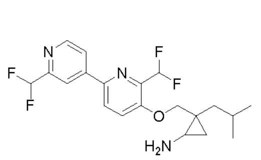 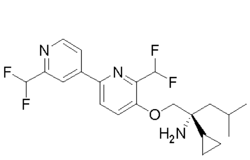 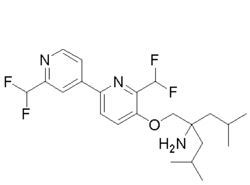

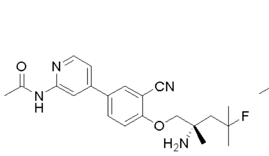 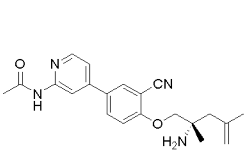 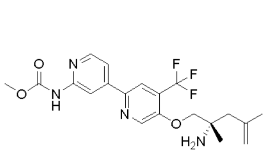 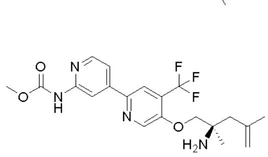

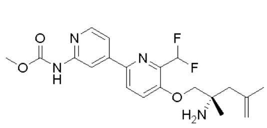 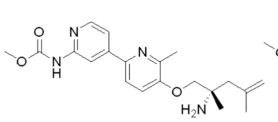 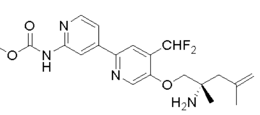 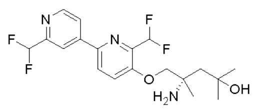

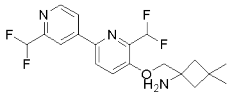 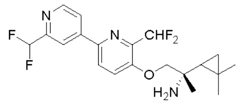

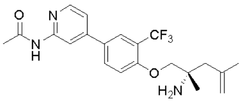 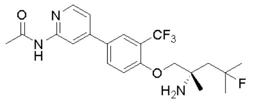 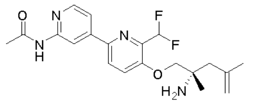 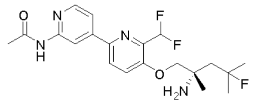

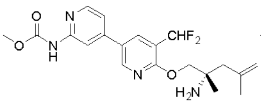 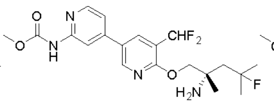 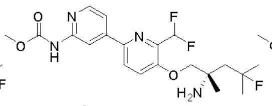 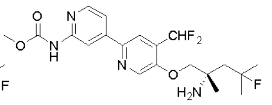

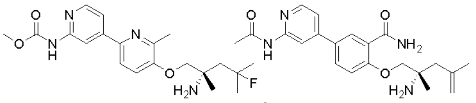 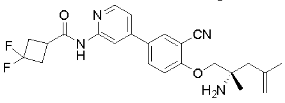 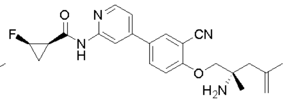

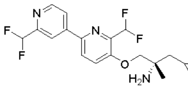 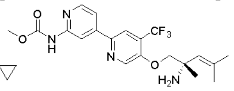 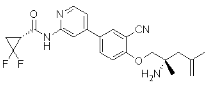 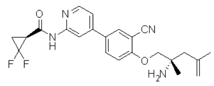

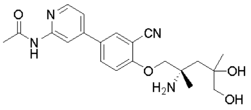 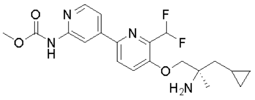 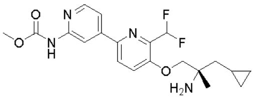.

The present invention further provides a pharmaceutical composition, comprising the compound, or the stereoisomer, deuterated compound, solvate, prodrug, metabolite, pharmaceutically acceptable salt or co-crystal thereof according to any one of the technical solutions described above, and a pharmaceutically acceptable carrier and/or excipient.

Further, the pharmaceutical composition or pharmaceutical preparation comprises 1-1500 mg of the compound, or the stereoisomer, deuterated compound, solvate, pharmaceutically acceptable salt or co-crystal thereof according to any one of the preceding technical solutions, and a pharmaceutically acceptable carrier and/or excipient.

The present invention further relates to the use of the compound, or the stereoisomer, deuterated compound, solvate, prodrug, metabolite, pharmaceutically acceptable salt or co-crystal thereof according to any one of the technical solutions described above, or the composition in the preparation of a drug for treating an AAK1-mediated disease, wherein the AAK1-mediated disease is neuropathic pain, such as diabetic neuropathic pain or post-herpetic neuralgia.

The present invention further provides a method for treating a disease in a mammal, the method comprising administering to a subject a therapeutically effective amount of the compound, or the stereoisomer, deuterated compound, solvate, pharmaceutically acceptable salt or co-crystal thereof according to any one of the preceding technical solutions, and a pharmaceutically acceptable carrier and/or excipient, wherein the therapeutically effective amount is preferably 1-1500 mg; the disease is preferably neuropathic pain; and the disease is more preferably diabetic neuropathic pain or post-herpetic neuralgia.

The present invention further provides a method for treating a disease in a mammal, the method comprising administering to the mammal a therapeutically effective amount of the compound, or the stereoisomer, deuterated compound, solvate, pharmaceutically acceptable salt or co-crystal thereof or the pharmaceutical composition according to the present invention. In some embodiments, the mammal according to the present invention comprises humans.

The term "effective amount" or "therapeutically effective amount" according to the present application refers to a sufficient amount of the compound disclosed in the present application that is administered to ameliorate, to some extent, one or more symptoms of a disease or condition being treated. In some embodiments, the outcome is the reduction and/or remission of signs, symptoms or causes of the disease, or any other desired change in the biological system. For example, an "effective amount" in terms of the therapeutic use is an amount of the composition comprising the compound disclosed in the present application that is required to provide clinically significant reduction of the symptoms of the disease. Examples of the therapeutically effective amount include, but are not limited to 1-1500 mg, 1-1400 mg, 1-1300 mg, 1-1200 mg, 1-1000 mg, 1-900 mg, 1-800 mg, 1-700 mg, 1-600 mg, 1-500 mg, 1-400 mg, 1-300 mg, 1-250 mg, 1-200 mg, 1-150 mg, 1-125 mg, 1-100 mg, 1-80 mg, 1-60 mg, 1-50 mg, 1-40 mg, 1-25 mg, 1-20 mg, 5-1500 mg, 5-1000 mg, 5-900 mg, 5-800 mg, 5-700 mg, 5-600 mg, 5-500 mg, 5-400 mg, 5-300 mg, 5-250 mg, 5-200 mg, 5-150

mg, 5-125 mg, 5-100 mg, 5-90 mg, 5-70 mg, 5-80 mg, 5-60 mg, 5-50 mg, 5-40 mg, 5-30 mg, 5-25 mg, 5-20 mg, 10-1500 mg, 10-1000 mg, 10-900 mg, 10-800 mg, 10-700 mg, 10-600 mg, 10-500 mg, 10-450 mg, 10-400 mg, 10-300 mg, 10-250 mg, 10-200 mg, 10-150 mg, 10-125 mg, 10-100 mg, 10-90 mg, 10-80 mg, 10-70 mg, 10-60 mg, 10-50 mg, 10-40 mg, 10-30 mg, 10-20 mg; 20-1500 mg, 20-1000 mg, 20-900 mg, 20-800 mg, 20-700 mg, 20-600 mg, 20-500 mg, 20-400 mg, 20-350 mg, 20-300 mg, 20-250 mg, 20-200 mg, 20-150 mg, 20-125 mg, 20-100 mg, 20-90 mg, 20-80 mg, 20-70 mg, 20-60 mg, 20-50 mg, 20-40 mg, 20-30 mg; 50-1500 mg, 50-1000 mg, 50-900 mg, 50-800 mg, 50-700 mg, 50-600 mg, 50-500 mg, 50-400 mg, 50-300 mg, 50-250 mg, 50-200 mg, 50-150 mg, 50-125 mg, 50-100 mg; 100-1500 mg, 100-1000 mg, 100-900 mg, 100-800 mg, 100-700 mg, 100-600 mg, 100-500 mg, 100-400 mg, 100-300 mg, 100-250 mg, or 100-200 mg.

The present invention relates to a pharmaceutical composition or pharmaceutical preparation comprising a therapeutically effective amount of the compound, or the stereoisomer, deuterated compound, solvate, pharmaceutically acceptable salt or co-crystal thereof according to the present invention, and a carrier and/or excipient. The pharmaceutical composition can be in a unit preparation form (the amount of the active drug in the unit preparation is also referred to as the "preparation specification"). In some embodiments, the pharmaceutical composition comprises the compound, or the stereoisomer, deuterated compound, solvate, pharmaceutically acceptable salt, or co-crystal thereof according to the present invention in an amount including but not limited to 1-1500 mg, 5-1000 mg, 10-800 mg, 20-600 mg, 25-500 mg, 40-200 mg, 50-100 mg, 1 mg, 1.25 mg, 2.5 mg, 5 mg, 10 mg, 12.5 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 850 mg, 900 mg, 950 mg, 1000 mg, 1100 mg, 1200 mg, 1300 mg, 1400 mg, and 1500 mg.

The present invention further provides a method for treating a disease in a mammal, the method comprising administering to a subject a therapeutically effective amount of the compound, or the stereoisomer, deuterated compound, solvate, pharmaceutically acceptable salt or co-crystal thereof according to the present invention, and a pharmaceutically acceptable carrier and/or excipient, wherein the therapeutically effective amount is preferably 1-1500 mg; the disease is preferably neuropathic pain; and the disease is more preferably diabetic neuropathic pain or post-herpetic neuralgia.

The present invention further provides a method for treating a disease in a mammal, the method comprising administering to a subject a drug, i.e., the compound, or the stereoisomer, deuterated compound, solvate, pharmaceutically acceptable salt or co-crystal thereof according to the present invention, and a pharmaceutically acceptable carrier and/or excipient in a daily dose of 1-1500 mg/day, wherein the daily dose can be a single dose or divided doses; in some embodiments, the daily dose includes, but is not limited to 10-1500 mg/day, 20-1500 mg/day, 25-1500 mg/day, 50-1500 mg/day, 75-1500 mg/day, 100-1500 mg/day, 200-1500 mg/day, 10-1000 mg/day, 20-1000 mg/day, 25-1000 mg/day, 50-1000 mg/day, 75-1000 mg/day, 100-1000 mg/day, 200-1000 mg/day, 25-800 mg/day, 50-800 mg/day, 100-800 mg/day, 200-800 mg/day, 25-400 mg/day, 50-400 mg/day, 100-400 mg/day, or 200-400 mg/day; in some embodiments, the daily dose includes, but is not limited to 1 mg/day, 5 mg/day, 10 mg/day, 20 mg/day, 25 mg/day, 50 mg/day, 75 mg/day, 100 mg/day, 125 mg/day, 150 mg/day, 200 mg/day, 400 mg/day, 600 mg/day, 800 mg/day, 1000 mg/day, 1200 mg/day, 1400 mg/day, or 1500 mg/day.

The present invention relates to a kit, wherein the kit can comprise a composition in the form of a single dose or multiple doses and comprises the compound, or the stereoisomer, deuterated compound, solvate, pharmaceutically acceptable salt or co-crystal thereof according to the present invention, and the amount of the compound, or the stereoisomer, deuterated compound, solvate, pharmaceutically acceptable salt or co-crystal thereof according to the present invention is identical to the amount of same in the above-mentioned pharmaceutical composition.

In the present invention, the amount of the compound, or the stereoisomer, deuterated compound, solvate, pharmaceutically acceptable salt or co-crystal thereof according to the present invention is calculated in the form of a free base in each case.

The term "preparation specification" refers to the weight of the active drug contained in each vial, tablet or other unit preparation.

## Synthetic Route

Those skilled in the art would have been able to prepare the compounds of the present invention by means of combining the documents WO 2017059085, WO 2017059080, and WO 2015153720 and known organic synthesis techniques, wherein the starting materials used therein are commercially available chemicals and (or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

References and monographs in the art introduce in detail the synthesis of reactants that can be used to prepare the compounds described herein, or provide articles describing the preparation method for reference. The references and monographs include: "Synthetic Organic Chemistry", John Wiley & Sons, Inc., New York; S. R. Sandler et al., "Organic Functional Group Preparations," 2nd Ed., Academic Press, New York, 1983; H. O. House, "Modern Synthetic Reactions", 2nd Ed., W. A. Benjamin, Inc. Menlo Park, Calif. 1972; T. L. Gilchrist, "Heterocyclic Chemistry", 2nd Ed., John Wiley & Sons, New York, 1992; J. March, "Advanced Organic Chemistry: Reactions, Mechanisms and Structure", 4th Ed., Wiley-Interscience, New York, 1992; Fuhrhop, J. and Penzlin G. "Organic Synthesis: Concepts, Methods, Starting Materials", Second, Revised and Enlarged Edition (1994) John Wiley & Sons ISBN: 3-527-29074-5; Hoffman, R.V. "Organic Chemistry, An Intermediate Text" (1996) Oxford University Press, ISBN 0-19-509618-5; Larock, R. C. "Comprehensive Organic Transformations: A Guide to Functional Group Preparations" 2nd Edition (1999) Wiley-VCH, ISBN: 0-471-19031-4; March, J. "Advanced Organic Chemistry: Reactions, Mechanisms, and Structure" 4th Edition (1992) John Wiley & Sons, ISBN: 0-471-60180-2; Otera, J. (editor) "Modern Carbonyl Chemistry" (2000) Wiley-VCH, ISBN: 3-527-29871-1; Patai, S. "Patai's 1992 Guide to the Chemistry of Functional Groups" (1992) Interscience ISBN: 0-471-93022-9; Solomons, T. W. G. "Organic Chemistry" 7th Edition (2000) John Wiley & Sons, ISBN: 0-471-19095-0; Stowell, J.C., "Intermediate Organic Chemistry" 2nd Edition (1993) Wiley-Interscience, ISBN: 0-471-57456-2; "Industrial Organic Chemicals: Starting Materials and Intermediates: An Ullmann's Encyclopedia" (1999) John Wiley & Sons, ISBN: 3-527-29645-X, in 8 volumes; "Organic Reactions" (1942-2000) John Wiley & Sons, in over 55 volumes; and "Chemistry of Functional Groups" John Wiley & Sons, in 73 volumes.

Specific and similar reactants can be selectively identified by the indexes of known chemicals prepared by the Chemical Abstracts Service of the American Chemical Society, wherein the indexes are available in most public libraries or university libraries and online. Chemicals that are known but not commercially available in the catalog are optionally prepared by custom chemical synthesis plants, wherein many of standard chemical supply plants (for example, those listed above) provide custom synthesis services. Reference document for the preparation and selection of the pharmaceutically acceptable salts of the compounds described herein is P. H. Stahl & C. G. Wermuth "Handbook of Pharmaceutical Salts", Verlag Helvetica Chimica Acta, Zurich, 2002.

**Term**

Unless otherwise specified, the terms of the present invention have the following meanings.

The carbon, hydrogen, oxygen, sulphur, nitrogen and halogen involved in the groups and compounds of the present invention all comprise isotopes thereof, and are optionally further substituted with one or more of the corresponding isotopes thereof, wherein the isotopes of carbon comprise $^{12}C$, $^{13}C$ and $^{14}C$; the isotopes of hydrogen comprise protium (H), deuterium (deuterium, also known as heavy hydrogen), and tritium (T, also known as superheavy hydrogen); the isotopes of oxygen comprise $^{16}O$, $^{17}O$ and $^{18}O$; the isotopes of sulphur comprise $^{32}S$, $^{33}S$, $^{34}S$ and $^{36}S$; the isotopes of nitrogen comprise $^{14}N$ and $^{15}N$; the isotope of fluorine comprises $^{19}F$; the isotopes of chlorine comprise $^{35}C1$ and $^{37}Cl$; and the isotopes of bromine comprise $^{79}Br$ and $^{81}Br$.

The expression "$C_{x-y}$ group" refers to a group comprising x to y carbon atoms, for example, "$C_{1-6}$ alkyl" refers to an alkyl group comprising 1-6 carbon atoms.

The term "halogen" refers to fluorine (F), chlorine (Cl), bromine (Br), iodine (I) or isotopes thereof.

The term "halo" or "substituted with halogen" means that the hydrogen atoms are substituted with one or more groups selected from F, Cl, Br, I, or isotopes thereof, wherein the upper limit of the number of halogen substituents is equal to the sum of the number of hydrogens that can be substituted in the group to be substituted. Without particular limitation, the number of halogen substituents is any integer between 1 and the upper limit, preferably 1-5 halogen, 1-3 halogen, 1-2 halogen, and 1 halogen; and when the number of halogen substituents is greater than 1, the group to be substituted can be substituted with the same or different halogen.

The term "halo $C_{1-6}$ alkyl" refers to an alkyl group comprising 1-6 carbon atoms in which one or more hydrogens are substituted with one or more halogen atoms (e.g., fluorine, chlorine, bromine, and iodine), wherein the upper limit of the number of halogen substituents is equal to the sum of the number of hydrogens that can be substituted in the alkyl group. Without particular limitation, the number of halogen substituents is any integer between 1 and the upper limit, preferably 1-5 halogen, 1-3 halogen, 1-2 halogen, or 1 halogen; and when the number of halogen substituents is greater than 1, the group to be substituted can be substituted with the same or different halogen. Examples include, but are not limited to $-CF_3$, $- CH_2Cl$, $-CH_2CF_3$, $-CCl_2$, $CF_3$, etc.

The term "deuterium" refers to the isotope deuterium of hydrogen (H).

The term "deuterated" or "deuterated compound" refers to the case where a hydrogen atom on a group, such as alkyl, cycloalkyl, alkylene, aryl, heteroaryl, mercapto, heterocycloalkyl, alkenyl and alkynyl is substituted with at least one deuterium atom, wherein the upper limit of the number of deuterium substituents is equal to the sum of the number of hydrogens that can be substituted in the group to be substituted. Without particular limitation, the number of deuterium substituents is any integer between 1 and the upper limit, preferably 1-20 deuterium atoms, 1-10 deuterium atoms, 1-6

deuterium atoms, 1-3 deuterium atoms, 1-2 deuterium atoms or 1 deuterium atom.

The term "alkyl" refers to a straight or branched saturated aliphatic hydrocarbon group. Unless otherwise specified, the alkyl refers to an alkyl group comprising 1 to 20 carbon atoms, preferably an alkyl group comprising 1 to 8 carbon atoms, more preferably an alkyl group comprising 1 to 6 carbon atoms, further preferably an alkyl group comprising 1 to 4 carbon atoms, and further preferably an alkyl group comprising 1-2 carbon atoms. Non-limiting examples of alkyl include methyl, ethyl, n-propyl, isopropyl, n-butyl, sec-butyl, neobutyl, tert-butyl, n-pentyl, isoamyl, neopentyl, n-hexyl, etc. The alkyl can be further substituted with any substituent.

The term "hydroxyalkyl" refers to alkyl substituted with hydroxyl, wherein the alkyl is as defined above.

The term "alkenyl" refers to a straight or branched hydrocarbon group comprising at least one carbon-carbon double bond (C=C), and the main chain comprises 2 to 18 (such as 2 to 8, further such as 2 to 6, and more further such as 2 to 4) carbon atoms unless otherwise specified. Examples of alkenyl include, but are not limited to vinyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc.; and the alkenyl can be optionally further substituted with any group.

The term "alkynyl" refers to a straight or branched hydrocarbon group containing at least one carbon-carbon triple bond (C≡C). The main chain comprises 2 to 18 (such as 2 to 8, further such as 2 to 6, and more further such as 2 to 4) carbon atoms. Ethynyl, 1-propynyl, 2-propynyl, butynyl, 2-butynyl, 3-butynyl, 1-methyl-2-propynyl, 4-pentynyl, 3-pentynyl, 1-methyl-2-butynyl, 2-hexynyl, 3-hexynyl, 2-heptynyl, 3-heptynyl, 4-heptynyl, 3-octynyl, 3-nonynyl, 4-decynyl, etc. The alkynyl can be optionally further substituted with any substituent.

The term "alkoxy" or "alkyloxy" refers to -O-alkyl. Without particular limitation, alkoxy or alkyloxy is -O-$C_{1-8}$ alkyl, preferably -O-$C_{1-6}$ alkyl, more preferably -O-$C_{1-4}$ alkyl, and further preferably -O-$C_{1-2}$ alkyl. Non-limiting examples of alkoxy or alkyloxy include methoxy, ethoxy, n-propoxy, isopropoxy, n-butoxy, secbutoxy, tert-butoxy, n-pentoxy, n-hexyloxy, cyclopropoxy, cyclobutoxy, etc. The alkoxy can be optionally further substituted with any substituent.

The term "haloalkoxy" refers to -O-haloalkyl. Without particular limitation, haloalkoxy is -O-halo $C_{1-8}$ alkyl, preferably -O-halo $C_{1-6}$ alkyl, more preferably -O-halo $C_{1-4}$ alkyl, and further preferably -O-halo $C_{1-2}$ alkyl, wherein the upper limit of the number of halogen substituents is equal to the sum of the number of hydrogens that can be substituted in the group to be substituted. Without particular limitation, the number of halogen substituents is any integer between 1 and the upper limit, preferably 1-5 halogen, 1-3 halogen, 1-2 halogen, and 1 halogen; and when the number of halogen substituents is greater than 1, the group to be substituted can be substituted with the same or different halogen. Non-limiting examples of haloalkoxy include monofluoromethoxy, difluoromethoxy, trifluoromethoxy, difluoroethyloxy, *etc.*

The term "cycloalkyl" refers to a substituted or unsubstituted, saturated or partially unsaturated non-aromatic hydrocarbon ring. Cycloalkyl can be a monocyclic, bicyclic or polycyclic ring, wherein the bicyclic or polycyclic ring can be a fused ring, a spiro ring or a bridged ring. Unless otherwise specified, cycloalkyl usually contains 3 to 20 carbon atoms. When cycloalkyl is monocyclic cycloalkyl, the cycloalkyl contains preferably 3-15 carbon atoms, preferably 3-10 carbon atoms, also preferably 3-8 carbon atoms, more preferably 3-6 carbon atoms, and further preferably 3-4 carbon atoms; and when cycloalkyl is bicyclic or polycyclic cycloalkyl, the cycloalkyl contains preferably 4-12 carbon atoms, preferably 4-11 carbon atoms, also preferably 5-11 carbon atoms, more preferably 6-11 carbon atoms, and further preferably 6-10 carbon atoms. Non-limiting examples of cycloalkyl include cyclopropyl, cyclobutyl, cyclopentyl, cyclohexyl, cycloheptyl, butenyl, cyclopentenyl, cyclohexenyl,

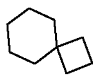, 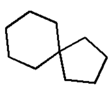, 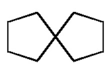,

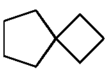, 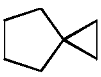, 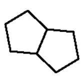, 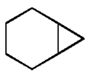, 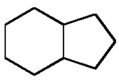, 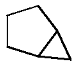, 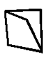, 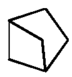, 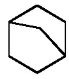;

etc.

The term "heterocycloalkyl" refers to a substituted or unsubstituted, saturated or partially unsaturated non-aromatic ring containing at least one heteroatom. Unless otherwise specified, heterocycloalkyl is a 3- to 20-membered ring. When heterocycloalkyl is monocyclic heterocycloalkyl, the heterocycloalkyl is preferably a 3- to 15-membered, preferably 3- to 10-membered, also preferably 3- to 8-membered, and further preferably 3- to 6-membered ring; and when heterocycloalkyl is bicyclic or polycyclic heterocycloalkyl, the heterocycloalkyl is preferably a 4- to 12-membered, preferably 4- to 11-membered, also preferably 5-to 11-membered, more preferably 6- to 11-membered, and further

preferably 6- to 10-membered ring. Heterocycloalkyl can be a monocyclic, bicyclic or polycyclic ring, wherein the bicyclic or polycyclic ring can be a bridged ring, a fused ring and a spiro ring, in which the heteroatoms are selected from heteroatoms N, S, O, P and Si and oxidation states thereof. When heterocycloalkyl is a bicyclic or polycyclic ring, at least one ring contains at least one heteroatom, and the heterocycloalkyl can be a bicyclic or polycyclic ring formed by a ring containing the heteroatom(s) and a ring containing no heteroatom. When heterocycloalkyl is connected to other groups, a connection point can be at a heteroatom or a carbon atom. Non-limiting examples of heterocycloalkyl include azetidinyl, morpholinyl, piperazinyl, piperidyl, tetrahydropyranyl, oxetanyl, pyranyl, azacyclopentenyl, azacyclohexenyl, oxacyclopentenyl, oxacyclohexenyl, etc.

The term "aryl" refers to a substituted or unsubstituted aromatic 5- to 15-membered carbocycle, and includes monocyclic aryl and fused aryl. Aryl is preferably a 5- to 10-membered aromatic ring, and further preferably a 5- to 8-membered aromatic ring. Aryl ring can be fused to a non-aryl ring (such as a heteroaryl, heterocycloalkyl or cycloalkyl ring), wherein the aryl ring is the connection site, and non-limiting examples thereof comprise phenyl, naphthyl, anthryl, phenanthryl,

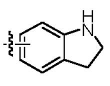, 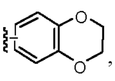, 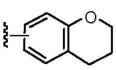, 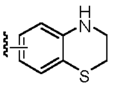, 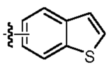, 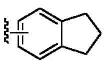, 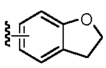, 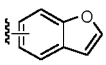,

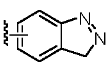, 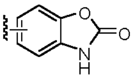, 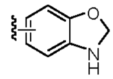, 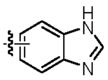, 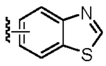, 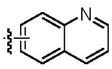 and 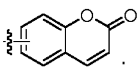.

The aryl can be optionally further substituted with any substituent.

The term "heteroaryl ring" or "heteroaryl" refers to a substituted or unsubstituted aromatic ring containing at least one heteroatom or group selected from heteroatoms N, S, O, P and Si and oxidation states thereof. Heteroaryl ring or heteroaryl can be a monocyclic, bicyclic or polycyclic ring, wherein the bicyclic or polycyclic ring can be a bridged ring, a fused ring and a spiro ring. Bicyclic or polycyclic heteroaryl ring or heteroaryl can be formed by fusion of heteroaryl to a non-heteroaryl ring such as cycloalkyl, heterocycloalkyl and aryl, or of heteroaryl to heteroaryl, wherein the heteroaryl ring is the connection site. Non-limiting examples of heteroaryl ring or heteroaryl include furyl, thienyl, pyrrolyl, oxazolyl, thiazolyl, imidazolyl, pyrazolyl, pyridyl, pyrimidinyl, pyridazinyl, pyrazinyl, indolyl, purinyl,

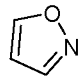, 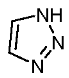, 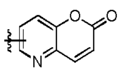,

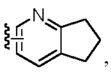, 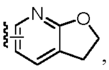, 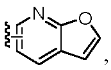, 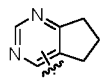, 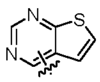, 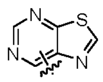, 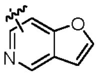, 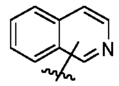,

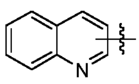,

etc. The heteroaryl can be optionally further substituted with any substituent.

The term "carboxyl" refers to -C(=O)-OH.

"Spiro ring" refers to a 5- to 20-membered polycyclic group sharing one carbon atom (referred to as a spiro atom) between substituted or unsubstituted rings, which may contain 0 to 5 double bonds, and may contain 0 to 5 heteroatoms or groups selected from N, O, S, P, Si and oxidation states thereof. The spiro ring is preferably 6- to 14-membered, further preferably 6- to 12-membered, and more preferably 6- to 10-membered. The spiro ring can be formed between cycloalkyl and heterocycloalkyl. The spiro ring is preferably a spiro ring formed by a three-membered ring and a three-membered ring, a three-membered ring and a four-membered ring, a three-membered ring and a five-membered ring, a three-membered ring and a six-membered ring, a four-membered ring and a four-membered ring, a four-membered ring and a five-membered ring, a four-membered ring and a six-membered ring, a five-membered ring and a five-membered ring or a five-membered ring and a six-membered ring; non-limiting examples of the spiro ring include

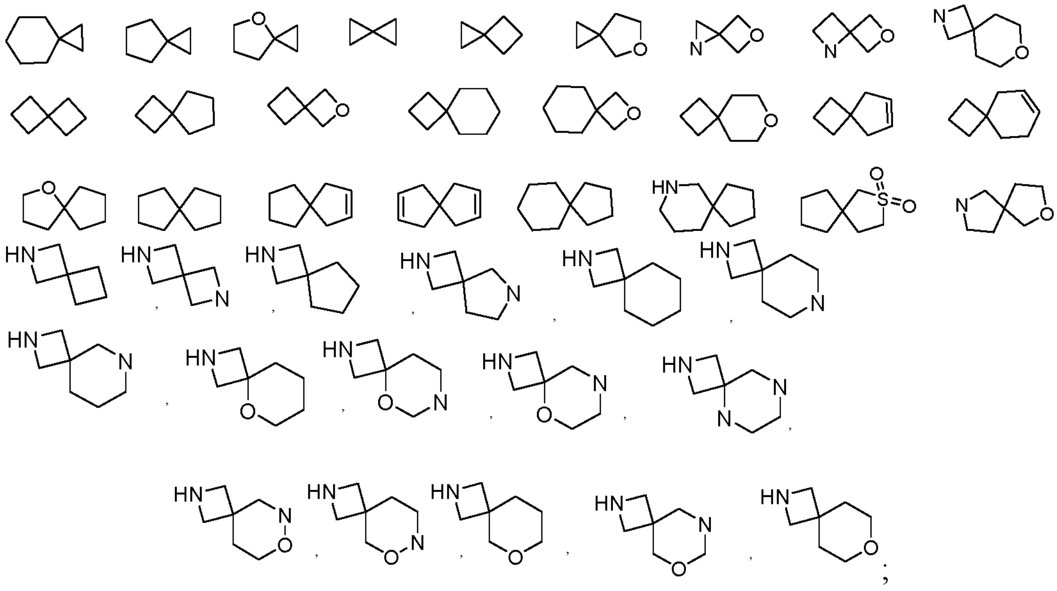

and the spiro ring can be optionally further substituted with any substituent.

A "fused ring" refers to a polycyclic group in which the rings share two adjacent atoms, wherein one or more rings may contain 0 or more double bonds, and may be substituted or unsubstituted, and each ring in the fused ring system may contain 0 to 5 heteroatoms selected from N, S, O, P, Si and oxidation states thereof. The fused ring is preferably 5- to 20-membered, further preferably 5- to 14-membered, more preferably 5- to 12-membered, and still further preferably 5-to 10-membered. Preferably, the fused ring may be in the form of a three-membered ring fused a four-membered ring (indicating a fused ring formed by a three-membered ring and a four-membered ring, and either the three-membered ring or the four-membered ring may be possibly used as the basic ring according to the IUPC nomenclature; similarly hereinafter), a three-membered ring fused a five-membered ring, a three-membered ring fused a six-membered ring, a four-membered ring fused a four-membered ring, a four-membered ring fused a five-membered ring, a four-membered ring fused a six-membered ring, a five-membered ring fused a five-membered ring, a five-membered ring fused a six-membered ring, and a six-membered ring fused a six-membered ring; and non-limiting examples include purine, quinoline, isoquinoline, benzopyran, benzofuran, benzothiophene,

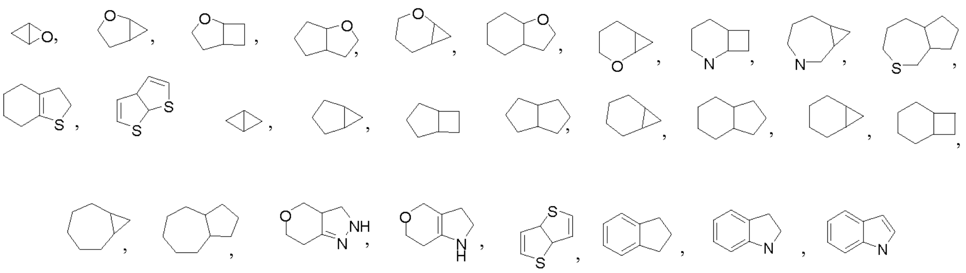

The fused ring can be optionally further substituted with any substituent.

A "bridged ring" refers to a ring system in which two rings share two non-adjacent atoms, which may contain 0 or more double bonds, and may be substituted or unsubstituted, wherein one or more rings may contain 0 to 5 heteroatoms selected from N, S, O, P, Si and oxidation states thereof. The ring atoms contain 5 to 20 atoms, preferably 5 to 14 atoms, further preferably 5 to 12 atoms, and still further preferably 5 to 10 atoms; and non-limiting examples include adamantane,

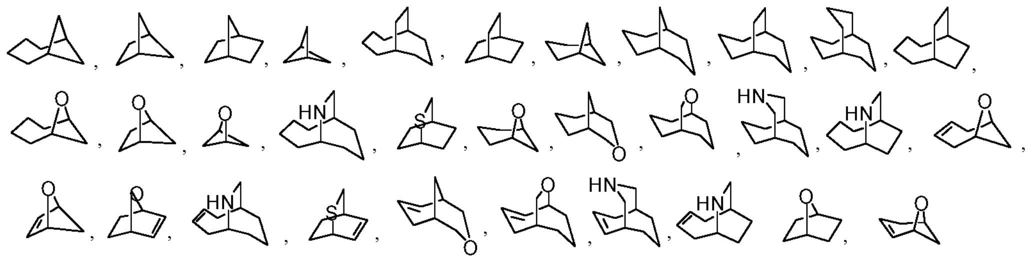

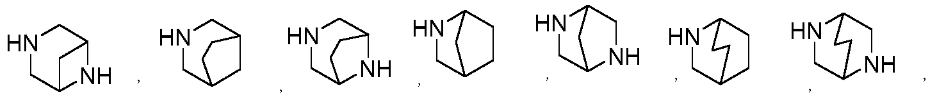

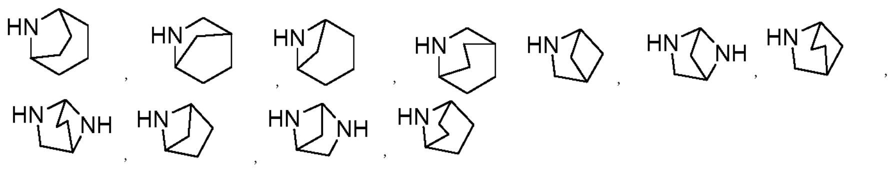

The heteroatom according to the present invention can be selected from N, O, S, Si, P atoms and oxidation states thereof.

The term "optional" or "optionally" refers to that the events or circumstances subsequently described may but not necessarily occur, and the description includes the occasions where the events or circumstances occur or do not occur. For example, "alkyl optionally substituted with F" means that the alkyl may but not necessarily be substituted by F, and the description includes the case where the alkyl is substituted with F and the case where the alkyl is not substituted with F.

Unless otherwise specified, substitution with a substituent described herein refers to substitution at a position allowed by chemical theory, and the number of substituents conforms to the rules of chemical bonding.

The term "pharmaceutically acceptable salt" refers to a salt of the compound of the present invention, which salt maintains the biological effectiveness and characteristics of a free acid or a free base and is obtained by reacting the free acid with a non-toxic inorganic base or organic base, or reacting the free base with a non-toxic inorganic acid or organic acid.

The term "pharmaceutical composition" represents a mixture of one or more compounds described herein or the stereoisomers, solvates, pharmaceutically acceptable salts, co-crystals or deuterated compounds thereof and other components comprising physiologically/pharmaceutically acceptable carriers and/or excipients.

The term "carrier" refers to: a system that does not cause significant irritation to the organism and does not eliminate the biological activity and characteristics of the administered compound, and can change the way the drug enters the human body and the distribution of the drug in the body, control the release rate of the drug and delivery the drug to targeted organs. Non-limiting examples of the carrier include microcapsule, microsphere, nanoparticle, liposome, etc.

The term "excipient" refers to: a substance that is not a therapeutic agent per se, but used as a diluent, adjuvant, adhesive and/or vehicle for addition to a pharmaceutical composition, thereby improving the disposal or storage properties thereof, or allowing to or promoting the formation of a compound or a pharmaceutical composition into a unit dosage form for administration. As is known to those skilled in the art, a pharmaceutically acceptable excipient can provide various functions and can be described as a wetting agent, a buffer, a suspending agent, a lubricant, an emulsifier, a disintegrating agent, an absorbent, a preservative, a surfactant, a colorant, a flavouring agent and a sweetening agent. Examples of pharmaceutically acceptable excipients include, but are not limited to: (1) sugars, such as lactose, glucose and sucrose; (2) starch, such as corn starch and potato starch; (3) cellulose and its derivatives, such as sodium carboxymethyl cellulose, ethyl cellulose, cellulose acetate, hydroxypropyl methylcellulose, hydroxypropyl cellulose, microcrystalline cellulose and croscarmellose (such as croscarmellose sodium); (4) tragacanth powder; (5) malt; (6) gelatine; (7) talc; (8) excipients, such as cocoa butter or suppository wax; (9) oils, such as peanut oil, cottonseed oil, safflower oil, sesame oil, olive oil, corn oil and soybean oil; (10) diols, such as propylene glycol; (11) polyols, such as glycerol, sorbitol, mannitol and polyethylene glycol; (12) esters, such as ethyl oleate and ethyl laurate; (13) agar; (14) buffers, such as magnesium hydroxide and aluminium hydroxide; (15) alginic acid; (16) pyrogen-free water; (17) isotonic saline; (18) Ringer's solution; (19) ethanol; (20) pH buffered solution; (21) polyester, polycarbonate and/or polyanhydride; and

(22) other non-toxic compatible substances used in a pharmaceutical preparation.

The term "stereoisomer" refers to an isomer produced as a result of different spatial arrangement of atoms in molecules, including cis-trans isomers, enantiomers and conformational isomers.

The term "solvate" refers to a substance formed by the compound of the present invention or the salt thereof and a stoichiometric or non-stoichiometric solvent bound by intermolecular non-covalent forces. When the solvent is water, the solvate is a hydrate.

The term "co-crystal" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio between various components. The co-crystal is a multi-component crystal, which includes both a binary co-crystal formed between two neutral solids and a multi-element co-crystal formed between a neutral solid and a salt or solvate.

## Brief Description of the Drawings

Figure 1 shows the time-MPT curve of the experiment with the SNL-induced mouse model of neuropathic pain.

## Detailed Description of Embodiments

The technical solutions of the present invention will be described in detail below in conjunction with the drawings and examples, but the scope of protection of the present invention includes but is not limited thereto.

The content of the present invention is described in detail with the following examples. If a specific condition is not indicated in the examples, a conventional condition is used in an experimental method. The listed examples are intended to better illustrate the content of the present invention, but should not be construed as limiting the content of the present invention. According to the above-mentioned content of the invention, those skilled in the art can make unsubstantial modifications and adjustments to the embodiments, which still fall within the protection scope of the present invention.

### Test method

The structures of the compounds are determined by nuclear magnetic resonance (NMR) or (and) mass spectrometry (MS). The NMR shift (δ) is given in the unit of 10-6 (ppm). NMR is measured with (Bruker Avance III 400 and Bruker Avance 300) NMR instrument, and the solvent for determination is deuterated dimethyl sulphoxide (DMSO-$d_6$), deuterated chloroform ($CDCl_3$), deuterated methanol ($CD_3OD$), and the internal standard is tetramethylsilane (TMS);

MS is determined with Agilent 6120B (ESI) and Agilent 6120B (APCI);
HPLC is determined with Agilent 1260DAD high pressure liquid chromatograph (Zorbax SB-C18 100 × 4.6 mm, 3.5 μM);
Yantai Huanghai HSGF254 or Qingdao GF254 silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm - 0.5 mm.
and for the column chromatography, Yantai Huanghai silica gel of 200-300 mesh silica gel is generally used as a carrier.

Description of abbreviations:

THF: Tetrahydrofuran
CbzCl: Benzyl chloroformate
NaOH: Sodium hydroxide
KOAc: Potassium acetate
DAST: Diethylaminosulphur trifluoride
Xphos: 2-dicyclohexylphosphino-2',4',6'-triisopropylbiphenyl
Xphos PdG2: Chloro(2-dicyclohexylphosphino-2',4',6'-triisopropyl-1,1'-biphenyl)[2-(2'-amino-1,1'-biphenyl)]palladium (II)

**Intermediate 1**: **6-bromo-2-(difluoromethyl)-3-fluoropyridine (immediate 1)**

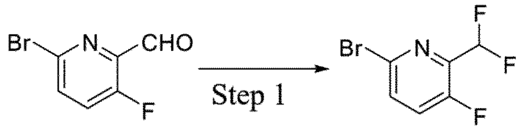


1A Intermediate 1

Step 1: 6-bromo-2-(difluoromethyl)-3-fluoropyridine **(immediate 1)**

Raw material **1A** (10 g, 49 mmol) was dissolved in 200 mL of dichloromethane, and the mixture was cooled to -20°C. DAST (11.7 mL, 88 mmol) was added and the resulting mixture was slowly warmed to room temperature and reacted for 5 h. Upon complete depletion of raw materials monitored by TLC, the reaction was quenched with saturated aqueous sodium bicarbonate solution. The resulting reaction mixture was extracted with ethyl acetate and the organic phase was subjected to rotary evaporation. Then the residue was purified by silica gel column (petroleum ether : ethyl acetate = 20 : 1) to obtain the target compound **intermediate** 1 (9.8 g, 89%).

$^1H$ NMR (400 MHz, $CDCl_3$) δ7.65 - 7.58 (m, 1H), 7.46 - 7.40 (m, 1H), 6.85 - 6.56 (m, 1H).

**Intermediate 2: (2-(difluoromethyl)pyridin-4-yl)boronic acid (immediate 2)**

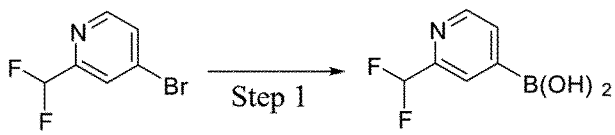


2A Intermediate 2

Step 1: (2-(difluoromethyl)pyridin-4-yl)boronic acid **(immediate 2)**

**2A** (5 g, 24 mmol), Xphos PdG2 (189 mg, 0.24 mmol, CAS: 1310584-14-5), Xphos (229 mg, 0.48 mmol, CAS 564483-18-7), bis(pinacolato)diboron (9.14 g, 36 mmol) and KOAc (7.07 g, 72 mmol) were added to a flask. After nitrogen replacement, 200 mL of ethanol was added and the mixture was heated to 80°C and reacted for 5 h. Upon complete depletion of raw materials monitored by TLC, water was added to quench the reaction. The system was subjected to rotary evaporation to remove ethanol and then extracted with ethyl acetate. The organic phase was subjected to rotary evaporation to obtain **intermediate 2** (5.1 g).

LC-MS (ESI): m/z = 174.1 [M+H]$^+$.

**Intermediate 3: (S)-2-amino-2,4-dimethylpent-4-en-1-ol**

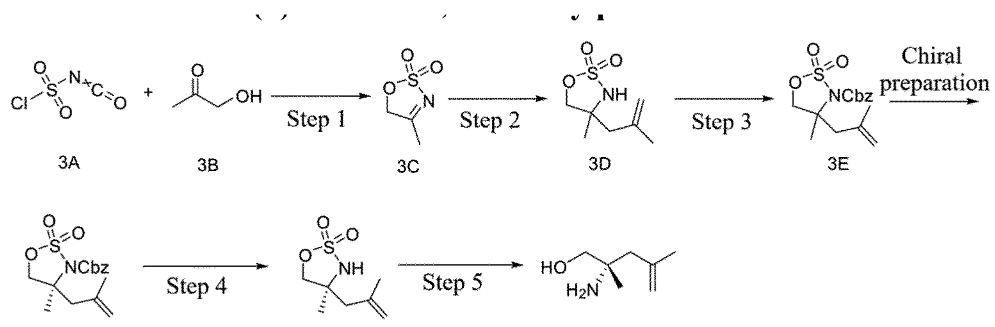


3A 3B 3C 3D 3E 3F 3G Intermediate 3

Step 1: 4-methyl-5H-1,2,3-oxathiazole 2,2-dioxide **(3C)**

Under nitrogen atmosphere, chlorosulphonyl isocyanate (62 mL) was added to a three-necked round bottom flask, then 200 mL of dichloromethane was added, and the system was cooled to 0°C. 27 mL of formic acid was dissolved

in 50 mL of dichloromethane. The mixture was slowly added to the system while the temperature was controlled at 0°C. After 30 minutes, the mixture was warmed to room temperature and stirred overnight. Hydroxyacetone (36.3 mL) and pyridine (58 mL) were dissolved in 1000 mL of dichloromethane. At 0°C, the mixture was slowly added dropwise to the system. After the addition was completed, the system was warmed to room temperature and stirred overnight. The system was subjected to rotary evaporation to remove the organic solvent and the residue was purified by silica gel column (eluent: dichloromethane) to obtain the title compound **3C** (36 g, 56%).

$^1$H NMR (400 MHz, $CDCl_3$) δ 5.06 (s, 2H), 2.42 (s, 3H).

Step 2: 4-methyl-4-(2-methylallyl)-1,2,3-oxathiazolidine 2,2-dioxide **(3D)**

Under nitrogen atmosphere, **3C** (36 g, 267 mmol) was dissolved in 800 mL of methyl tert-butyl ether. The system was cooled to 0°C and then a solution of 2-methylallylmagnesium chloride in tetrahydrofuran (0.55 L, 0.5 M) was added dropwise. Upon complete depletion of raw materials monitored by TLC, the reaction was quenched by the addition of saturated aqueous ammonium chloride solution, and the resulting mixture was extracted with ethyl acetate and then subjected to rotary evaporation. The residue was purified by silica gel column to obtain the title compound **3D** (43 g, 84%).

$^1$H NMR (400 MHz, $CDCl_3$) δ 5.06 - 5.01 (m, 1H), 4.85 - 4.83 (m, 1H), 4.59 (s, 1H), 4.38 (d, 1H), 4.27 (d, 1H), 2.57 - 2.50 (m, 1H), 2.42 - 2.29 (m, 1H), 1.84 (s, 3H), 1.46 (s, 3H).

Step 3: Benzyl 4-methyl-4-(2-methylallyl)-1,2,3-oxathiazolidine-3-carboxylate 2,2-dioxide **(3E)**

Under nitrogen, **3D** (1.91 g, 10 mmol) was dissolved in 50 mL of tetrahydrofuran. A solution of 1 M potassium tert-butoxide in tetrahydrofuran (15 mL) was added, followed by CbzCl (2.1 mL, 15 mmol). Upon complete depletion of raw materials monitored by TLC, the reaction was quenched by the addition of saturated aqueous ammonium chloride solution. The system was subjected to rotary evaporation to remove tetrahydrofuran, extracted with ethyl acetate, and then subjected to rotary evaporation. The residue was then purified by silica gel column (petroleum ether : ethyl acetate = 10 : 1) to obtain the title compound **3E** (2.6 g, 80%).

LC-MS (ESI): m/z = 343.0 $[M+NH_4]^+$.

120 g of **3E** was subjected to chiral preparation to obtain the target compound **3F** (55 g).

Preparation method: instrument: Waters SFC 150 Mgm, column: DAICEL CHIRALPAK OJ (250 mm × 50 mm, 10 μm); mobile phase: A for $CO_2$ and B for MeOH (BASE); gradient: 10% B; flow rate: 130 mL/min, back pressure: 100 bar; column temperature: 35°C; wavelength: 220 nm; cycle time: 4.5 min; sample preparation: sample concentration: 157.5 mg/ml, ethanol solution; sample injection: 0.8 ml/injection. After separation, the fractions were dried on a rotary evaporator at the bath temperature of 40°C to obtain compound **3F** (retention time: 0.680 minutes).

Step 4: (S)-4-methyl-4-(2-methylallyl)-1,2,3-oxathiazolidine 2,2-dioxide **(3G)**

Compound **3F** (5 g, 15.4 mmol) was dissolved in 500 mL of methanol and 50 mg of 10% palladium on carbon catalyst was added. The mixture was subjected to hydrogen replacement. Upon the disappearing of fluorescence monitored by TLC, the system was filtered by suction to remove palladium on carbon from the system. The resulting filtrate was subjected to rotary evaporation to obtain the title compound **3G** (crude), which was directly used in the next step.

Step 5: (S)-2-amino-2,4-dimethylpent-4-en-1-ol **(intermediate 3)**

Compound **3G** was dissolved in 150 mL of tetrahydrofuran. At 0°C, lithium aluminium hydride (1.8 g, 47.4 mmol) was added in portions and the mixture was warmed to room temperature and stirred overnight. Water (1.8 mL), 10% aqueous sodium hydroxide solution (3.6 mL) and water (5.4 mL) were added and the mixture was stirred for 1 h. The resulting reaction mixture was filtered by suction to remove the solid. The resulting filtrate was subjected to rotary evaporation to obtain **intermediate 3** (crude product), which was directly used in the next reaction.

LC-MS (ESI): m/z = 130.1 $[M+H]^+$.

**Intermediate 4: (2-((methoxycarbonyl)amino)pyridin-4-yl)boronic acid (intermediate 4)**

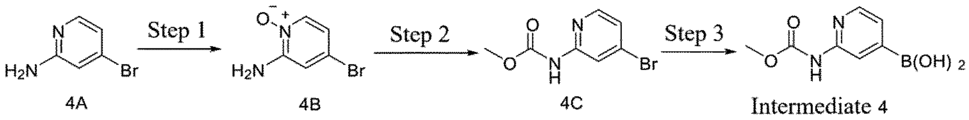


Step 1: 2-amino-4-bromopyridine 1-oxide **(4B)**

Compound **4A** (10 g, 57.8 mmol) was dissolved in 200 mL of acetone. At room temperature, m-chloroperoxybenzoic acid (11 g, 63.6 mmol) was dissolved in 200 mL of acetone and then the resulting mixture was added. The reaction mixture was stirred for 5 min to produce a large quantity of a solid. The solid was collected by suction filtration, washed with acetone, and dried to obtain compound **4B** (crude product, 10.7 g, 98%).
LC-MS (ESI): m/z = 189.0 and 191.0 $[M+H]^+$.

Step 2: Methyl (4-bromopyridin-2-yl)carbamate **(4C)**

Compound **4B** (crude, 10.7 g) was dissolved in trimethyl orthoformate (200 mL) and 1.25 mL of boron trifluoride diethyl etherate was added. The system was heated to 105°C and reacted overnight. The system was subjected to rotary evaporation to remove the organic phase and then separated by column chromatography to obtain compound **4C** (9.1 g, 69%).
LC-MS (ESI): m/z = 231.0 and 233.0 $[M+H]^+$.

Step 3: (2-((methoxycarbonyl)amino)pyridin-4-yl)boronic acid **(intermediate 4)**

Compound **4C** (3.5 g, 15.1 mmol), Xphos PdG2 (600 mg, 0.76 mmol, CAS: 1310584-14-5), Xphos (700 mg, 1.47 mmol, CAS 564483-18-7), potassium acetate (4.5 g, 45.8 mmol) and bis(pinacolato)diboron (6 g, 23.6 mmol) were dissolved in 250 mL of ethanol in a round bottom flask. The system was subjected to nitrogen replacement, warmed to 80°C and reacted overnight. The system was subjected to rotary evaporation to remove ethanol and then extracted with ethyl acetate to obtain the title compound **intermediate 4** (4 g).
LC-MS (ESI): m/z = 197.1 $[M+H]^+$.

**Intermediate 5: 5-bromo-3-(difluoromethyl)-2-fluoropyridine**

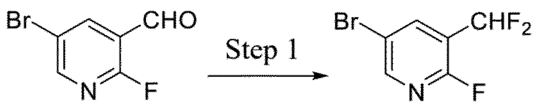


**5A** Intermediate 5

Step 1: 5-bromo-3-(difluoromethyl)-2-fluoropyridine **(intermediate 5)**

Raw material **5A** (5.00 g, 24.51 mmol) was dissolved in 100 mL of dichloromethane, and the mixture was cooled to -20°C. DAST (6.5 mL, 49.02 mmol) was added, and the resulting mixture was slowly warmed to room temperature and reacted for 2 h. Upon complete depletion of raw materials monitored by TLC, the reaction was quenched with saturated aqueous sodium bicarbonate solution and extracted with dichloromethane. The organic phase was subjected to rotary evaporation and the residue was purified by silica gel column (petroleum ether : ethyl acetate = 20 : 1) to obtain **intermediate 5** (5.00 g, 90.27%).
$^1H$ NMR (400 MHz, $CDCl_3$) $\delta$ 8.40 (dd, 1H), 8.15 (dt, 1H), 6.95 - 6.67 (m, 1H).

**Example 1**

1-((2',5-bis(difluoromethyl)-[3,4'-bipyridin]-6-yl)oxy)-4-fluoro-2,4-dimethylpentan-2-amine **(compound 1)**

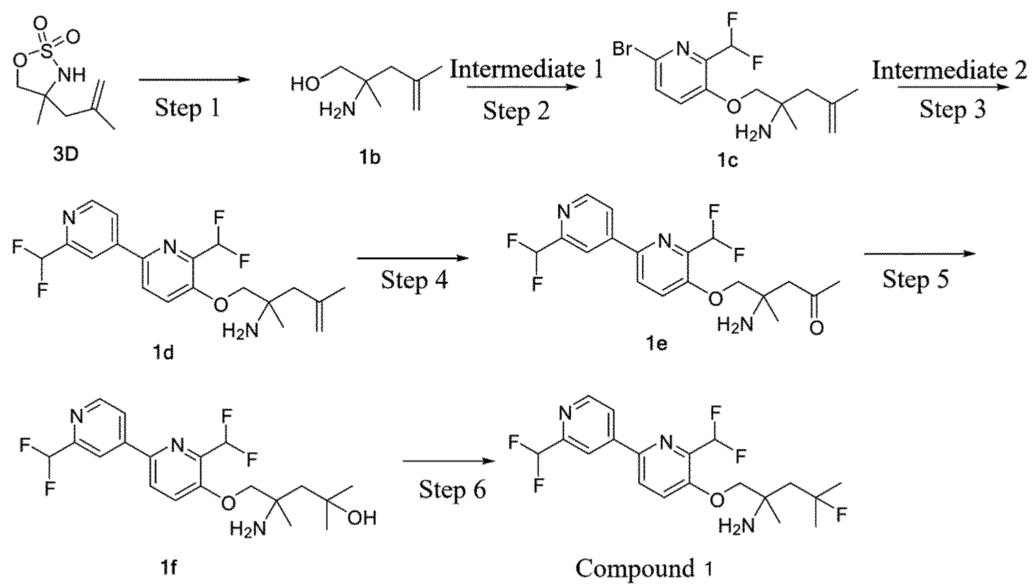


3D 1b 1c 1d 1e 1f Compound 1

Step 1: 2-amino-2,4-dimethylpent-4-en-1-ol **(1b)**

**3D** (8 g, 42 mmol) was dissolved in 500 mL of tetrahydrofuran. The system was cooled to 0°C and lithium aluminium hydride (3.99 g, 105 mmol) was slowly added. Then the mixture was warmed to room temperature and reacted for 6 h. Water (4 mL), 8 M aqueous NaOH solution, and water (12 mL) were sequentially added and the mixture was stirred for 1 h. The resulting mixture was filtered by suction to remove the solid and the resulting filtrate was subjected to rotary evaporation to obtain the target compound **1b** (crude product, 9 g), which was directly used in the next step without purification.

LC-MS (ESI): m/z = 130.2 $[M+H]^+$.

Step 2: 1-((6-bromo-2-(difluoromethyl)pyridin-3-yl)oxy)-2,4-dimethylpent-4-en-2-amine **(1c)**

Crude product **1b** (2 g) was added to a solution of potassium tert-butoxide in tetrahydrofuran (27 mL). At room temperature, the mixture was stirred for 5 min and then **intermediate 1** (4 g, 18 mmol) was added. The resulting mixture was subjected to nitrogen replacement, then heated to 80°C and reacted overnight. The system was subjected to rotary evaporation to remove the organic phase, and the residue was separated and purified by silica gel column chromatography (dichloromethane : methanol = 10 : 1) to obtain the target compound **1c**(1.1 g, 35%).

LC-MS (ESI): m/z = 335.1 and 337.1 $[M+H]^+$.

Step 3: 1-((2',6-bis(difluoromethyl)-[2,4'-bipyridin]-5-yl)oxy)-2,4-dimethylpent-4-en-2-amine **(1d)**

**Intermediate 2** (1.1 g, 3.3 mmol), 1c (880 mg, 5 mmol), potassium phosphate (9.2 g, 43 mmol), Xphos PdG2 (500 mg, 0.63 mmol, CAS: 1310584-14-5), and Xphos (650 mg, 1.36 mmol, CAS 564483-18-7) were added to a sealed tube, and 30 mL of tetrahydrofuran was added. After nitrogen replacement, the mixture was warmed to 80°C and reacted for 5 h. Upon complete depletion of raw materials monitored by TLC, the system was filtered by suction to remove the solid, which was then washed with methanol. The filtrate was collected and subjected to rotary evaporation, and the residue was separated and purified by silica gel column chromatography (dichloromethane : methanol = 10 : 1) to obtain the title compound **1d** (360 mg, 29%).

LC-MS (ESI): m/z = 384.2 $[M+H]^+$.

$^1H$ NMR (400 MHz, DMSO-$d_6$) δ 8.80 - 8.76 (m, 1H), 8.42 - 8.36 (m, 1H), 8.32 (s, 1H), 8.24 - 8.18 (m, 1H), 7.84 - 7.79 (m, 1H), 7.42 - 6.88 (m, 2H), 4.87 (s, 1H), 4.72 (s, 1H), 3.88 (s, 2H), 2.22 (s, 2H), 1.78 (s, 3H), 1.15 (s, 3H).

Step 4: 4-amino-5-((2',6-bis(difluoromethyl)-[2,4'-bipyridin]-5-yl)oxy)-4-methylpentan-2-one **(1e)**

**1d** (360 mg, 0.94 mmol) was dissolved in 20 mL of dichloromethane. The mixture was cooled to -60°C and ozone was introduced. Upon complete depletion of raw materials monitored by TLC, 1 g of triphenylphosphine was added and the system was warmed to room temperature and stirred for 15 min. The organic phase was subjected to rotary evaporation and the residue was separated and purified by silica gel column chromatography (dichloromethane :

methanol = 10 : 1) to obtain the title compound **1e** (300 mg, 83%).

LC-MS (ESI): m/z = 386.2 $[M+H]^+$.

Step 5: 4-amino-5-((2',6-bis(difluoromethyl)-[2,4'-bipyridin]-5-yl)oxy)-2,4-dimethylpentan-2-ol **(1f)**

Under nitrogen atmosphere, **1e** (300 mg, 0.78 mmol) was dissolved in 20 mL of tetrahydrofuran and the system was cooled to 0°C. A solution of methylmagnesium bromide in THF (1 mL, 3 M) was added and the mixture was slowly warmed to room temperature. Upon complete depletion of raw materials monitored by TLC, the reaction was quenched by the addition of saturated aqueous ammonium chloride solution, and extracted with dichloromethane. The organic phase was subjected to rotary evaporation to obtain the title compound **1f** (240 mg, 0.6 mmol), which was directly used in the next step.

LC-MS (ESI): m/z = 402.2 $[M+H]^+$.

Step 6: 1-((2',6-bis(difluoromethyl)-[2,4'-bipyridin]-5-yl)oxy)-4-fluoro-2,4-dimethylpentan-2-amine **(compound 1)**

Under nitrogen atmosphere, **1f** (240 mg, 0.6 mmol) was dissolved in 15 mL of dichloromethane and the mixture was cooled to -78°C. DAST (0.4 mL, 2.8 mmol) was added and the system was slowly warmed to room temperature. Upon complete depletion of raw materials monitored by TLC, the reaction was quenched by the addition of saturated aqueous sodium bicarbonate solution and extracted with dichloromethane. The organic phase was subjected to rotary evaporation and then the resulting product was separated by HPLC and lyophilized to obtain the title compound **1** (110 mg, 42%).

LC-MS (ESI): m/z = 404.2 $[M+H]^+$.

$^1H$ NMR (400 MHz, DMSO-$d_6$) δ 8.82 - 8.76 (m, 1H), 8.42 - 8.36 (m, 1H), 8.32 (s, 1H), 8.23 - 8.18 (m, 1H), 7.81 - 7.75 (m, 1H), 7.41 - 6.89 (m, 2H), 3.95 (s, 2H), 1.94 - 1.86 (m, 2H), 1.49 - 1.36 (m, 6H), 1.23 (s, 3H).

**Example 2**

1-((2',6-bis(difluoromethyl)-[2,4'-bipyridin]-5-yl)oxy)-2-methyl-3-(1-methylcyclopropyl) propan-2-amine **(compound 2)**

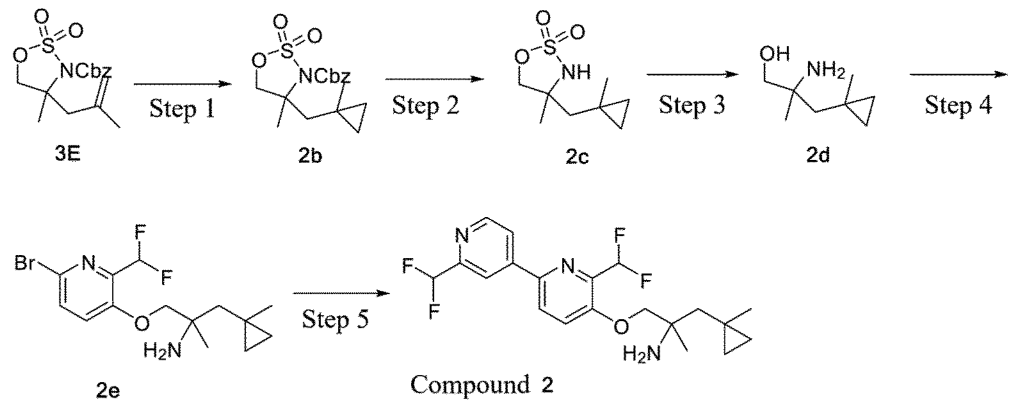


3E 2b 2c 2d

2e Compound 2

Step 1: Benzyl 4-methyl-4-((1-methylcyclopropyl)methyl)-1,2,3-oxathiazolidine-3-carboxylate 2,2-dioxide **(2b)**

Under nitrogen, diethylzinc (12 mL, 2 M toluene solution) was added to a three-necked flask and 50 mL of dichloromethane was added. The system was cooled to 0°C. Trifluoroacetic acid (1.8 mL, 24 mmol) was added. Upon complete gas evolution, diiodomethane (2 mL, 24 mmol) was added and the mixture was stirred at 0°C for 20 min. **3E** (2.6 g, 8 mmol) was added and the mixture was warmed to room temperature and stirred overnight. The reaction was quenched with water and then extracted with dichloromethane. The organic phase was subjected to rotary evaporation to obtain the title compound **2b** (1.57 g, 58%).

LC-MS (ESI): m/z = 357.1$[M+NH_4]^+$.

Step 2: 4-methyl-4-((1-methylcyclopropyl)methyl)-1,2,3-oxathiazolidine 2,2-dioxide **(2c)**

**2b** (1.57 g, 4.63 mmol) was dissolved in 50 mL of methanol and 400 mg of 10% Pd/C was added. Under

hydrogen atmosphere, the mixture was heated to 60°C and reacted overnight. Upon complete depletion of raw materials, the system was filtered by suction to remove palladium on carbon. The filtrate was collected and subjected to rotary evaporation to obtain the title compound **2c** (929 mg, 97%).

Step 3: 2-amino-2-methyl-3-(1-methylcyclopropyl)propan-1-ol **(2d)**

2c (929 mg, 4.5 mmol) was dissolved in 50 mL of tetrahydrofuran and at 0°C, lithium aluminium hydride (600 mg, 15.8 mmol) was added. The mixture was warmed to room temperature and stirred overnight. Water (0.6 mL), 10% aqueous sodium hydroxide solution (1.2 mL) and water (1.8 mL) were sequentially added. The mixture was stirred for 30 min and then filtered by suction to remove the solid. The resulting filtrate was subjected to rotary evaporation to obtain a crude containing the target compound **2d** (close to 1 g), which was directly used in the next step without further purification.

LC-MS (ESI): m/z = 144.2[M+H]$^+$.

Step 4: 1-((6-bromo-2-(difluoromethyl)pyridin-3-yl)oxy)-2-methyl-3-(1-methylcyclopropyl)propan-2-amine **(2e)**

The crude containing 2d (1 g) and a solution of 1 M potassium tert-butoxide in THF (15 mL) were stirred at room temperature for 5 min and intermediate 1 (1.13 g, 5 mmol) was added. After nitrogen replacement, the mixture was warmed to 80°C and stirred overnight. After the reaction was completed, the system was subjected to rotary evaporation and the residue was separated and purified by silica gel column chromatography (dichloromethane : methanol = 10 : 1) to obtain title compound **2e** (600 mg, 2-step total yield: 37%).

LC-MS (ESI): m/z = 349.1 and 351.1[M+H]$^+$.

Step 5: 1-((2',6-bis(difluoromethyl)-[2,4'-bipyridin]-5-yl)oxy)-2-methyl-3-(1-methylcyclopropyl) propan-2-amine (compound **2)**

**2e** (600 mg, 1.7 mmol), intermediate 2 (500 mg, 2.84 mmol), potassium phosphate (5.2 g, 24.3 mmol), Xphos PdG2 (280 mg, 0.35 mmol, CAS: 1310584-14-5), and Xphos (364 mg, 0.76 mmol, CAS 564483-18-7) were added to a sealed tube, and 20 mL of tetrahydrofuran was added. After nitrogen replacement, the mixture was warmed to 80°C and reacted for 5 h. Upon complete depletion of raw materials monitored by TLC, the system was filtered by suction to remove the solid, which was then washed with methanol. The filtrate was collected and subjected to rotary evaporation and the residue was separated and purified by silica gel column chromatography (dichloromethane : methanol = 10 : 1) and preparative HPLC and lyophilized to obtain title compound **2** (117 mg, 17%).

LC-MS (ESI): m/z = 398.2 [M+H]$^+$.

$^1$H NMR (400 MHz, DMSO-$d_6$) δ 8.80 - 8.76 (m, 1H), 8.42 - 8.36 (m, 1H), 8.32 (s, 1H), 8.23 - 8.18 (m, 1H), 7.83 - 7.78 (m, 1H), 7.37 - 6.87 (m, 2H), 3.93 (s, 2H), 1.63 - 1.49 (m, 3H), 1.44 - 1.37 (m, 1H), 1.19 (s, 3H), 1.14 (s, 3H), 0.36 - 0.14 (m, 4H).

**Examples 3 and 4**

(S)-1-((2', 6-bis(difluoromethyl)-[2,4'-bipyridin]-5-yl)oxy)-2,4-dimethylpent-4-en-2-amine and (R)-1-((2',6-bis(difluoromethyl)-[2,4'-bipyridin]-5-yl)oxy)-2,4-dimethylpent-4-en-2-amine (compound 3 and compound 4)

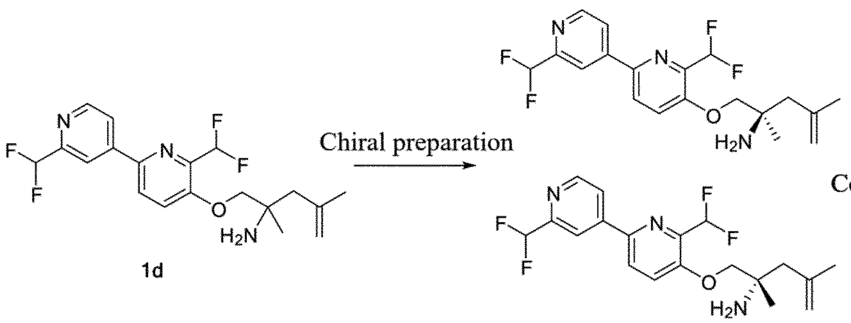


1d

Compound 3 and compound 4

**1d** (80 mg) was subjected to chiral resolution to obtain compound **3** (33.7 mg) and compound **4** (25.3 mg).

Preparation method:

instrument: SHIMADZU LC-20AP, column: DAICEL CHIRALPAK IG (250 mm × 30 mm, 10 μm); mobile phase: A: n-hexane, B: ethanol (0.1% $NH_3{\bullet}H_2O$); gradient: 8% B gradient elution; flow rate: 120 mL/min, column temperature: 25°C, wavelength: 254 nm, cycle time: 16 min; sample preparation: sample concentration: 1.5 mg/ml, ethanol solution; sample injection: 2 ml/injection. After separation, the fractions were dried on a rotary evaporator at the bath temperature of 40°C to obtain P1 (retention time: 2.658 minutes, set to be compound 3) and P2 (retention time: 4.205 minutes, set to be compound 4).

## Example 5

(S)-N-(4-(4-((2-amino-2,4-dimethylpent-4-en-1-yl)oxy)-3-cyanophenyl)pyridin-2-yl)acetamide (compound **5)**

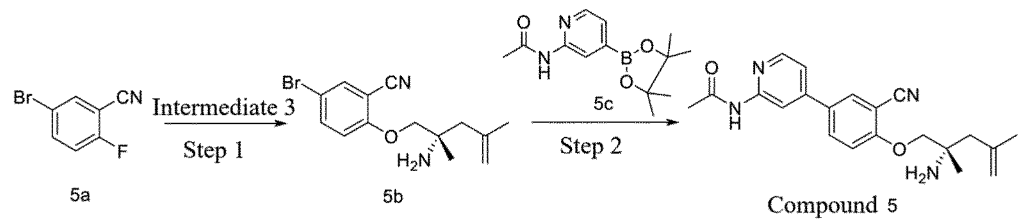


Compound 5

Step 1: (S)-2-((2-amino-2,4-dimethylpent-4-en-1-yl)oxy)-5-bromobenzonitrile **(5b)**

**Intermediate 3** (0.5 g, 3.87 mmol) and raw material **5a** (1.17 g, 5.80 mmol) were dissolved in 10 ml of anhydrous tetrahydrofuran and then a solution of 1 M potassium tert-butoxide in tetrahydrofuran (4.64 ml, 4.64 mmol) was added. The mixture was heated to 70°C and reacted for 16 h. Upon complete depletion of raw materials monitored by TLC, the reaction mixture was concentrated and the residue was purified by column chromatography (dichloromethane : methanol = 20 : 1) to obtain the target compound **5b** (0.8 g, 67%).

LC-MS (ESI): m/z = 311.1 $[M+H]^+$.

Step 2: (S)-N-(4-(4-((2-amino-2,4-dimethylpent-4-en-1-yl)oxy)-3-cyanophenyl) pyridin-2-yl)acetamide (compound **5)**

Raw material **5b** (0.7 g, 2.26 mmol), **5c** (0.81 g, 4.52 mmol, prepared with reference to WO 2010038465), and anhydrous potassium carbonate (0.94 g, 6.78 mmol) were dissolved in dioxane (10 ml) and water (2 ml). Under nitrogen protection, X-PhosPd G 2 (0.18 g, 0.23 mmol) was added and the mixture was heated to 90°C and reacted for 6 h. When the reaction was complete as shown by LC-MS, the reaction mixture was concentrated and the residue was purified by silica gel column (dichloromethane : methanol = 10 : 1) to obtain crude compound **5,** which was then separated and purified by silica gel column chromatography (acetonitrile : water = 40 : 60) to obtain compound **5** (130 mg, 15.63%).

LC-MS (ESI): m/z = 365.3 $[M+H]^+$.

$^1H$ NMR (400 MHz, DMSO-$d_6$) δ 10.55 (s, 1H), 8.39 - 8.31 (m, 2H), 8.12 (d, 1H), 7.99 (dd, 1H), 7.43 (dd, 1H), 7.38 (d, 1H), 4.86 (dd, 1H), 4.71 (s, 1H), 3.89 (s, 2H), 2.23 (s, 2H), 2.12 (s, 3H), 1.79 (s, 3H), 1.15 (s, 3H).

## Example 6

(S)-N-(4-(4-((2-amino-4-fluoro-2,4-dimethylpentyl)oxy)-3-cyanophenyl)pyridin-2-yl)acetamide (compound **6)**

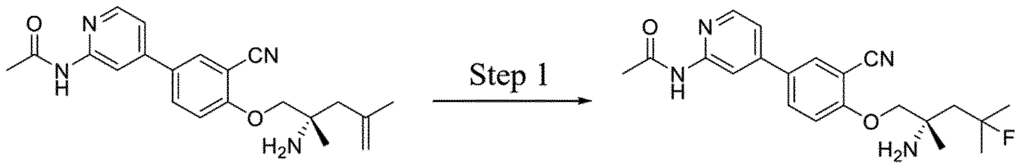


Compound 5 Compound 6

Step 1: (S)-N-(4-(4-((2-amino-4-fluoro-2,4-dimethylpentyl)oxy)-3-cyanophenyl) pyridin-2-yl)acetamide (compound **6)**

Ferric nitrate nonahydrate (0.44 g, 1.08 mmol) was dissolved in water (10 ml). The mixture was ultra-sonicated for 5 min and cooled to 0°C. Then a solution of selective fluorination reagent (0.38 g, Mol: 1.08 mmol) in 5 ml of acetonitrile was added, followed by a solution of compound **5** (100 mg, 0.27 mmol) in 5 ml of acetonitrile. Sodium borohydride (0.13 g, 3.51 mmol) was then added in portions. The mixture was reacted for 2 h. When the reaction of the raw materials was completed as shown by LC-MS, the reaction mixture was purified by column chromatography (acetonitrile : water = 40 : 60) to obtain compound 6 (90 mg, 87.60%).
LC-MS (ESI): m/z = 385.0[M+H]$^+$.
$^1$H NMR (400 MHz, DMSO-$d_6$) δ 10.56 (s, 1H), 8.39 - 8.31 (m, 2H), 8.19 (s, 1H), 8.12 (d, 1H), 8.00 (dd, 1H), 7.43 (dd, 1H), 7.37 (d, 1H), 4.04 - 3.94 (m, 2H), 2.12 (s, 3H), 1.96 (s, 1H), 1.91 (d, 1H), 1.47 (d, 3H), 1.42 (d, 3H), 1.27 (s, 3H).

**Examples 7 and 8**

Methyl-(S)-(5-((2-amino-2,4-dimethylpent-4-en-1-yl)oxy)-4-(trifluoromethyl)-[2,4'-bipyridin]-2'-yl)carbamate and methyl-(R)-(5-((2-amino-2,4-dimethylpent-4-en-1-yl)oxy)-4-(trifluoromethyl)-[2,4'-bipyridin]-2'-yl)carbamate **(compound 7 and compound 8)**

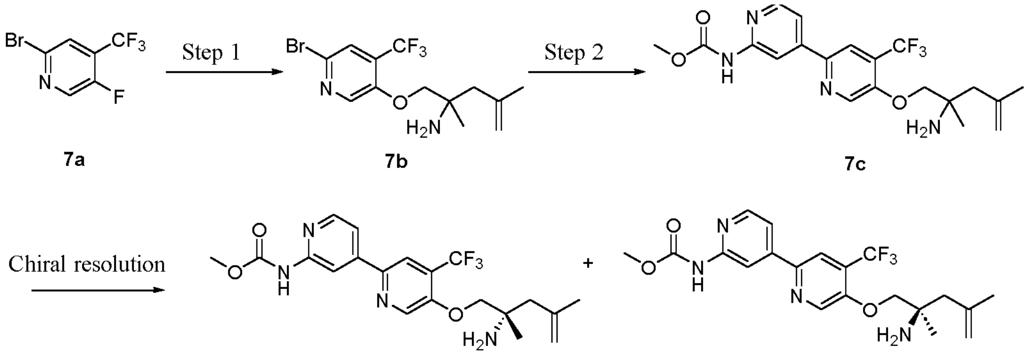


7a 7b 7c

**Compound 7, compound 8**

Step 1: 1-((6-bromo-4-(trifluoromethyl)pyridin-3-yl)oxy)-2,4-dimethylpent-4-en-2-amine **(7b)**

Compound **7a** (1 g, 4.1 mmol), compound **1b** (1 g, 0.4 mmol) and 15 mL of potassium tert-butoxide (1 M in THF) were added to a sealed tube. After nitrogen replacement, the system was warmed to 80°C and reacted for 3 h. The reaction mixture was subjected to rotary evaporation and the residue was mixed with silica gel and separated by column chromatography to obtain the target compound **7b** (1 g, 69%).
LC-MS (ESI): m/z = 353.1 and 355.1[M+H]$^+$.

Step 2: Methyl -(5-((2-amino-2,4-dimethylpent-4-en-1-yl)oxy)-4-(trifluoromethyl)-[2,4'-bipyridin]-2'-yl)carbamate (7c)

**Intermediate 4** (1 g, 2.8 mmol), **7b** (1 g, 3.6 mmol), Xphos PdG2 (400 mg, 0.5 mmol, CAS: 1310584-14-5), Xphos (500 mg, 1.05 mmol, CAS 564483-18-7), and potassium phosphate (9 g, 42.4 mmol) were added to a sealed tube, and 20 mL of tetrahydrofuran was added. After nitrogen replacement, the system was warmed to 80°C and reacted for 3 h. The resulting reaction mixture was mixed with silica gel, separated by column chromatography and preparative HPLC, and then lyophilized to obtain compound **7c** (100 mg, 9%).
LC-MS (ESI): m/z = 425.2 [M+H]$^+$.
$^1$H NMR (400 MHz, DMSO-$d_6$) δ 10.25 (s, 1H), 8.81 (s, 1H), 8.53 (s, 1H), 8.38 - 8.34 (m, 1H), 8.17 (s, 1H), 7.75 - 7.71 (m, 1H), 4.86 (s, 1H), 4.69 (s, 1H), 4.04 (s, 2H), 3.71 (s, 3H), 2.20 (s, 2H), 1.78 (s, 3H), 1.13 (s, 3H).
**7c** (90 mg) was subjected to chiral resolution to obtain compound **7** (29.7 mg) and compound **8** (31.0 mg).
Preparation method: instrument: Waters 150 AP, column: DAICEL CHIRALCEL AD (250 mm × 30 mm, 10 μm); mobile phase: (phase A: $CO_2$, phase B: EtOH (0.1% $NH_3$•$H_2O$)); gradient: 50% mobile phase B isocratic elution; flow rate: 80 mL/min, back pressure: 100 bar, column temperature: 35°C; wavelength: 220 nm; cycle time: 9.2 min; sample preparation: sample concentration: 5 mg/ml, acetonitrile solution; sample injection: 2 ml/injection. After separation, the fractions were dried on a rotary evaporator at the bath temperature of 40°C to obtain P1 (retention time: 0.846

minutes, set to be compound 7) and P2 (retention time: 1.441 minutes, set to be compound 8).

**Example 9**

Methyl (S)-(5-((2-amino-2,4-dimethylpent-4-en-1-yl)oxy)-6-(difluoromethyl)-[2,4'-bipyridin]-2'-yl)carbamate **(compound 9)**

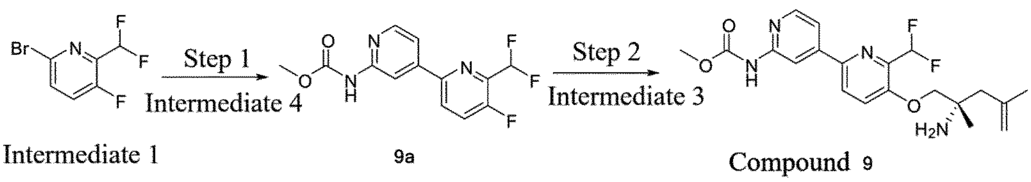


Intermediate 1 9a Compound 9

Step 1: Methyl (6-(difluoromethyl)-5-fluoro-[2,4'-bipyridin]-2'-yl)carbamate (9a)

**Intermediate 4** (500 mg, 1.8 mmol), **intermediate 1** (500 mg, 2.2 mmol), Xphos PdG2 (200 mg, 0.25 mmol, CAS: 1310584-14-5), Xphos (250 mg, 0.52 mmol, CAS 564483-18-7), and potassium phosphate (4.5 g, 21.2 mmol) were added to a sealed tube, and 20 mL of tetrahydrofuran was added. After nitrogen replacement, the system was warmed to 80°C and reacted for 3 h. The resulting reaction mixture was mixed with silica gel and separated by column chromatography to obtain compound 9a (197 mg, 37%).

LC-MS (ESI): m/z = 298.1 $[M+H]^+$.

Step 2: Methyl (S)-(5-((2-amino-2,4-dimethylpent-4-en-1-yl)oxy)-6-(difluoromethyl)-[2,4'-bipyridin]-2'-yl)carbamate **(compound 9)**

Compound **9a** (197 mg, 0.66 mmol), **intermediate 3** (90 mg, 0.7 mmol) and 1 mL of potassium tert-butoxide (1 M in THF) were added to a sealed tube. After nitrogen replacement, the system was warmed to 80°C and reacted for 3 h. The reaction solution was concentrated to dryness and the residue was purified by preparative separation to obtain the target **compound 9** (30 mg, 11%).

LC-MS (ESI): m/z = 407.1 $[M+H]^+$.

$^1$H NMR (400 MHz, DMSO-$d_6$) δ 10.23 (s, 1H), 8.49 (s, 1H), 8.38 - 8.33 (m, 1H), 8.18 - 8.13 (m, 1H), 7.79 - 7.75 (m, 1H), 7.69 - 7.63 (m, 1H), 7.40 - 7.08 (m, 1H), 4.86 (s, 1H), 4.71 (s, 1H), 3.88 (s, 2H), 3.71 (s, 3H), 2.23 (s, 2H), 1.78 (s, 3H), 1.14 (s, 3H).

**Example 10**

Methyl (S)-(5-((2-amino-2,4-dimethylpent-4-en-1-yl)oxy)-6-methyl-[2,4'-bipyridin]-2'-yl)carbamate **(compound 10)**

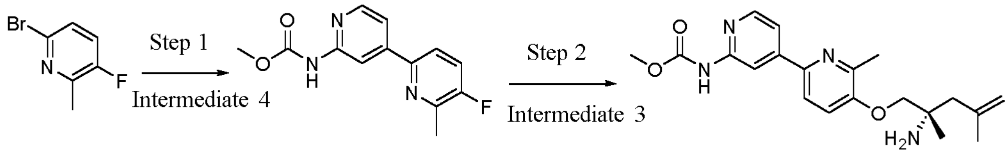


**10a** **10b** **Compound 10**

Step 1: Methyl (5-fluoro-6-methyl-[2,4'-bipyridin]-2'-yl)carbamate **(10b)**

**10a** (1.5 g, 7.89 mmol), **intermediate 4** (2.3 g, 11.84 mmol), potassium phosphate (21.8 g, 102.57 mmol), Xphos PdG2 (1.24 g, 1.58 mmol, CAS: 1310584-14-5), and Xphos (1.5 g, 3.16 mmol, CAS 564483-18-7) were added to a sealed tube, and 60 mL of tetrahydrofuran was added. After nitrogen replacement, the mixture was warmed to 80°C and reacted for 5 h. Upon complete depletion of raw materials monitored by TLC, the system was filtered by suction to remove the solid, which was then washed with methanol. The filtrate was collected and subjected to rotary evaporation and the residue was purified by silica gel column (dichloromethane : methanol = 10 : 1) to obtain the title compound **10b**

(1.4 g, 68%).

LC-MS (ESI): m/z = 262.0 $[M+H]^+$.

Step 2: Methyl (S)-(5-((2-amino-2,4-dimethylpent-4-en-1-yl)oxy)-6-methyl-[2,4'-bipyridin]-2'-yl)carbamate **(compound 10)**

**Intermediate 3** (495 mg, 3.83 mmol) was added to 15 mL of DMF solution. Under an ice bath, NaH (275 mg, 11.49 mmol) was added and the mixture was stirred for 10 min. Then compound **10b** (1 g, 3.83 mmol) was added. After nitrogen replacement, the mixture was reacted at 0°C for 1 h. The reaction was quenched by the addition of water and the mixture was then extracted with ethyl acetate. The organic phase was subjected to rotary evaporation and the residue was purified by column chromatography (dichloromethane : methanol = 10 : 1) to obtain **compound 10** (110 mg).

LC-MS (ESI): m/z = 371.2 $[M+H]^+$.

$^1H$ NMR (400 MHz, DMSO-$d_6$) $\delta$ 10.16 (s, 1H), 8.47 (s, 1H), 8.29 (d, 1H), 7.80 (d, 1H), 7.62 (dd, 1H), 7.40 (d, 1H), 4.86 (s, 1H), 4.70 (s, 1H), 3.75 (s, 2H), 3.70 (s, 3H), 2.50 (s, 3H), 2.23 (s, 2H), 1.78 (s, 3H), 1.58 (s, 2H), 1.14 (s, 3H).

**Example 11**

Methyl (S)-(5-((2-amino-2,4-dimethylpent-4-en-1-yl)oxy)-4-(difluoro-l3-methyl)-[2,4'-bipyridin]-2'-yl)carbamate **(compound 11)**

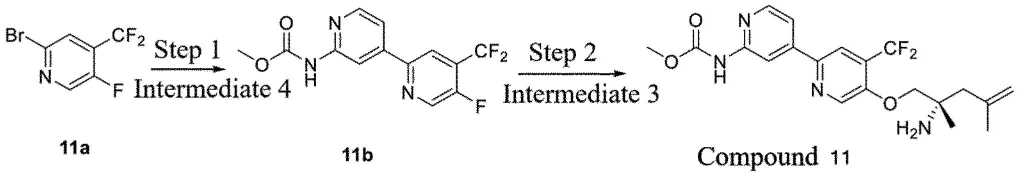


**11a** **11b** Compound 11

Step 1: Methyl (4-(difluoro-l3-methyl)-5-fluoro-[2,4'-bipyridin]-2'-yl)carbamate **(11b)**

**11a** (1.0 g, 4.42 mmol), **intermediate 4** (1.3 g, 6.64 mmol), potassium phosphate (12.2 g, 57.52 mmol), Xphos PdG2 (0.7 g, 0.88 mmol, CAS: 1310584-14-5), and Xphos (0.85 g, 1.77 mmol, CAS 564483-18-7) were added to a sealed tube, and 30 mL of tetrahydrofuran was added. After nitrogen replacement, the mixture was warmed to 80°C and reacted for 5 h. Upon complete depletion of raw materials monitored by TLC, the system was filtered by suction to remove the solid, which was then washed with methanol. The filtrate was collected and subjected to rotary evaporation and the residue was purified by silica gel column (dichloromethane : methanol = 10 : 1) to obtain the title compound **11b** (600 mg, 46%).

LC-MS (ESI): m/z = 298.0 $[M+H]^+$.

Step 2: Methyl (S)-(5-((2-amino-2,4-dimethylpent-4-en-1-yl)oxy)-4-(difluoro-l3-methyl)-[2,4'-bipyridin]-2'-yl)carbamate **(compound 11)**

**Intermediate 3** (260 mg, 2.02 mmol) was added to a solution of potassium tert-butoxide in tetrahydrofuran (3 mL) and the mixture was stirred at room temperature for 5 min. A solution of compound **11b** (600 mg, 2.03 mmol) in tetrahydrofuran (6 mL) was added. After nitrogen replacement, the mixture was heated to 80°C and reacted overnight. The system was subjected to rotary evaporation to remove the organic phase and the residue was purified by column chromatography (dichloromethane : methanol = 10 : 1) to obtain compound 11 (20 mg).

LC-MS (ESI): m/z = 407.1 $[M+H]^+$.

$^1H$ NMR (400 MHz, DMSO-$d_6$) $\delta$ 10.24 (s, 1H), 8.68 (s, 1H), 8.52 (s, 1H), 8.35 (d, 1H), 8.07 (s, 1H), 7.69 (dd, 1H), 7.48 - 7.21 (m, 1H), 4.86 (s, 1H), 4.71 (s, 1H), 3.99 (s, 2H), 3.71 (s, 3H), 2.22 (s, 2H), 1.79 (s, 3H), 1.13 (s, 3H).

**Example 12**

(S)-4-amino-5-((2',6-bis(difluoromethyl)-[2,4'-bipyridin]-5-yl)oxy)-2,4-dimethylpentan-2-ol **(compound 12)**

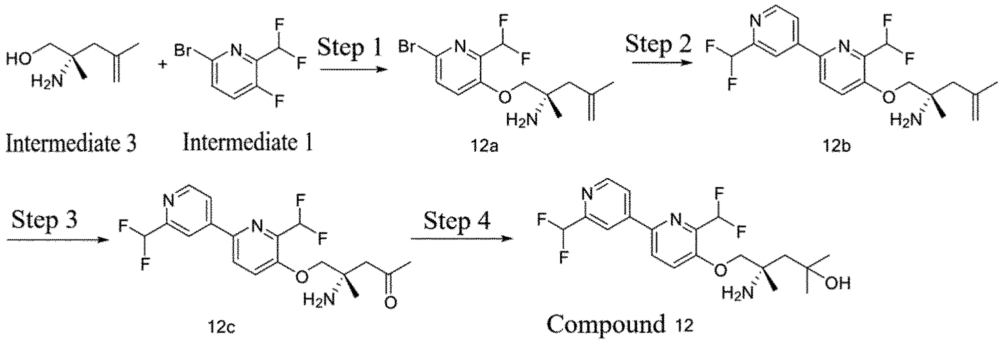


Step 1: (S)-1-((6-bromo-2-(difluoromethyl)pyridin-3-yl)oxy)-2,4-dimethylpent-4-en-2-amine **(12a)**

**Intermediate 3** (1 g, 7.7 mmol), **intermediate 1** (1.6 g, 7.1 mmol) and 12 mL of potassium tert-butoxide (1 M in THF) were added to a sealed tube. After nitrogen replacement, the system was warmed to 80°C and reacted for 3 h. The system was cooled to room temperature, mixed with silica gel, and separated by column chromatography (petroleum ether : ethyl acetate = 1 : 1 to ethyl acetate ) to obtain the target compound **12a** (500 mg, 21%).
LC-MS (ESI): m/z = 355.1 $[M+H]^+$.

Step 2: (S)-1-((2',6-bis(difluoromethyl)-[2,4'-bipyridin]-5-yl)oxy)-2,4-dimethylpent-4-en-2-amine **(12b)**

Compound **12a** (500 mg, 1.5 mmol), **intermediate 2** (620 mg, 3.6 mmol), Xphos PdG2 (200 mg, 0.25 mmol, CAS: 1310584-14-5), Xphos (250 mg, 0.52 mmol, CAS 564483-18-7), and potassium phosphate (4.5 g, 21.2 mmol) were added to a sealed tube, and 20 mL of tetrahydrofuran was added. After nitrogen replacement, the system was warmed to 80°C and reacted for 3 h. The resulting reaction mixture was mixed with silica gel and separated by column chromatography (petroleum ether : ethyl acetate = 1 : 1 to ethyl acetate) to obtain compound **12b** (350 mg, 61%).
LC-MS (ESI): m/z = 384.2 $[M+H]^+$.

Step 3: (S)-4-amino-5-((2',6-bis(difluoromethyl)-[2,4'-bipyridin]-5-yl)oxy)-4-methylpentan-2-one **(12c)**

Compound **12b** (350 mg, 0.91 mmol) was dissolved in 20 mL of dichloromethane. The system was cooled to -78°C and ozone was introduced. Upon complete depletion of raw materials monitored by TLC, excess triphenylphosphine was added and the mixture was slowly warmed to room temperature, mixed with silica gel, and separated by column chromatography (petroleum ether : ethyl acetate = 1 : 1 to ethyl acetate) to obtain compound 12c (310 mg, 89%).
LC-MS (ESI): m/z = 386.1 $[M+H]^+$.

Step 4: (S)-4-amino-5-((2',6-bis(difluoromethyl)-[2,4'-bipyridin]-5-yl)oxy)-2,4-dimethylpentan-2-ol **(compound 12)**

Compound 12c (160 mg, 0.42 mmol) was dissolved in 10 mL of tetrahydrofuran and the mixture was subjected to nitrogen replacement. At 0°C, 1.4 mL of methylmagnesium chloride (3 M, dissolved in THF) was added. The mixture was slowly warmed to room temperature, and the reaction was quenched by the addition of saturated aqueous ammonium chloride solution. The system was subjected to rotary evaporation to remove the organic phase, extracted with dichloromethane and then subjected to rotary evaporation. The residue was purified by preparative separation and then lyophilized to obtain compound 12 (30 mg, 18%).
LC-MS (ESI): m/z = 402.2 $[M+H]^+$.
$^1H$ NMR (400 MHz, DMSO-$d_6$) δ 8.81 - 8.78 (m, 1H), 8.42 - 8.37 (m, 1H), 8.32 (s, 1H), 8.23 - 8.19 (m, 1H), 7.81 - 7.74 (m, 1H), 7.41 - 6.88 (m, 2H), 4.02 - 3.91 (m, 2H), 1.71 (d, 1H), 1.60 (d, 1H), 1.27 (s, 3H), 1.23 (s, 3H), 1.16 (s, 3H).

**Example 13**

(S)-N-(4-(4-((2-amino-2,4-dimethylpent-4-en-1-yl)oxy)-3-(trifluoromethyl) phenyl)pyridin-2-yl)acetamide **(compound 13)**

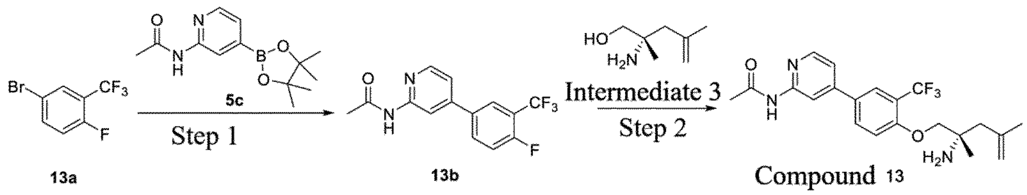


13a 13b Compound 13

Step 1: Tert-butyl N-(4-(4-fluoro-3-(trifluoromethyl)phenyl)pyridin-2-yl)acetamide **(13b)**

**13a** (1.5 g, 6.17 mmol), **5c** (1.93 g, 7.40 mmol), $Pd_3(dba)_2$ (485.0 mg, 0.617 mmol), X-Phos (588.6 mg, 1.23 mmol), and $K_3PO_4$ (13.0 g, 61.7 mmol) were sequentially added to a single-necked flask and then THF (40 mL) was added. The system was subjected to nitrogen replacement 3 times and reacted at 80°C for 4 hours. After the reaction was completed, THF was removed by rotary evaporation. Water (100 mL) was added and the mixture was extracted with ethyl acetate (100 mL) twice. The organic phases were combined and dried over anhydrous sodium sulphate, and the solvent was removed. The residue was then separated by column chromatography (petroleum ether : ethyl acetate (v/v) = 2 : 1) to obtain the title compound **13b** as a white solid (1.45 g, 78.8%).

LC-MS (ESI): m/z = 299.1 $[M+H]^+$.

Step 2: (S)-N-(4-(4-((2-amino-2,4-dimethylpent-4-en-1-yl)oxy)-3-(trifluoromethyl) phenyl)pyridin-2-yl)acetamide **(compound 13)**

**Intermediate 3** (300.0 mg, 2.32 mmol) was added to a sealed tube and then THF (20 mL) was added. Subsequently, **13b** (831.6 mg, 2.79 mmol) and potassium tert-butoxide (781.3 mg, 6.97 mmol) were sequentially added and the mixture was purged with nitrogen for 2 minutes and reacted at 80°C for 4 hours. After the reaction was completed, THF was removed by rotary evaporation. Water (50 mL) was added and the mixture was extracted with ethyl acetate (50 mL) twice. The organic phases were combined and dried over anhydrous sodium sulphate, and the solvent was removed. The residue was then separated by column chromatography (dichloromethane : methanol (v/v) = 20 : 1) to obtain the title compound **13** (300 mg, 31.7%).

$^1H$ NMR (400 MHz, $CDCl_3$) δ 8.43 (s, 1H), 8.29 (d, 1H), 8.17 (s, 1H), 7.88 (d, 1H), 7.81 (dd, 1H), 7.22 (dd, 1H), 7.05 (d, 1H), 4.97-4.93 (m, 1H), 4.79-4.77 (m, 1H), 3.85 (q, 2H), 2.38-2.30 (m, 2H), 2.24 (s, 3H), 1.82 (s, 3H), 1.27 (s, 3H).

LC-MS (ESI): m/z = 408.2 $[M+H]^+$.

**Example 14**

(S)-N-(4-(4-((2-amino-4-fluoro-2,4-dimethylpentyl)oxy)-3-(trifluoromethyl) phenyl)pyridin-2-yl)acetamide (compound **14)**

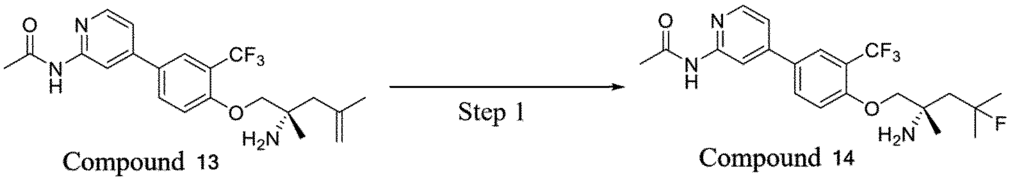


Compound 13 Compound 14

Step 1: (S)-N-(4-(4-((2-amino-4-fluoro-2,4-dimethylpentyl)oxy)-3-(trifluoromethyl)phenyl)pyridin-2-yl)acetamide (compound **14)**

Ferric nitrate nonahydrate (1.20 g, 2.95 mmol) was dissolved in water (15 mL). The mixture was ultra-sonicated for 5 min and then acetonitrile (15 mL) was added. The mixture was cooled to 0°C and subjected to nitrogen replacement 3 times. Then a selective fluorination reagent (1.04 g, 2.95 mmol) was added, followed by a solution of compound **13** (300 mg, 0.74 mmol) in 5 mL of acetonitrile. Sodium borohydride (365.0 mg, 9.58 mmol) was added in portions. The mixture was reacted for 2 h. When the reaction of the raw materials was completed as shown by LC-MS, ammonia water (2 mL) was added to quench the reaction, followed by extraction with DCM twice. The organic phases were combined and dried over anhydrous sodium sulphate and the solvent was removed. The residue was separated by column chromatography (dichloromethane : methanol = 20 : 1) to obtain compound **14** (200 mg, 63.5%).

$^1H$ NMR (400 MHz, $CDCl_3$) δ 8.43 (s, 1H), 8.29 (d, 1H), 8.09 (s, 1H), 7.87 (d, 1H), 7.81 (dd, 1H), 7.22 (dd, 1H),

7.07 (d, 1H), 4.00-3.96 (m, 2H), 2.24 (s, 3H), 2.02 (dd, 2H), 1.51 (d, 3H), 1.45 (d, 3H), 1.38 (s, 3H).
LC-MS (ESI): m/z = 428.2 $[M+H]^+$.

**Example 15**

(S)-N-(5-((2-amino-2,4-dimethylpent-4-en-1-yl)oxy)-6-(difluoromethyl)-[2,4'-bipyridin]-2'-yl)acetamide **(compound 15)**

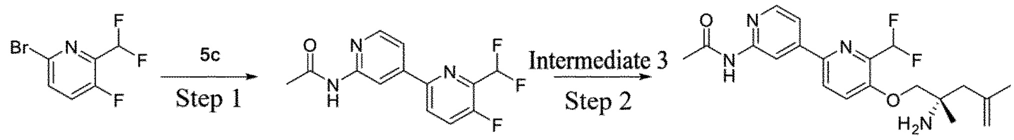


Intermediate 1 15a Compound 15

Step 1: N-(6-(difluoromethyl)-5-fluoro-[2,4'-bipyridin]-2'-yl)acetamide (15a)

**5c** (900 mg), **intermediate 1** (633 mg, 2.8 mmol), Xphos PdG2 (250 mg, 0.32 mmol), Xphos (500 mg, 1.05 mmol), and potassium phosphate (6.0 g, 28.3 mmol) were added to a sealed tube, and 30 mL of tetrahydrofuran was added. After nitrogen replacement, the system was warmed to 80°C and reacted for 3 h. The resulting reaction mixture was mixed with silica gel and separated by column chromatography to obtain compound **15a** (428 mg, 54%).
LC-MS (ESI): m/z = 282.2 $[M+H]^+$.

Step 2: (S)-N-(5-((2-amino-2,4-dimethylpent-4-en-1-yl)oxy)-6-(difluoromethyl) -[2,4'-bipyridin]-2'-yl)acetamide (compound **15)**

Compound **15a** (200 mg, 0.71 mmol), **intermediate 3** (100 mg, 0.77 mmol) and 2.5 mL of potassium tert-butoxide (1 M in THF) were added to a sealed tube. After nitrogen replacement, the system was warmed to 80°C and reacted for 3 h. The reaction mixture was purified by preparative separation to obtain compound **15** (89 mg, 32%).
LC-MS (ESI): m/z = 391.1 $[M+H]^+$.
$^1H$ NMR (400 MHz, DMSO-$d_6$) $\delta$ 10.54 (s, 1H), 8.68 (s, 1H), 8.41 - 8.37 (m, 1H), 8.16 - 8.10 (m, 1H), 7.81 - 7.75 (m, 1H), 7.72 - 7.67 (m, 1H), 7.38 - 7.07(m, 1H), 4.86 (s, 1H), 4.71 (s, 1H), 3.88 (s, 2H), 2.23 (s, 2H), 2.12 (s, 3H), 1.78 (s, 3H), 1.14 (s, 3H).

**Example 16**

(S)-N-(5-((2-amino-4-fluoro-2,4-dimethylpentyl)oxy)-6-(difluoromethyl)-[2,4'-bipyridin]-2'-yl)acetamide (compound **16)**

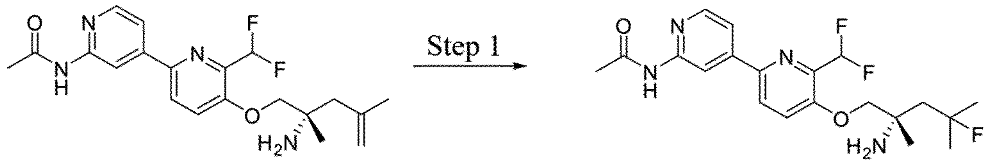


Compound 15 Compound 16

Step 1: (S)-N-(5-((2-amino-4-fluoro-2,4-dimethylpentyl)oxy)-6-(difluoromethyl) -[2,4'-bipyridin]-2'-yl)acetamide (compound **16)**

Ferric nitrate nonahydrate (133 mg, 0.33 mmol) was dissolved in water (3 mL) and the mixture was subjected to nitrogen replacement and then cooled to 0°C. Selective fluorination reagent (117 mg, 0.33 mmol) and 3 mL of acetonitrile were added. Compound **15** (35 mg, 0.09 mmol) was dissolved in 3 mL of acetonitrile and the resulting mixture was added to the system. The mixture was stirred for 5 min and then sodium borohydride (40 mg, 1.05 mmol) was added in portions. The resulting mixture was reacted for 30 min while the temperature was maintained at 0°C. Ammonia water (1 mL) was added to quench the reaction, followed by extraction with the mixed solvents of dichloromethane and methanol

(10 : 1). After rotary evaporation, the residue was purified by preparative HPLC to obtain compound **16** (10 mg, 28%).

LC-MS (ESI): m/z = 411.3 $[M+H]^+$.

$^1H$ NMR (400 MHz, DMSO-$d_6$) δ 10.55 (s, 1H), 8.69 (s, 1H), 8.43 - 8.35 (m, 1H), 8.18 - 8.10 (m, 1H), 7.78 - 7.73 (m, 1H), 7.72 - 7.65 (m, 1H), 7.40 - 7.05 (m, 1H), 3.94 (s, 2H), 2.12 (s, 3H), 1.95 - 1.86 (m, 2H), 1.50 - 1.36 (m, 6H), 1.23 (s, 3H).

**Example 17**

(S)-methyl-(6-((2-amino-2,4-dimethylpent-4-en-1-yl)oxy)-5-(difluoromethyl)-[3,4'-bipyridin]-2'-yl)carbamate **(compound 17)**

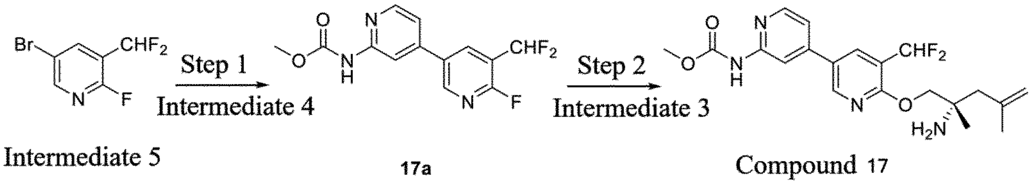


Intermediate 5 17a Compound 17

Step 1: Methyl (5-(difluoromethyl)-6-fluoro-[3,4'-bipyridin]-2'-yl)carbamate **(17a)**

**Intermediate 5** (2.00 g, 8.85 mmol), **intermediate 4** (2.08 g, 10.62 mmol), Xphos PdG2 (1.40 g, 1.78 mmol, CAS: 1310584-14-5), Xphos (1.70 g, 3.56 mmol, CAS 564483-18-7), and potassium phosphate (22.00 g, 103.77 mmol) were added to a sealed tube, and 100 mL of tetrahydrofuran was added. After nitrogen replacement, the system was warmed to 80°C and reacted for 16 h. The resulting reaction mixture was mixed with silica gel and separated by column chromatography to obtain compound **17a** (2.30 g, 87.44%).

LC-MS (ESI): m/z = 298.1 $[M+H]^+$.

Step 2: Methyl (S)-(6-((2-amino-2,4-dimethylpent-4-en-1-yl)oxy)-5-(difluoromethyl)-[3,4'-bipyridin]-2'-yl)carbamate **(compound 17)**

Compound **17a** (2.30 g, 7.74 mmol), **intermediate 3** (840 mg, 6.50 mmol) and 20 mL of potassium tert-butoxide (1 M in THF) were added to a sealed tube. After nitrogen replacement, the system was warmed to 80°C and reacted for 16 h. The reaction mixture was mixed with silica gel and separated by column chromatography to obtain a crude, which was purified by preparative separation to obtain the target **compound 17** (190 mg, 7.31%).

LC-MS (ESI): m/z = 407.1 $[M+H]^+$.

$^1H$ NMR (400 MHz, DMSO-$d_6$) δ 10.29 (s, 1H), 8.69 (s, 1H), 8.35 (d, 1H), 8.22 (s, 1H), 8.10 (s, 1H), 7.44 (dd, 1H), 7.41 - 7.14 (m, 1H), 4.85 (s, 1H), 4.71 (s, 1H), 4.13 (s, 2H), 3.71 (s, 3H), 2.22 (s, 2H), 1.79 (s, 3H), 1.12 (s, 3H).

**Example 18**

(S)-methyl-(6-((2-amino-4-fluoro-2,4-dimethylpentyl)oxy)-5-(difluoromethyl) -[3,4'-bipyridin]-2'-yl)carbamate **(compound 18)**

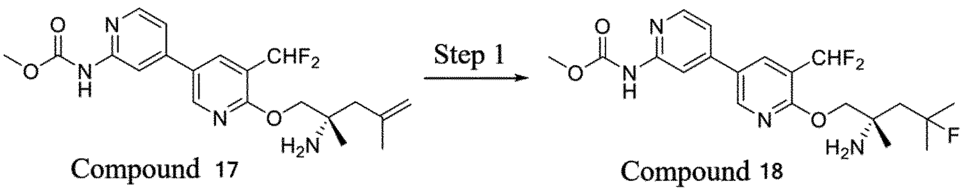


Compound 17 Compound 18

Ferric nitrate (680 mg, 1.67 mmol) was dissolved in water (5 mL). The mixture was ultra-sonicated for 5 min and cooled to 0°C. Then a solution of selective fluorination reagent (590 mg, 1.67 mmol) in 5 mL of acetonitrile was added, followed by a solution of **compound 17** (170 mg, 0.42 mmol) in 5 mL of acetonitrile. Sodium borohydride (210

mg, 5.45 mmol) was then added in portions. The mixture was reacted for 1 h. When the reaction of the raw materials was completed as shown by LC-MS, the reaction mixture was diluted by the addition of water and then extracted with dichloromethane. The organic phases were combined, dried and concentrated to obtain a crude, which was purified by preparative separation to obtain the target **compound 18** (80 mg, 44.85%).

LC-MS (ESI): m/z = 427.2[M+H]$^+$.

$^1$H NMR (400 MHz, $CD_3OD$) $\delta$ 8.63 (s, 1H), 8.30 (d, 1H), 8.22 (s, 1H), 8.15 (d, 1H), 7.34 (dd, 1H), 7.22 - 6.94 (m, 1H), 4.38 (s, 2H), 3.80 (s, 3H), 2.05 - 2.04 (m, 1H), 2.00 -1.99 (m, 1H), 1.49 (s, 3H), 1.43 (s, 3H), 1.35 (s, 3H).

**Example 19**

Methyl (S)-(5-((2-amino-4-fluoro-2,4-dimethylpentyl)oxy)-6-(difluoromethyl) -[2,4'-bipyridin]-2'-yl)carbamate (compound 19)

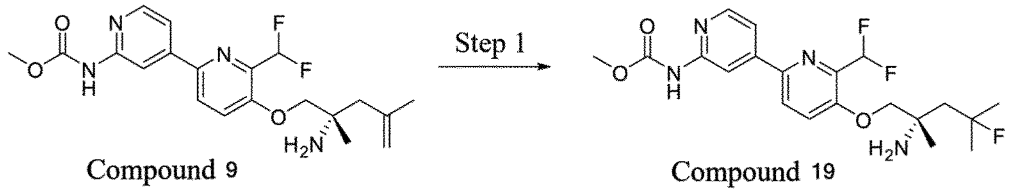


Compound 9 Compound 19

Step 1: Methyl (S)-(5-((2-amino-4-fluoro-2,4-dimethylpentyl)oxy)-6-(difluoromethyl)-[2,4'-bipyridin]-2'-yl)carbamate (compound **19)**

Ferric nitrate nonahydrate (324 mg, 0.8 mmol) was dissolved in water (7 mL) and the mixture was subjected to nitrogen replacement and then cooled to 0°C. Selective fluorination reagent (284 mg, 0.8 mmol) and 7 mL of acetonitrile were added and **compound 9** (81 mg, 0.2 mmol) was dissolved in 7 mL of acetonitrile. The resulting mixture was added to the system. The resulting mixture was stirred for 5 min and then sodium borohydride (100 mg, 2.6 mmol) was added in portions. The reaction mixture was reacted for 30 min while the temperature was maintained at 0°C. Ammonia water (2.5 mL) was added to quench the reaction, followed by extraction with the mixed solvents of dichloromethane : methanol (10 : 1). After rotary evaporation, the residue was purified by preparative HPLC to obtain compound **19** (9 mg, 11%).

LC-MS (ESI): m/z = 427.2 [M+H]$^+$.

$^1$H NMR (400 MHz, DMSO-$d_6$) $\delta$ 10.25 (s, 1H), 8.49 (s, 1H), 8.38 - 8.33 (m, 1H), 8.19 - 8.13 (m, 1H), 7.78 - 7.72 (m, 1H), 7.68 - 7.64 (m, 1H), 7.38 - 7.09 (m, 1H), 3.94 (s, 2H), 3.71 (s, 3H), 1.95 - 1.86 (m, 2H), 1.50 - 1.37 (m, 6H), 1.23 (s, 3H).

**Example** 20

Methyl (S)-(5-((2-amino-4-fluoro-2,4-dimethylpentyl)oxy)-4-(difluoromethyl) -[2,4'-bipyridin]-2'-yl)carbamate (compound 20)

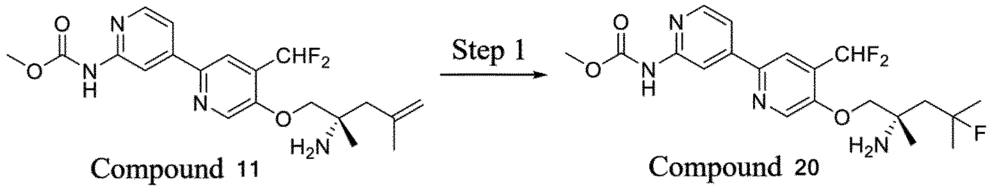


Compound 11 Compound 20

Ferric nitrate nonahydrate (180 mg, 0.44 mmol) was dissolved in water (3 mL). The mixture was ultra-sonicated for 5 min and cooled to 0°C. Then a solution of selective fluorination reagent (157 mg, 0.44 mmol) in 3 mL of acetonitrile was added, followed by a solution of **compound** 11 (45 mg, 0.11 mmol) in 3 mL of acetonitrile. Sodium borohydride (55 mg, 1.44 mmol) was then added in portions. The mixture was reacted for 1 h. When the reaction of the raw materials was completed as shown by LC-MS, the reaction mixture was diluted by the addition of water and then extracted with

dichloromethane. The organic phases were combined, dried and concentrated to obtain a crude, which was purified by preparative separation to obtain **compound 20** (2.4 mg, 5%).

LC-MS (ESI): m/z = 427.2[M+H]$^+$.

$^1$H NMR (400 MHz, $CD_3OD$) $\delta$ 8.58 (s, 1H), 8.49 (s, 1H), 8.33 (d, 1H), 8.06 (s, 1H), 7.66 (d, 1H), 7.31 - 7.04 (m, 1H), 4.17 (s, 2H), 3.80 (s, 3H), 2.07 (d, 1H), 2.02 (d, 1H), 1.50 (s, 3H), 1.44 (s, 3H), 1.38 (s, 3H).

**Example 21**

Methyl (S)-(5-((2-amino-4-fluoro-2,4-dimethylpentyl)oxy)-6-methyl-[2,4'-bipyridin]-2'-yl) carbamate **(compound 21)**

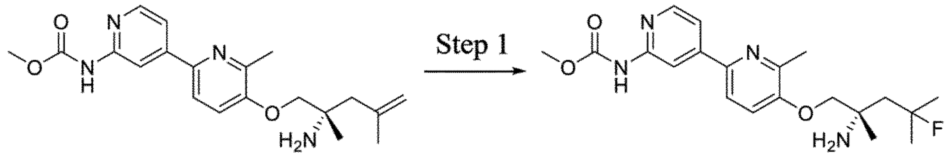


Compound 10 Compound 21

Ferric nitrate nonahydrate (88 mg, 0.22 mmol) was dissolved in water (2 mL). The mixture was ultra-sonicated for 5 min and cooled to 0°C. Then a solution of selective fluorination reagent (76 mg, 0.22 mmol) in 2 mL of acetonitrile was added, followed by a solution of **compound 10** (20 mg, 0.05 mmol) in 2 mL of acetonitrile. Sodium borohydride (30 mg, 0.79 mmol) was then added in portions. The mixture was reacted for 1 h. When the reaction of the raw materials was completed as shown by LC-MS, the reaction mixture was diluted by the addition of water and then extracted with dichloromethane. The organic phases were combined, dried and concentrated to obtain a crude, which was purified by preparative separation to obtain **compound 21** (10 mg, 47%).

LC-MS (ESI): m/z = 391.3 [M+H]$^+$.

$^1$H NMR (400 MHz, $CD_3OD$) $\delta$ 8.42 (s, 1H), 8.27 (d, 1H), 7.76 (d, 1H), 7.61 (d, 1H), 7.40 (d, 1H), 3.98 (s, 2H), 3.80 (s, 3H), 2.57 (s, 3H), 2.09 (s, 1H), 2.04 (s, 1H), 1.50 (d, 3H), 1.45 (d, 3H), 1.40 (s, 3H).

**Example 22**

1-(((2',6-bis(difluoromethyl)-[2,4'-bipyridin]-5-yl)oxy)methyl)cyclopentan-1-amine (**compound 22**)

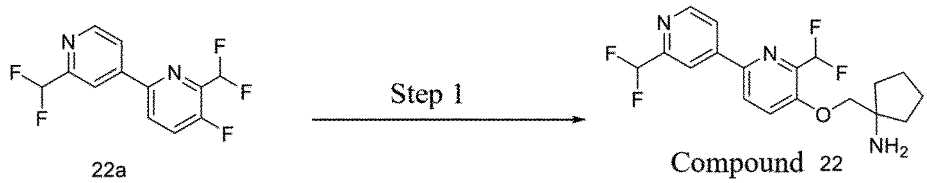


22a Compound 22

Step 1: 1-(((2',6-bis(difluoromethyl)-[2,4'-bipyridin]-5-yl)oxy)methyl) cyclopentan-1-amine (compound **22**)

Compound **22a** (50 mg, 0.15 mmol, synthesized with reference to WO 2017059085), 1-amino-1-hydroxymethylcyclopentane (26 mg, 0.22 mmol) and potassium tert-butoxide (50 mg, 0.45 mmol) were dissolved in THF (5 mL). The mixture was reacted at 70°C for 10 hours. When the reaction was complete as shown by LC-MS, the reaction mixture was concentrated and the residue was separated by column chromatography (dichloromethane : methanol = 20 : 1) to obtain compound **22** (20 mg, 36%).

LC-MS (ESI): m/z = 370.3 [M+H]$^+$.

$^1$H NMR (400 MHz, DMSO-$d_6$) $\delta$ 8.81-8.46 (m, 1H), 8.34 (s, 1H), 8.24 (d, 1H), 8.16 (s, 2H), 7.89 (d, 1H), 7.51 (t, 1H), 7.05 (t, 1H), 4.25 (s, 2H), 2.08 - 1.55 (m, 8H).

## Example 23

(S)-5-(2-acetamidopyridin-4-yl)-2-((2-amino-2,4-dimethylpent-4-en-1-yl)oxy)benzamide (compound **23**)

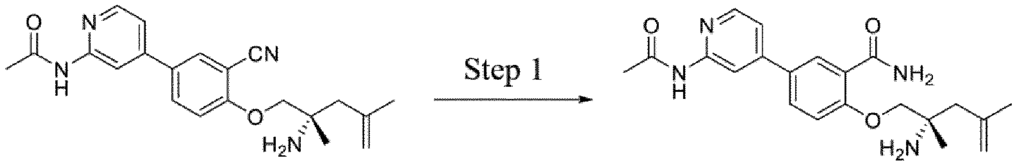


Compound 5 Compound 23

Step 1: (S)-5-(2-acetamidopyridin-4-yl)-2-((2-amino-2,4-dimethylpent-4-en-1-yl)oxy)benzamide (compound **23**)

Compound **5** (100 mg, 0.27 mmol), potassium carbonate (110 mg, 0.81 mmol), 37% hydrogen peroxide (50 mg, 0.54 mmol), and dimethyl sulphoxide (21 mg, 0.27 mmol) were dissolved in methanol (5 mL) and the mixture was reacted at room temperature for 3 hours and then purified by C-18 reverse phase column chromatography (acetonitrile : water = 40 : 60) to obtain compound **23** (50 mg, 48%).
LC-MS (ESI): m/z = 383.5[M+H]$^+$.
$^1$H NMR (400 MHz, DMSO-$d_6$) $\delta$ 10.52 (s, 1H), 8.37 (s, 1H), 8.33-8.10 (m, 2H), 8.04 (s, 1H), 7.83 (d, 1H), 7.67 (s, 1H), 7.38 -7.26 (m, 2H), 4.87 (s, 1H), 4.72 (s, 1H), 3.90 (s, 2H), 2.21 (d, 2H), 2.12 (s, 3H), 1.79 (s, 3H), 1.14 (s, 3H).

## Example 24

(S)-N-(4-(4-((2-amino-2,4-dimethylpent-4-en-1-yl)oxy)-3-cyanophenyl) pyridin-2-yl)-3,3-difluorocyclobutane-1-carboxamide (compound **24**)

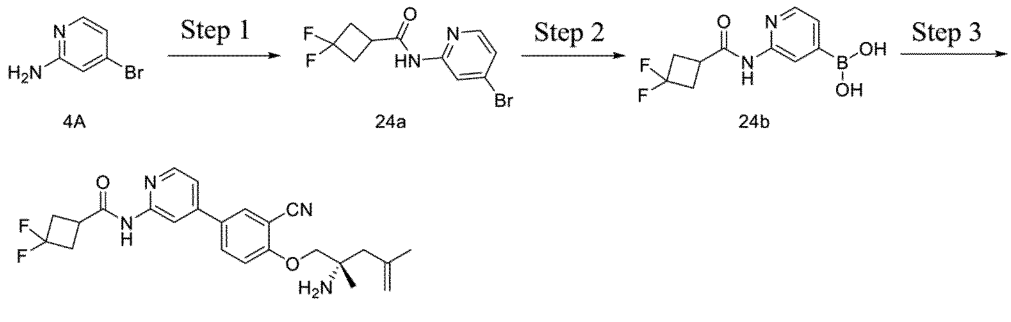


4A 24a 24b

Compound 24

Step 1: N-(4-bromopyridin-2-yl)-3,3-difluorocyclobutane-1-carboxamide **(24a)**

3,3-difluorocyclobutane-1-carboxylic acid (790 mg, 5.78 mmol) was dissolved in dichloromethane (10 mL). Oxalyl chloride (810 mg, 6.36 mmol) was diluted with dichloromethane (10 mL) and the mixture was slowly added dropwise to the reaction solution. The reaction mixture was reacted for 2 hours, concentrated, then diluted with dichloromethane (10 mL) and added dropwise to a solution of compound **4A** (1 g, 5.78 mmol) and triethylamine (0.88 g, 8.67 mmol) in dichloromethane (10 mL). The resulting mixture was reacted at room temperature for 2 hours, washed with saturated aqueous sodium bicarbonate solution and then water, dried over anhydrous sodium sulphate and concentrated to obtain compound **24a** (1.2 g, 74%).
LC-MS (ESI): m/z = 292.1 [M+H]$^+$.

Step 2: (2-(3,3-difluorocyclobutane-1-carboxamido)pyridin-4-yl)boronic acid **(24b)**

Compound **24a** (2 g, 7.2 mmol), bis(pinacolato)diboron (2.2 g, 8.6 mmol) and potassium acetate (14 g, 14

mmol) were dissolved in 1,4-dioxane (50 mL). Under nitrogen protection, 1,1'-bis(diphenylphosphino)ferrocene palladium dichloride (II) (0.5 g, 0.72 mmol) was added. Under nitrogen protection, the reaction mixture was heated to 100°C and reacted for 16 hours. When the reaction was complete as shown by LC-MS, the reaction mixture was concentrated. Water was then added and a solid was precipitated out and filtered off to obtain an aqueous phase, which was concentrated to obtain crude compound **24b** (3 g).

LC-MS (ESI): m/z = 257.1 $[M+H]^+$.

Step 3: (S)-N-(4-(4-((2-amino-2,4-dimethylpent-4-en-1-yl)oxy)-3-cyanophenyl) pyridin-2-yl)-3,3-difluorocyclobutane-1-carboxamide **(compound 24)**

Compound **5b** (200 mg, 0.64 mmol), compound 24b (16 mg, 0.64 mmol) and potassium carbonate (88 mg, 0.64 mmol) were dissolved in water (2 mL) and 1,4-dioxane (10 mL). Under nitrogen protection, Xphos PdG2 (50 mg, 0.064 mmol) was then added. Under nitrogen protection, the mixture was heated to 80°C, reacted for 5 hours and concentrated. The residue was purified by column chromatography (DCM : MeOH = 10 : 1) to obtain a crude, which was purified by C-18 reverse phase column chromatography (acetonitrile : water = 30 : 70) to obtain **compound 24** (110 mg, 39%).

LC-MS (ESI): m/z = 441.5 $[M+H]^+$.

$^1H$ NMR (400 MHz, DMSO-$d_6$) $\delta$ 10.79 (s, 1H), 8.40 (d, 2H), 8.21-8.15 (m, 3H), 8.08 (d, 1H), 7.49 (d, 2H), 5.04 (s, 1H), 4.91 (s, 1H), 4.28-4.17 (m, 2H), 3.33 - 3.22 (m, 1H), 2.86 - 2.75 (m, 4H), 2.62 (d, 1H), 2.43 (d, 1H), 1.81 (s, 3H), 1.40 (s, 3H).

**Example 25**

(1R,2R)-N-(4-(4-(((S)-2-amino-2,4-dimethylpent-4-en-1-yl)oxy)-3-cyanophenyl)pyridin-2-yl)-2-fluorocyclopropane-1-carboxamide (compound **25**)

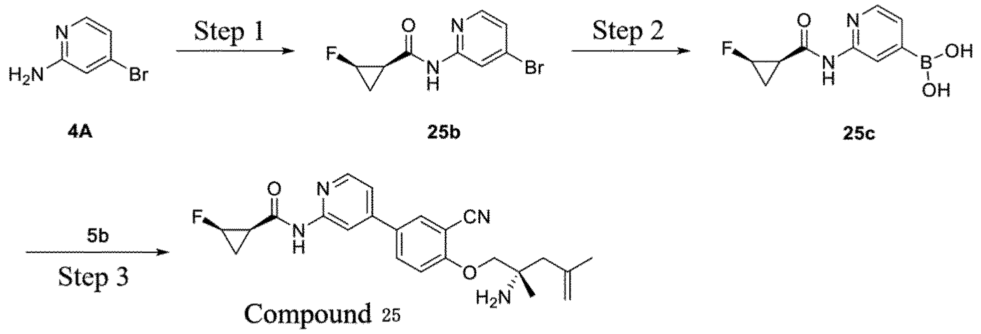


**4A** **25b** **25c**

Compound 25

Step 1: (1R,2R)-N-(4-bromopyridin-2-yl)-2-fluorocyclopropane-1-carboxamide **(25b)**

(1R,2R)-2-fluoro-clopropanecarboxylic acid (3.00 g, 28.82 mmol) was added to dichloromethane (40 mL) and after nitrogen replacement, N,N-dimethylformamide (1 mL) was added. Oxalyl chloride (4.02 g, 31.70 mmol) was diluted with dichloromethane (5 mL). At room temperature, the diluted solution of oxalyl chloride in dichloromethane was slowly added dropwise to the reaction solution, and after the addition was completed, the mixture was stirred at room temperature overnight. After the reaction was complete, the reaction solution was concentrated to dryness and anhydrous dichloromethane (10 mL) was added to prepare standby reaction solution **1.** Raw material **4A** was dissolved in dichloromethane (40 mL) and pyridine (3.42 g, 43.23 mmol) was added. Under nitrogen protection, the mixture was stirred for 15 min and then standby reaction solution 1 was slowly added dropwise while the temperature was controlled at 10°C-20°C. After the addition was completed, the resulting mixture was stirred for 1 hour. After the reaction was complete, water (50 mL) was added and the mixture was washed with saturated sodium bicarbonate (50 mL). The aqueous phase was extracted with dichloromethane (50 mL). The organic layers were combined, dried over anhydrous sodium sulphate and filtered. The filtrate was subjected to rotary evaporation and the residue was purified by silica gel column (petroleum ether : ethyl acetate (v/v) = 99 : 1-2 : 1) to obtain the title compound **25b** (5.20 g, 69%).

LC-MS (ESI): m/z = 261.0 $[M+H]^+$.

Step 2: (2-((1R,2R)-2-fluorocyclopropane-1-carboxamido)pyridin-4-yl)boronic acid (**25c**)

**25b** (1.00 g, 3.86 mmol), KOAc (1.14 g, 11.58 mmol) and bis(pinacolato)diboron (1.47 g, 5.79 mmol) were sequentially added to a mixed solution of dioxane (50 mL) and water (10 mL). After nitrogen replacement, [1,1'-bis(diphenylphosphino)ferrocene]palladium dichloride (II) (0.14 g, 0.19 mmol) was added. After additional nitrogen replacement, the mixture was reacted for 3 hours while the temperature was maintained at 80°C. The reaction process was monitored with LC-MS. After the reaction was complete, the reaction mixture was cooled to room temperature and filtered. The filtrate was concentrated to dryness. Water (200 mL) was added and the resulting mixture was ultra-sonicated for 15 min and then filtered. The filtrate was concentrated to dryness. Dioxane (200 mL) was added and the resulting mixture was ultra-sonicated for 15 min and then filtered. The filtrate was concentrated to dryness to obtain the title compound **25c** (0.75 g, 86.74%).

LC-MS (ESI): m/z = 225.1 $[M+H]^+$.

Step 3: (1R,2R)-N-(4-(4-(((S)-2-amino-2,4-dimethylpent-4-en-1-yl)oxy)-3-cyanophenyl)pyridin-2-yl)-2-fluorocyclopropane-1-carboxamide **(compound 25)**

**5b** (391 mg, 1.26 mmol), **25c** (0.42 g, 1.89 mmol) and potassium carbonate (0.35 g, 2.52 mmol) were sequentially added to a mixed solution of dioxane (50 mL) and water (10 mL). After nitrogen replacement, chloro(2-dicyclohexylphosphino-2',4',6'-triisopropyl-1,1 '-biphenyl)[2-(2'-amino-1,1'-biphenyl)]palladium (II) (0.01 g, 0.13 mmol) was added. After additional nitrogen replacement, the resulting mixture was reacted for 5 hours while the temperature was maintained at 80°C. After the reaction was complete, the reaction mixture was filtered and concentrated and the residue was separated and purified by reverse phase column chromatography (C18 spherical 20-35 nm 100A 120 g; water : acetonitrile (v/v) = 99 : 5-2 : 1) to obtain compound **25** (160 mg, 31.06%).

LC-MS (ESI): m/z = 409.3 $[M+H]^+$.

$^1$H NMR (400 MHz, DMSO-$d_6$) δ 10.92 (s, 1H), 8.40 - 8.35 (m, 2H), 8.13 - 8.00 (m, 2H), 7.46 - 7.37 (m, 2H), 5.05 - 5.00 (m, 1H), 4.86 - 4.71 (m, 3H), 3.89 (s, 2H), 2.24 (s, 3H), 1.82 - 1.60 (m, 5H), 1.15 (s, 4H).

**Example 26**

(S)-1-((2', 6-bis(difluoromethyl)-[2,4'-bipyridin]-5-yl)oxy)-3-cyclopropyl-2-methylpropan-2-amine **(compound 26)**

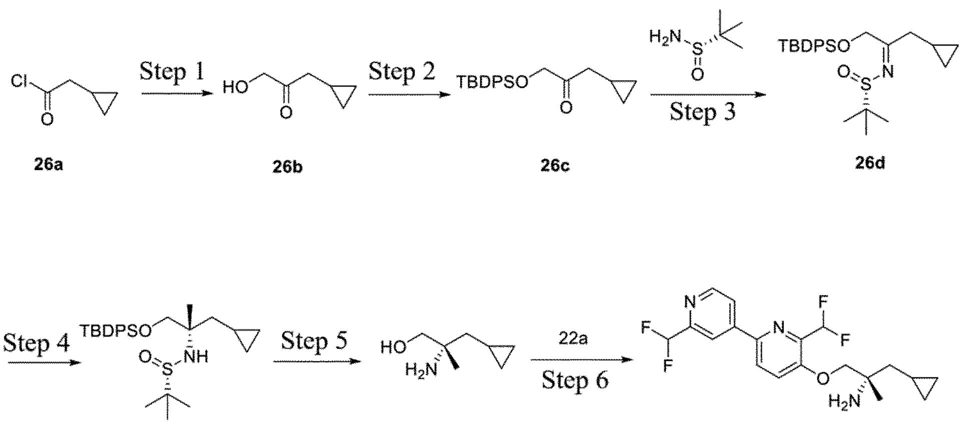


26a 26b 26c 26d

26e 26f Compound 26

Step 1: 1-cyclopropyl-3-hydroxypropan-2-one (**26b**)

**26a** (2.5 g, 21.1 mmol) and tris(trimethylsilyloxy)ethylene (13.6 g, 46.4 mmol) were sequentially added to a sealed tube. The mixture was purged with nitrogen for 2 minutes and reacted at 80°C for 12 hours. The mixture was then cooled to room temperature. Hydrochloric acid (2 M, 30 mL) and THF (30 mL) were added. The resulting mixture was reacted at 80°C for 2 hours and then extracted with EA twice. The organic phase was washed with saturated sodium bicarbonate and then water, dried over anhydrous sodium sulphate and concentrated to obtain the title compound **26b** as a light yellow oil (1.0 g, 41%), which was directly used in the next reaction.

LC-MS (ESI): m/z = 115.1 $[M+H]^+$.

Step 2: 1-((tert-butyldiphenylsilyl)oxy)-3-cyclopropylpropan-2-one (**26c**)

**26b** (1.5 g, 13.1 mmol) was dissolved in DCM (30 mL) in a single-necked flask, and TEA (3.99 g, 39.4 mmol) and DMAP (0.16 g, 1.31 mmol) were added. Subsequently, TBDPSCl (4.33 g, 15.8 mmol) was added. The resulting mixture was stirred at room temperature overnight. After the reaction was completed, the reaction mixture was washed with water, dried over anhydrous sodium sulphate and concentrated. The residue was purified by column chromatography (PE : EA = 30 : 1) to obtain compound **26c** as a colourless oil (1.9 g, 41%).
LC-MS (ESI): m/z = 353.2 $[M+H]^+$.

Step 3: (S)-N-(1-((tert-butyldiphenylsilyl)oxy)-3-cyclopropylpropan-2-ylidene) -2-methylpropane-2-sulphinamide (**26d**)

**26c** (1.9 g, 5.39 mmol) was dissolved in THF (30 mL) in a single-necked flask and then S-tert-butylsulphinamide (0.80 g, 6.47 mmol) was added. Subsequently, $Ti(OiPr)_4$ (4.6 g, 16.17 mmol) was added. The mixture was subjected to nitrogen replacement 3 times and reacted at 80°C for 20 hours. After the reaction was completed, saturated brine was added to quench the reaction. The mixture was then extracted with ethyl acetate twice, dried over anhydrous sodium sulphate and concentrated to obtain the title compound **26d** as a yellow oil (1.0 g, 40%), which was directly used in the next reaction.
LC-MS (ESI): m/z = 456.2 $[M+H]^+$.

Step 4: N-((S)-1-((tert-butyldiphenylsilyl)oxy)-3-cyclopropyl-2-methylpropan -2-yl)-2-methylpropane-2-sulphinamide (**26e**)

Methylmagnesium bromide (1.31 g, 11 mmol) was dissolved in DCM (20 mL) and the mixture was stirred at 0°C. Then a solution of **26d** (1.0 g, 2.19 mmol) in DCM was added dropwise to the reaction flask. The reaction mixture was reacted at this temperature for 3 hours. After the reaction was completed, a large quantity of water was added to quench the reaction. Then the mixture was exacted with DCM twice. The organic phases were combined and dried over anhydrous sodium sulphate and the solvent was removed. Subsequently, the residue was separated by column chromatography (petroleum ether : ethyl acetate (v/v) = 10 : 1) to obtain compound **26e** as a colourless oil (0.6 g, 58%).
LC-MS (ESI): m/z = 472.2 $[M+H]^+$.

Step 5: (S)-2-amino-3-cyclopropyl-2-methylpropan-1-ol (**26f**)

**26e** (0.6 g, 1.27 mmol) was dissolved in dioxane (5 mL) and then concentrated hydrochloric acid (5 mL) was added. Subsequently, the mixture was reacted at 100°C for 5 hours. After the reaction was completed, the reaction solution was adjusted to a basic pH with ammonia in methanol. Then the solvent was completely removed by rotary evaporation. Subsequently, the residue was separated by column chromatography (dichloromethane : methanol (v/v) = 10 : 1) to obtain compound **26f** as a colourless oil (80 mg, 48%).
LC-MS (ESI): m/z = 130.1 $[M+H]^+$.

Step 6: (S)-1-((2',6-bis(difluoromethyl)-[2,4'-bipyridin]-5-yl)oxy)-3-cyclopropyl-2-methylpropan-2-amine (compound **26**)

**26f** (80.0 mg, 0.62 mmol) was added to a sealed tube and then THF (10 mL) was added. Subsequently, **22a** (230.0 mg, 0.83 mmol) and potassium tert-butoxide (208.3 mg, 1.86 mmol) were sequentially added and the mixture was purged with nitrogen for 2 minutes and reacted at 80°C for 4 hours. After the reaction was completed, THF was removed by rotary evaporation. Water (50 mL) was added and the mixture was extracted with ethyl acetate (50 mL) twice. The organic phases were combined, dried over anhydrous sodium sulphate and concentrated. The residue was then separated by column chromatography (dichloromethane : methanol (v/v) = 20 : 1) to obtain the title compound **26** (28 mg, 11%).
$^1H$ NMR (400 MHz, $CDCl_3$) $\delta$ 8.73 (d, 1H), 8.20 (s, 1H), 8.01 (d, 1H), 7.94 (d, 1H), 7.44 (d, 1H), 7.02-6.57 (m, 2H), 4.03 (q, 2H), 1.64-1.36 (m, 2H), 1.36 (s, 3H), 0.75-0.70 (m, 1H), 0.53-0.48 (m, 2H), 0.15-0.08 (m, 2H).
LC-MS (ESI): m/z = 384.2 $[M+H]^+$.

**Example 27**

Methyl (5-((2-amino-2,4-dimethylpent-3-en-1-yl)oxy)-4-(trifluoromethyl)-[2,4'-bipyridin]-2'-yl)carbamate (compound **27**)

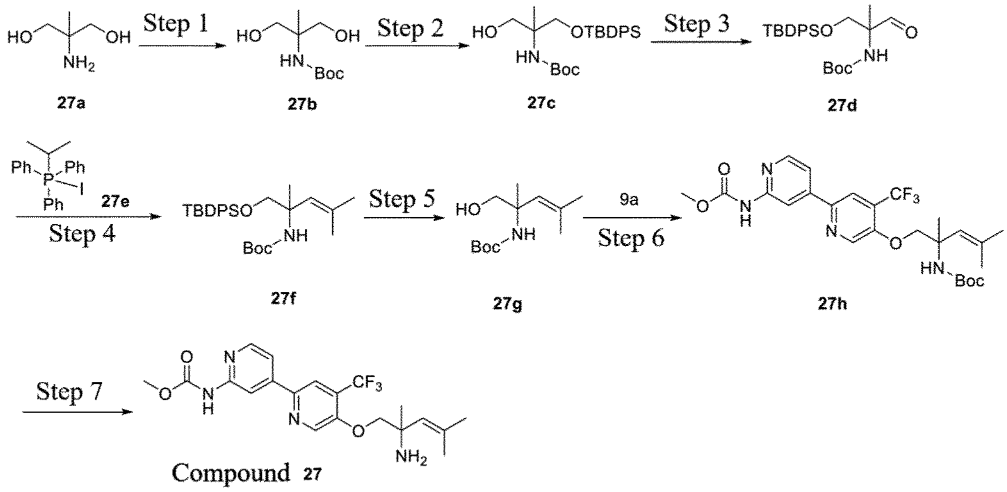


27a 27b 27c 27d 27f 27g 27h

Compound 27

Step 1: Tert-butyl (1,3-dihydroxy-2-methylpropan-2-yl)carbamate (**27b**)

**27a** (10 g, 95.11 mmol) was dissolved in 125 mL of (MeOH : THF = 100 : 25) and di-tert-butyl dicarbonate (31.14 g, 142.67 mmol) was added while the mixture was cooled to 0°C in an ice bath. Sodium bicarbonate (15.98 g, 190.22 mmol) was added with stirring. After stirring, the mixture was transferred to room temperature and reacted for 12 hours. The reaction process was monitored by TLC. After the reaction was complete, the reaction mixture was extracted with ethyl acetate and washed with water. The organic phase was dried over anhydrous sodium sulphate and concentrated to obtain a crude, which was separated by silica gel column chromatography (petroleum ether : ethyl acetate = 2 : 1) to obtain 18 g of the title compound **27b** (yield: 92%).

LC-MS (ESI): m/z = 150.2 $[M+H-tBu]^+$.

Step 2: Tert-butyl N-(1-[(tert-butyldiphenylsilyl)oxy]-3-hydroxy-2-methylpropan-2-yl)carbamate (**27c**)

Compound **27b** (18 g, 87.69 mmol) and imidazole (5.98 g, 87.69 mmol) were dissolved in DMF (200 mL). The mixture was cooled to 0°C. TBDPSCl (20.46 mL, 78.92 mmol) was slowly added. The resulting mixture was stirred at room temperature overnight. Water was added and the resulting mixture was extracted with EA. Liquid separation was performed. The organic phase was washed with water and liquid separation was performed, followed by extraction with saturated brine and additional liquid separation. The organic phase was dried, concentrated and subjected to rotary evaporation to obtain a crude, which was separated and purified by column chromatography (petroleum ether : ethyl acetate = 10 : 1) to obtain compound **27c** (20 g, 51%).

LC-MS (ESI): m/z = 444.3 $[M+H]^+$.

Step 3: Tert-butyl (1-((tert-butyldiphenylsilyl)oxy)-2-methyl-3-oxopropan-2-yl)carbamate (**27d**)

Compound **27c** (20 g, 45 mmol) was dissolved in acetone (400 mL) and IBX (19 g, 67.62 mmol) was added. The mixture was stirred at 60°C overnight. The mixture was cooled to room temperature, filtered by suction and washed with EA. The filtrate was concentrated and subjected to rotary evaporation to obtain compound **27d** (18 g, 90%).

LC-MS (ESI): m/z = 344.1 $[M+H-Boc]^+$.

Step 4: Tert-butyl (1-((tert-butyldiphenylsilyl)oxy)-2,4-dimethylpent-3-en-2-yl)carbamate (**27f**)

Compound **27e** (14.1 g, 32.61 mmol) was dissolved in THF (100 mL). Under nitrogen protection, the mixture was cooled to 0°C and n-butyllithium (1.6 M, 20.37 mL) was added. The resulting mixture was stirred at 0°C for 5 minutes and cooled to -78°C and a solution of compound **27d** (6 g, 13.59 mmol) in 30 mL of THF was added. At -78°C, the mixture was reacted for 30 minutes, transferred to an ice-water bath, and reacted for another 1 hour. The reaction was quenched by saturated $NH_4Cl$. The mixture was extracted with EA and liquid separation was performed. The organic phase was dried and concentrated and the residue was separated and purified by column chromatography (petroleum ether : ethyl acetate = 10 : 1) to obtain the crude product of compound **27f** (0.5 g).

LC-MS (ESI): m/z = 468.2 $[M+H]^+$.

Step 5: Tert-butyl (1-hydroxy-2,4-dimethylpent-3-en-2-yl)carbamate (**27g**)

Compound **27f** (0.5 g, 1.07 mmol) was dissolved in THF (5 mL) and TBAF (1 M, 2 mL) was added. The mixture was stirred at room temperature overnight, concentrated and subjected to rotary evaporation. The residue was separated and purified by column chromatography (dichloromethane : methanol = 10 : 1) to obtain compound **27 g** (170 mg).
LC-MS (ESI): m/z = 174.2 $[M+H-tBu]^+$.
$^1H$ NMR (400 MHz, $CDCl_3$) δ 5.25 (m, 1H), 4.87 (s, 1H), 3.78 (d, 1H), 3.52(d, 1H), 1.78 (d, 3H), 1.73 (d, 3H), 1.43 (s, 9H), 1.38 (s, 3H).

Step 6: Tert-butyl (1-((2'-((methoxycarbonyl)amino)-4-(trifluoromethyl)-[2,4'-bipyridin]-5-yl)oxy)-2,4-dimethylpent-3-en-2-yl)carbamate (**27h**)

Compound **9a** (84 mg, 0.27 mmol) and a solution of 1M potassium tert-butoxide in THF (0.4 mL) were stirred at room temperature for 5 min and compound **27g** (61 mg, 0.26 mmol) was added. After nitrogen replacement, the mixture was warmed to 80°C and stirred overnight. After the reaction was completed, the reaction mixture was concentrated and the crude was separated and purified by column chromatography (dichloromethane : methanol = 10 : 1) to obtain the title compound **27h** (23 mg, 17%).
LC-MS (ESI): m/z = 525.2 $[M+H]^+$.

Step 7: Methyl (5-((2-amino-2,4-dimethylpent-3-en-1-yl)oxy)-4-(trifluoromethyl)-[2,4'-bipyridin]-2'-yl)carbamate (compound **27**)

Compound **27h** (23 mg, 0.04 mmol) was dissolved in dichloromethane (2 mL) and TFA (0.3 mL) was added. The mixture was stirred at room temperature for 1 hour. The reaction mixture was concentrated and subjected to rotary evaporation. The residue was purified by HPLC to obtain the title compound **27** (5 mg, 27%).
LC-MS (ESI): m/z = 425.2 $[M+H]^+$.
$^1H$ NMR (400 MHz, $CD_3OD$) δ8.79 (s, 1H), 8.54 (s, 1H), 8.35 (d, 1H), 8.17 (s, 1H), 7.68 (dd, 1H), 5.36 (s, 1H), 4.53 (d, 1H), 4.44 (d, 1H), 3.80 (s, 3H), 1.89 (d, 3H), 1.87 (d, 3H), 1.72 (s, 3H).
$^{19}F$ NMR (376 MHz, $CD_3OD$) δ -62.71.

**Example 28**

N-(4-(4-(((2S)-2-amino-4,5-dihydroxy-2,4-dimethylpentyl)oxy)-3-cyanophenyl)pyridin-2-yl)acetamide (**compound 28**)

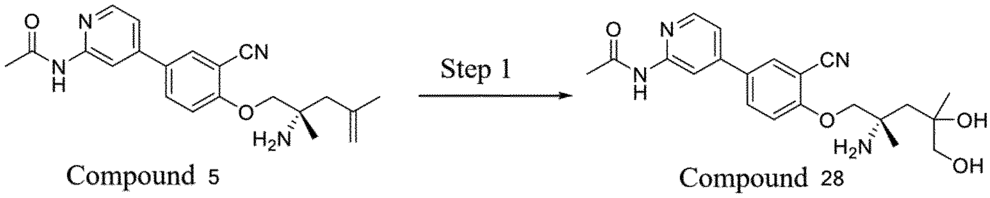


Compound 5 Compound 28

Step 1: N-(4-(4-(((2S)-2-amino-4,5-dihydroxy-2,4-dimethylpentyl)oxy)-3-cyanophenyl)pyridin-2-yl) acetamide (**28**)

Compound **5** (100 mg, 0.27 mmol), potassium osmate dihydrate (10 mg, 0.027 mmol) and N-methylmorpholine oxide (95 mg, 0.81 mmol) were dissolved in water (1 mL) and acetone (3 mL). The mixture was reacted at room temperature for 3 hours and then purified by C-18 reverse phase column chromatography (acetonitrile : water = 40 : 60) to obtain compound **28** (50 mg, 46%).
LC-MS (ESI): m/z = 399.5$[M+H]^+$.
$^1H$ NMR (400 MHz, DMSO-$d_6$) δ 10.55 (s, 1H), 8.37 - 8.32 (m, 2H), 8.12 (d, 1H), 7.98 (d, 1H), 7.45 - 7.36 (m, 2H), 4.87 - 4.83 (m, 1H), 4.71 (s, 1H), 3.88 (s, 2H), 2.23 (s, 2H), 2.12 (s, 3H), 1.79 (s, 3H), 1.54 (s, 2H), 1.14 (s, 3H).

**Examples 29 and 30**

(1R)-N-(4-(4-{[(2S)-2-amino-2,4-dimethylpent-4-en-1-yl]oxy}-3-cyanophenyl)pyridin-2-yl)-2,2-difluorocyclopropane-1-carboxamide and (1S)-N-(4-(4-{[(2S)-2-amino-2,4-dimethylpent-4-en-1-yl]oxy}-3-cyanophenyl)pyridin-2-yl)-2,2-difluorocyclopropane-1-carboxamide **(compound 29 and compound 30)**

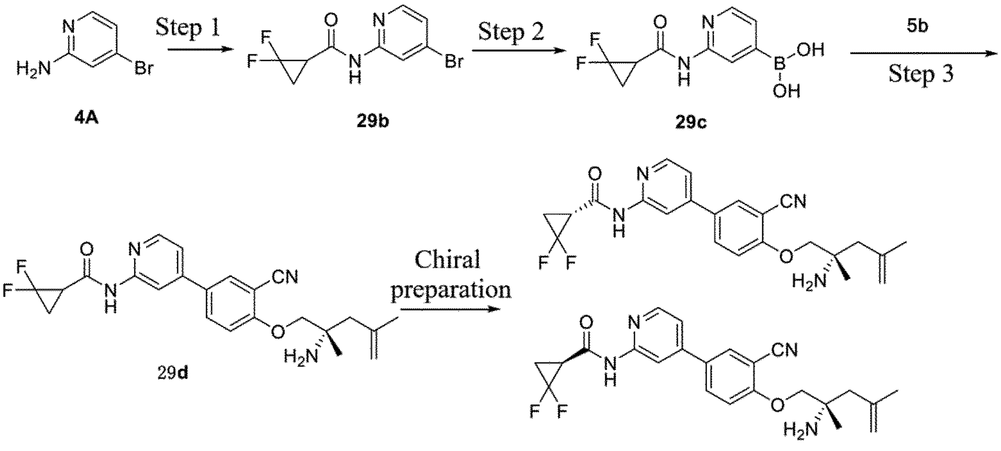


4A 29b 29c

29**d**

Compound 29 Compound 30

Step 1: N-(4-bromopyridin-2-yl)-2,2-difluorocyclopropane-1-carboxamide **(29b)**

**4A** (2.00 g, 11.56 mmol), 2,2-difluoro-clopropanecarboxylic acid (1.41 g, 11.56 mmol), N-methylimidazole (1.90 g, 23.12 mmol) and N,N,N',N'-tetramethylchloroformamidinium hexafluorophosphate (3.89 g, 13.87 mmol) were sequentially added to dichloromethane (50 mL), and after nitrogen replacement, the mixture was stirred overnight. After the reaction was complete, water (50 mL) was added and the mixture was washed with saturated sodium bicarbonate (50 mL). The aqueous phase was extracted with dichloromethane (50 mL). The organic layers were combined, dried over anhydrous sodium sulphate and filtered. The filtrate was subjected to rotary evaporation and the residue was purified by silica gel column (petroleum ether : ethyl acetate (v/v) = 99 : 1-2 : 1) to obtain intermediate **29b** (5.20 g, 69%).

LC-MS (ESI): m/z = 277.0 $[M+H]^+$.

Step 2: (2-(2,2-difluorocyclopropaneamido)pyridin-4-yl)boronic acid **(29c)**

**29b** (800 mg, 2.89 mmol), potassium acetate (850 mg, 8.67 mmol) and bis(pinacolato)diboron (1.10 g, 4.33 mmol) were sequentially added to a mixed solution of dioxane (50 mL) and water (10 mL). After nitrogen replacement, [1,1'-bis(diphenylphosphino)ferrocene]palladium dichloride (II) (0.11 g, 0.14 mmol) was added. After additional nitrogen replacement, the mixture was reacted for 3 hours while the temperature was maintained at 80°C. The reaction process was monitored with LC-MS. After the reaction was complete, the mixture was cooled to room temperature and filtered. The filtrate was concentrated to dryness.

Acetonitrile (200 mL) was added and the resulting mixture was ultra-sonicated for 15 min and then filtered. The filtrate was concentrated to dryness to obtain intermediate **29c** (0.5 g, 71%).

LC-MS (ESI): m/z = 243.1 $[M+H]^+$.

Step 3: N-(4-(4-(((S)-2-amino-2,4-dimethylpent-4-en-1-yl)oxy)-3-cyanophenyl)pyridin-2-yl)-2,2-difluorocyclopropane-1-carboxamide (**29d**)

**5b** (500 mg, 1.61 mmol), **29c** (0.97 g, 4.03 mmol) and potassium carbonate (0.67 g, 4.83 mmol) were sequentially added to a mixed solution of dioxane (100 mL) and water (20 mL). After nitrogen replacement, chloro(2-dicyclohexylphosphino-2',4',6'-triisopropyl-1,1 '-biphenyl)[2-(2'-amino-1,1'-biphenyl)]palladium (II) (0.01 g, 0.13 mmol) was added. After additional nitrogen replacement, the resulting mixture was reacted for 5 hours while the temperature was maintained at 80°C. After the reaction was complete, the reaction mixture was filtered and concentrated and the residue was

separated and purified by reverse phase column chromatography (C18 spherical 20-35 nm 100A 120 g; water : acetonitrile (v/v) = 99 : 5-2 : 1) to obtain compound **29d** (420 mg, 61.17%).

LC-MS (ESI): m/z = 427.2 $[M+H]^+$.

$^1$H NMR (400 MHz, DMSO-$d_6$) δ 11.10 (s, 1H), 8.46 - 8.25 (m, 2H), 8.14 (m, 1H), 8.01 (m, 1H), 7.50 (m, 1H), 7.37 (m, 1H), 4.86 (m, 1H), 4.70 (m, 1H), 3.89 (s, 2H), 3.14 - 2.92 (m, 1H), 2.23 (s, 2H), 2.15 - 1.93 (m, 2H), 1.70 (m, 5H), 1.15 (s, 3H).

Chiral preparation

**29d** (420 mg) was subjected to chiral resolution to obtain two isomers: $P_1$ (151 mg, retention time: 1.264 minutes, set to be compound 29) and $P_2$ (150 mg, retention time: 2.080 minutes, set to be compound 30).

Preparation method:

instrument: MG II preparative SFC (SFC-14); column: ChiralPak IC, 250 × 30 mm I.D., 10 μm; mobile phase: A, $CO_2$ B, ethanol (0.1% $NH_3$•$H_2O$); gradient: 35% B gradient elution; flow rate: 80 mL/min; column temperature: 38°C; wavelength: 220 nm; cycle time: 5.5 min; sample preparation: sample concentration: 11.25 mg/mL, dichloromethane/methanol solution; sample injection: 1 ml/injection. After separation, the fractions were dried on a rotary evaporator at the bath temperature of 40°C to obtain the desired isomers, respectively.

**Examples 31 and 32**

Methyl (S)-(5-(2-amino-3-cyclopropyl-2-methylpropoxy)-6-(difluoromethyl)-[2,4'-bipyridin]-2'-yl)carbamate and methyl (R)-(5-(2-amino-3-cyclopropyl-2-methylpropoxy)-6-(difluoromethyl)-[2,4'-bipyridin]-2'-yl)carbamate (compound **31** and compound **32**)

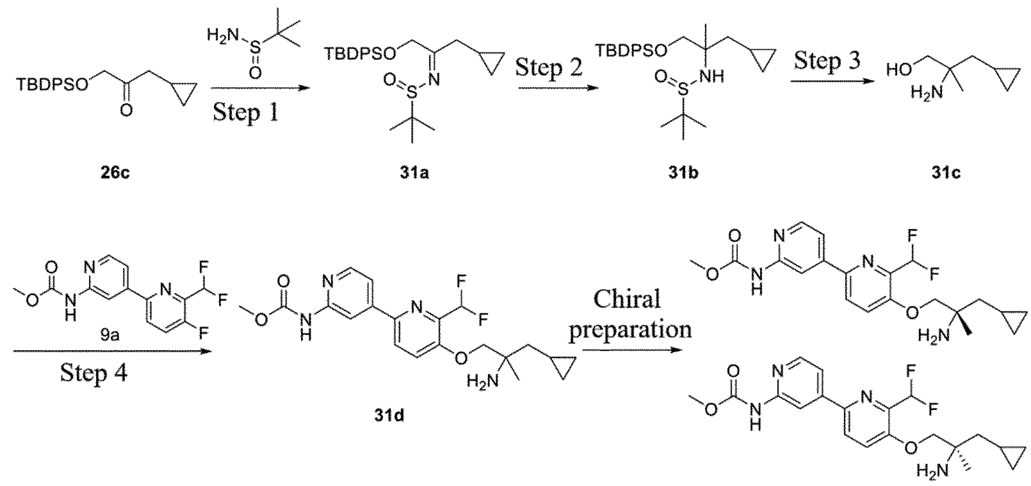


26c 31a 31b 31c

31d

Compound 31 Compound 32

Step 1: N-(1-((tert-butyldiphenylsilyl)oxy)-3-cyclopropylpropan-2-ylidene)-2-methylpropane-2-sulphinamide (**31a**)

**26c** (1.9 g, 5.39 mmol) was dissolved in THF (30 mL) in a single-necked flask and then tert-butylsulphinamide (0.80 g, 6.47 mmol) was added. Subsequently, Ti(Oi-Pr)$_4$ (4.6 g, 16.17 mmol) was added. The mixture was subjected to nitrogen replacement 3 times and reacted at 80°C for 20 hours. After the reaction was completed, saturated brine was added to quench the reaction. The mixture was then extracted with ethyl acetate twice, dried over anhydrous sodium sulphate and concentrated to obtain the title compound **31a** as a yellow oil (1.0 g, 40%), which was directly used in the next reaction.

LC-MS (ESI): m/z = 456.2 $[M+H]^+$.

Step 2: N-(-1-((tert-butyldiphenylsilyl)oxy)-3-cyclopropyl-2-methylpropan-2-yl)-2-methylpropane-2-sulphinamide (**31b**)

Methylmagnesium bromide (1.31 g, 11 mmol) was dissolved in DCM (20 mL) and the mixture was stirred at 0°C. Then a solution of **31a** (1.0 g, 2.19 mmol) in DCM was added dropwise to the reaction flask. The reaction mixture was reacted at this temperature for 3 hours. After the reaction was completed, water was added to quench the reaction. Then the mixture was exacted with DCM twice. The organic phases were combined and dried over anhydrous sodium sulphate and the solvent was removed. Subsequently, the residue was separated by silica gel column chromatography (petroleum ether : ethyl acetate (v/v) = 10 : 1) to obtain compound **31b** as a colourless oil (0.6 g, 58%).
LC-MS (ESI): m/z = 472.2 $[M+H]^+$.

Step 3: 2-amino-3-cyclopropyl-2-methylpropan-1-ol **(31c)**

**31b** (0.6 g, 1.27 mmol) was dissolved in dioxane (5 mL) and then concentrated hydrochloric acid (5 mL) was added. The mixture was reacted at 100°C for 5 hours. After the reaction was completed, the reaction solution was adjusted to a basic pH with ammonia in methanol. Then the solvent was completely removed by rotary evaporation. Subsequently, the residue was separated by silica gel column chromatography (DCM: methanol (v/v) = 10 : 1) to obtain compound **31c** as a colourless oil (80 mg, 48.7%).
LC-MS (ESI): m/z = 130.1 $[M+H]^+$.

Step 4: Methyl (5-(2-amino-3-cyclopropyl-2-methylpropoxy)-6-(difluoromethyl) -[2,4'-bipyridin]-2'-yl)carbamate (**31d**)

**31c** (80.0 mg, 0.62 mmol) was added to a sealed tube and then THF (10 mL) was added. **9a** (221.0 mg, 0.74 mmol) and potassium tert-butoxide (208.3 mg, 1.86 mmol) were sequentially added and the mixture was purged with nitrogen for 2 minutes and reacted at 80°C for 4 hours. After the reaction was completed, THF was removed by rotary evaporation. Water (50 mL) was added and the mixture was extracted with ethyl acetate (50 mL) twice. The organic phases were combined and dried over anhydrous sodium sulphate, and the solvent was removed. The residue was then separated by column chromatography (dichloromethane : methanol (v/v) = 20 : 1) to obtain the title compound **31d** (140 mg, 55.7%).
$^1H$ NMR (400 MHz, $CDCl_3$) $\delta$ 8.53 (s, 1H), 8.36 (m, 1H), 7.95 (m, 1H), 7.71 (d, 1H), 7.39 (d, 1H), 6.84 (t, 1H), 3.95 (q, 2H), 3.85 (s, 3H), 1.60-1.47 (m, 2H), 1.31 (s, 3H), 0.77-0.70 (m, 1H), 0.53-0.48 (m, 2H), 0.15-0.08 (m, 2H).
LC-MS (ESI): m/z = 407.2 $[M+H]^+$.

Chiral preparation

**31d** (140 mg) was subjected to chiral resolution to obtain two isomers: $\mathbf{P_1}$ (60 mg, retention time: 1.847 minutes, set to be compound **31**) and $\mathbf{P_2}$ (60 mg, retention time: 2.203 minutes, set to be compound **32**).

Preparation method:

instrument: Waters 150 MGM; column: Chiralpak Column; mobile phase: A, $CO_2$, B, IPA (0.1% $NH_3$•$H_2O$); gradient: 40% B gradient elution; flow rate: 80 mL/min; column temperature: 35°C; wavelength: 220 nm; cycle time: 7.6 min; sample preparation: sample concentration: 6.0 mg/mL, acetonitrile solution; sample injection: 2.5 ml/injection. After separation, the fractions were dried on a rotary evaporator at the bath temperature of 30°C to obtain $\mathbf{P_1}$ and $\mathbf{P_2}$. Then the products were dried in a lyophilizer at -80°C for removing the solvent to obtain $\mathbf{P_1}$ and $\mathbf{P_2}$.

**Biological test**

**1. *In vitro* AAK1 enzyme activity assay**

Compound stock solution (concentration: 10 mM, dissolved in DMSO) was diluted with DMSO to 0.2 mM and then diluted with DMSO in 5-fold gradient to obtain compound solutions with 10 concentrations. Subsequently, the compound solutions with different concentrations were diluted 50-fold in 1×kinase reaction buffer (containing 40 mM Tris, 20 mM $MgCl_2$, 0.1% BSA and 0.5 mM DTT) for later use. AAK1 (Signalchem, Cat# A01-11G-10) was diluted with 1×kinase reaction buffer to 2-fold the final concentration (final concentrations: 30 nM and 28 nM). AAK1 was added to a 384-well white plate at 2 μL/well, and the compounds were then added at 1 μL/well. The plate was sealed with a plate-sealing film, centrifuged at 1000 rpm for 30 seconds and then placed at room temperature for 10 minutes. A mixed solution of ATP (Promega, Cat# V914B) and substrate Micro2 (GenScript, Cat# PE0890) was formulated at 4-fold the final concentration (for AAK1, the corresponding final concentrations of ATP: 15 μM and 5 μM, and the corresponding

final concentration of Micro2: 0.1 mg/mL). To the reaction plate was added the mixed solution of ATP and the substrate at 1 μL/well. The plate was sealed with a plate-sealing film and centrifuged at 1000 rpm for 30 seconds. The reaction was carried out at room temperature for 60 minutes (AAK1). ADP-Glo (Promega, Cat# V9102) was transferred to the 384-well plate at 4 μL/well and centrifugation was carried out at 1000 rpm for 1 minute. The mixture was incubated at 25°C for 40 minutes. A detection solution was transferred to the 384-well plate at 8 μL/well and centrifugation was carried out at 1000 rpm for 1 minute. The resulting mixture was incubated at 25°C for 40 minutes. The RLU (Relative luminescence unit) signal values were read using a Biotek multilable microplate reader and the percent inhibition was calculated according to the following formula: $[1-(LUM_{compound}-LUM_{positive\ control}) / (LUM_{negative\ control}-LUM_{positive\ control})] \times 100$. $IC_{50}$ values were calculated using Graphpad 7.0 software using a four-parameter non-linear fit equation. The specific results are shown in Table 1.

Table 1 Inhibitory activity against AAK1

| Compound No. | $IC_{50}$/nM |
|---|---|
| Compound 1 | 14.72 |
| Compound 2 | 9.29 |
| Compound 3 | 10.92 |
| Compound 5 | 6.37 |
| Compound 6 | 10.97 |
| Compound 7 | 13.81 |
| Compound 10 | 11.47 |
| Compound 11 | 5.74 |
| Compound 13 | 10.57 |
| Compound 14 | 12.62 |
| Compound 15 | 19.82 |
| Compound 16 | 22.26 |
| Compound 17 | 26.77 |
| Compound 18 | 9.55 |
| Compound 19 | 37.73 |
| Compound 20 | 16.06 |
| Compound 21 | 13.74 |
| Compound 22 | 32.08 |
| Compound 23 | 38.62 |
| Compound 24 | 22.46 |
| Compound 25 | 11.94 |
| Compound 26 | 11.01 |
| Compound 27 | 43.09 |
| Compound 28 | 5.96 |
| Compound 29 | 10.98 |
| Compound 30 | 9.08 |
| Compound 31 | 10.92 |

Conclusion: the compounds of the present invention show relatively high inhibitory activity against the AAK1 receptor.

## 2. Pharmacokinetic test in beagle dogs

**Experimental animals:** male beagle dogs, about 8-11 kg, 6 beagle dogs/compound, purchased from Beijing Marshall Biotechnology Co., Ltd.

**Experimental method:** on the day of the experiment, 12 beagle dogs were randomly grouped according to their body weights. the animals were fasted with water available for 12 to 14 h one day before the administration, and were fed 4 h after the administration; and the administration was performed according to Table 2.

Table 2. Administration information

| Group | Number | Administration information | | | | | |
|---|---|---|---|---|---|---|---|
| | Male | Test compounds | Administration dosage (mg/kg) | Administration concentration (mg/mL) | Administration volume (mL/kg) | Collected sample | Mode of administration |
| G1 | 3 | LX-9211 | 1 | 1 | 1 | Plasma | Intravenously |
| G2 | 3 | | 3 | 0.6 | 5 | Plasma | Intragastrically |
| G3 | 3 | Compound 19 | 1 | 1 | 1 | Plasma | Intravenously |
| G4 | 3 | | 3 | 0.6 | 5 | Plasma | Intragastrically |

Notes: Vehicle for intravenous administration: 5% DMA+5% Solutol+90% Saline; vehicle for intragastric administration: 0.5% MC (DMA: dimethylacetamide; Solutol: polyethylene glycol-15-hydroxystearate; Saline: physiological saline; MC: methylcellulose)

Before and after the administration, 1 ml of blood was taken from the jugular veins or limb veins, and placed in an EDTAK2 centrifuge tube. Centrifugation was performed at 5000 rpm at 4°C for 10 min, and plasma was collected. Blood sampling time points for both the LX9211 intravenous administration group and intragastric administration group include: 0 min, 5 min, 15 min, 30 min, 1 h, 2 h, 4 h, 6 h, 8 h, 10 h, 12 h, and 24 h, and blood sampling time points for both the compound 19 intravenous administration group and intragastric administration group include: 0 min, 5 min, 15 min, 30 min, 1 h, 2 h, 4 h, 6 h, 8 h, 10 h, 12 h, 24 h, and 48 h. Before analysis and detection, all samples were stored at -80°C. The samples were analysed quantitatively by LC-MS/MS. The experimental results are shown in Table 3.

Table 3. Pharmacokinetic parameters of test compounds in plasma of beagle dogs

| Test compounds | Mode of administration | CL (mL/min/kg) | $Vd_{ss}$ (L/kg) | $AUC_{0-t}$ (hr×ng/mL) | F (%) |
|---|---|---|---|---|---|
| LX-9211 | i.v. (1 mg/kg) | 22.2±7.7 | 9.08±1.4 | 751±204 | - |
| | i.g. (3 mg/kg) | - | - | 500±183 | 22.2±8.1 |
| Compound 19 | i.v. (1 mg/kg) | 39.8±10 | 23.6±3.8 | 398±94 | - |
| | i.g. (3 mg/kg) | | | 1353±83 | 113±6.9 |

-: not applicable.
Notes: LX-9211 has a structure of

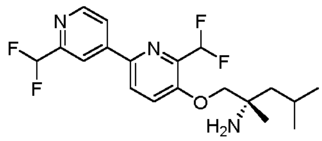

.

**Conclusion:** Conclusion: The compounds of the present invention possess good pharmacokinetic characteristics.

### 3. Test for hERG potassium ion channel

**Experimental platform:** electrophysiological manual patch-clamp system

**Cell line:** Chinese hamster ovary (CHO) cell lines stably expressing hERG potassium ion channel

**Experimental method:** In CHO (Chinese Hamster Ovary) cells stably expressing hERG potassium channel, whole cell patch-clamp technique was used to record hERG potassium channel current at room temperature. The glass microelectrode was made of a glass electrode blank (BF150-86-10, Sutter) by a puller. The tip resistance after filling the liquid in the electrode was about 2-5 MΩ. The glass microelectrode can be connected to the patch-clamp amplifier by inserting the glass microelectrode into an amplifier probe. The clamping voltage and data recording were controlled and recorded by the pClamp 10 software through a computer. The sampling frequency was 10 kHz, and the filtering frequency was 2 kHz. After the whole cell records were obtained, the cells were clamped at -80 mV, and the step voltage that induced the hERG potassium current ($I_{hERG}$) was depolarized from -80 mV to +20 mV for 2 s, then repolarized to -50 mV, and returned to -80 mV after 1 s. This voltage stimulation was given every 10 s, and the administration process was started after the hERG potassium current was confirmed to be stable (at least 1 minute). The compound was administered for at least 1 minute at each test concentration, and at least 2 cells ($n \geq 2$) were tested at each concentration.

**Data processing:** Data analysis processing was carried out by using pClamp 10, GraphPad Prism 5 and Excel software. The inhibition degree of hERG potassium current (peak value of hERG tail current induced at -50 mV) at different compound concentrations was calculated by the following formula:

$$\text{Inhibition\%} = [1 - (I / Io)] \times 100\%$$

wherein, Inhibition % represents the percentage of inhibition of hERG potassium current by the compound, and I and *I*o represent the amplitude of hERG potassium current after and before dosing, respectively.

Compound $IC_{50}$ was calculated using GraphPad Prism 5 software by fitting according to the following equation:

$$Y = Bottom + (Top-Bottom)/(1+10^{((LogIC50-X)\times HillSlope)})$$

Among the equation, X represents the Log value of the tested concentration of the test sample, Y represents the inhibition percentage at the corresponding concentration, and Bottom and Top represent the minimum and maximum inhibition percentage, respectively.

**Experimental results:** $IC_{50}$ values of the inhibitory effect of the test compounds on hERG potassium channel current are shown in Table 4.

Table 4 Inhibition of test compounds on hERG potassium channel current

| Test compounds | Inhibition rate at the highest concentration tested | $IC_{50}$ (μM) |
|---|---|---|
| LX-9211 | 102.2±4.08%@40 μM | 1.82 |
| Compound 3 | 86.6±1.77%@40 μM | 8.75 |
| Compound 19 | 68.9±1.80%@40 μM | 24.1 |

**4. Spinal nerve ligation (SNL)-induced mouse model of neuropathic pain**

Male C57BL/6J mice (8 weeks old) purchased from Jinan Pengyue Experimental Animal Breeding Co., Ltd. were adaptively raised for one week and then the models were established. The specific establishment method comprises the following steps:

1) disinfecting the surgical instrument and ligature;
2) anesthetizing the mice with isoflurane and then placing the mice on the operating table in the prone position;
3) shaving the fur near the hip bones of the mice and preparing the skin, and making an incision of about 2 cm along the spine;
4) isolating the fascia along the spine, performing blunt dissection of the muscles, and exposing the transverse process of L5;
5) carefully cutting the transverse process of L5 with forceps and exposing the L5 spinal nerve;
6) carefully isolating the L5 nerve with a glass dissecting needle, and ligating the L5 nerve with a 5-0 ligature; and
7) suturing the muscles and skin and disinfecting same with iodophor.

The mice that were unsuccessful in modeling were eliminated the next day after modeling (marker for successful modeling: mice with their hind paws curled up). After modeling, the mice were stroked for 3 to 5 minutes every day to ensure that the animals were familiar with the experimenter, and then the mice were placed on a metal pain measuring frame for 40 to 60 minutes of adaptation. After 3 days of acclimatization, Von Frey filaments (Aesthesio®; 0.16 g, 0.4 g, 0.6 g, 1.0 g, 1.4 g and 2.0 g) were used to test the pre-administration baseline values of the animals (Ascending testing approach). Each animal was tested twice and average values were taken, with intervals of at least 5 minutes. The animals were grouped according to the baseline values (10 animals per group). After the grouping, LX-9211 (1 and 10 mg/kg), compound 3 (1 and 10 mg/kg) or vehicle (40% PEG-400 + 10% ethanol + 15% Tween 80 + 35% physiological saline) were administered intragastrically. The mice were tested for the mechanical pain threshold (MPT) at 1, 3 and 6 hours after the administration. Time-MPT curve was plotted using GraphPad 8.3.0, and statistical analysis was performed.

Results and conclusion: The results are shown in Figure 1. At 1, 3 and 6 hours after a single administration, LX-9211 and compound 3 (both at the dose of 10 mg/kg) effectively increased the pain threshold of mice after SNL modelling. The analgesic efficacy of LX-9211 (10 mg/kg) reached the peak at 1 hour after the administration, and the efficacy gradually decreased thereafter. The analgesic efficacy of compound 3 (10 mg/kg) reached the peak at 3 hours after the administration and tended to be stable at 1 hour to 6 hours after the administration. Compound 3 had better efficacy than LX-9211 at 3 and 6 hours after the administration. The above data show that as an analgesic, compound 3 has better pharmacodynamic activity than LX-9211.

**5. Mouse brain/blood ratio test**

**5.1 Experimental animals:** Male ICR mice, 20-25 g, 9 mice/compound. Purchased from Chengdu Ddossy Experimental Animals Co., Ltd.

**5.2 Experimental design:** on the day of the experiment, 18 ICR mice were randomly grouped according to their body weights. The animals were fasted with water available for 12 to 14 h one day before the administration, and were fed 4 h after the administration;

Table 5. Administration information

| Group | Number | Administration information | | | | | |
|---|---|---|---|---|---|---|---|
| | Male | Test compounds | Administration dosage (mg/kg) | Administration concentration (mg/mL) | Administration volume (mL/kg) | Collected samples | Mode of administration |
| G1 | 9 | LX9211 | 10 | 1 | 10 | Plasma | Intragastrically |
| G2 | 9 | Compound 3 | 10 | 1 | 10 | Plasma | Intragastrically |

Notes: Vehicle for intragastric administration: 40% PEG-400+10% Ethanol+15% Tween 80+35% Saline; (Saline; Ethanol; Tween 80)

After intragastric administration, whole blood and brain tissue were collected at 0.5 h, 4 h and 24 h, and the whole blood was centrifuged to separate the plasma. The brain tissue was rinsed with cold physiological saline to remove the residual blood on the surface, drained out and then homogenized. Before analysis and detection, all samples were stored at -80°C. The samples were analyzed quantitatively by LC-MS/MS.

The test results are shown in Table 6.

Table 6. Pharmacokinetic parameters of compound in plasma of mice

| Test compounds | Mode of administration | Plasma $AUC_{0-t}$ (hr*ng/mL) | Brain tissue $AUC_{0-t}$ (hr*ng/g) | Brain/plasma ratio |
|---|---|---|---|---|
| LX9211 | i.g. (10 mg/kg) | 6643 | 110424 | 16.6 |
| Compound 3 | i.g. (10 mg/kg) | 572 | 36853 | 64.5 |
| -: not applicable. | | | | |

Conclusion: The compounds of the present invention, especially compound 3, have a high brain penetrability.

## Claims

1. A compound of formula (I), or a stereoisomer, deuterated compound, solvate, prodrug, metabolite, pharmaceutically acceptable salt or co-crystal thereof,

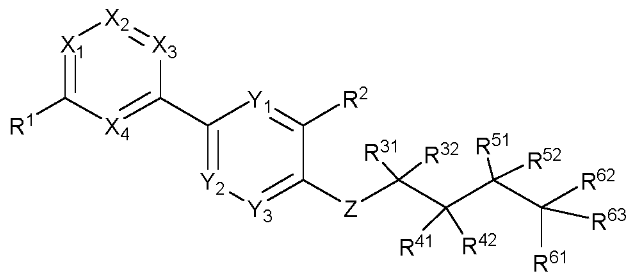

(I)

**characterised in that**

$X_1$, $X_2$, $X_3$ and $X_4$ are each independently selected from N or $CR^x$;
$Y_1$, $Y_2$ and $Y_3$ are each independently selected from N or $CR^y$;
Z is selected from $NR^z$ or O;
$R^z$ is selected from H, deuterium, halogen, $C_{1-6}$ alkyl, halo $C_{1-6}$ alkyl, or deuterated $C_{1-6}$ alkyl;
$R^x$ and $R^y$ are each independently selected from H, deuterium, halogen, amino, nitro, cyano, hydroxyl, sulphonyl, $C_{1-6}$ alkyl, halo $C_{1-6}$ alkyl, deuterated $C_{1-6}$ alkyl, $C_{1-6}$ alkoxy, halo $C_{1-6}$ alkoxy, deuterated $C_{1-6}$ alkoxy, hydroxy $C_{1-6}$ alkyl, $C_{3-6}$ cycloalkyl, or 4- to 6-membered heterocycloalkyl containing 1-3 heteroatoms selected from N, S and O, wherein the alkyl, cycloalkyl and heterocycloalkyl are optionally further substituted with 1-3 $R^A$ substituents;
$R^1$ and $R^2$ are each independently selected from H, deuterium, halogen, amino, -COOH, cyano, sulphonyl, aminoacyl, $C_{1-6}$ alkyl, halo $C_{1-6}$ alkyl, deuterated $C_{1-6}$ alkyl, $C_{1-6}$ alkoxy, halo $C_{1-6}$ alkoxy, deuterated $C_{1-6}$ alkoxy, hydroxy $C_{1-6}$ alkyl, $C_{3-6}$ cycloalkyl, 4- to 6-membered heterocycloalkyl containing 1-3 heteroatoms selected from N, S and O, $-NHC(O)C_{1-6}$ alkyl, $-NHC(O)C_{3-6}$ cycloalkyl, $-NHC(O)C_{4-6}$ heterocycloalkyl, $-NHC(O)NHC_{1-6}$ alkyl, or - $NHC(O)OC_{1-6}$ alkyl, wherein the alkyl, cycloalkyl and heterocycloalkyl are optionally further substituted with 1-3 $R^A$ substituents;
$R^{31}$ and $R^{32}$ are each independently selected from H, deuterium, halogen, cyano, hydroxyl, $C_{1-6}$ alkyl, halo $C_{1-6}$ alkyl, deuterated $C_{1-6}$ alkyl, $C_{1-6}$ alkoxy, halo $C_{1-6}$ alkoxy, deuterated $C_{1-6}$ alkoxy, or hydroxy $C_{1-6}$ alkyl; or $R^{31}$ and $R^{32}$ together with the carbon atom to which they are attached form $C_{3-6}$ cycloalkyl or 4- to 6-membered heterocycloalkyl containing 1-3 heteroatoms selected from N, S and O, wherein the cycloalkyl and heterocycloalkyl are optionally further substituted with 1-3 $R^A$ substituents;

$R^{41}$ and $R^{42}$ are each independently selected from H, deuterium, amino, $C_{1-6}$ alkyl, halogen, cyano, hydroxyl, halo $C_{1-6}$ alkyl, deuterated $C_{1-6}$ alkyl, $C_{1-6}$ alkoxy, halo $C_{1-6}$ alkoxy, -$C_{1-6}$ alkyl-$C_{3-6}$ cycloalkyl, $C_{3-6}$ cycloalkyl, 4- to 6-membered heterocycloalkyl containing 1-3 heteroatoms selected from N, S and O, deuterated $C_{1-6}$ alkoxy, or hydroxy $C_{1-6}$ alkyl;

or $R^{41}$ and $R^{42}$ together form $C_{3-6}$ cycloalkyl or 4- to 6-membered heterocycloalkyl containing 1 heteroatom selected from O or S, wherein the cycloalkyl and heterocycloalkyl are optionally further substituted with 1-3 $R^A$ substituents;

$R^{51}$ and $R^{52}$ are each independently selected from H, deuterium, amino, halogen, $C_{1-6}$ alkyl, cyano, hydroxyl, halo $C_{1-6}$ alkyl, deuterated $C_{1-6}$ alkyl, $C_{1-6}$ alkoxy, halo $C_{1-6}$ alkoxy, -$C_{1-6}$ alkyl-$C_{3-6}$ cycloalkyl, $C_{3-6}$ cycloalkyl, 4- to 6-membered heterocycloalkyl containing 1-3 heteroatoms selected from N, S and O, deuterated $C_{1-6}$ alkoxy, or hydroxy $C_{1-6}$ alkyl;

or $R^{51}$ and $R^{52}$ together with the carbon atom to which they are attached form $C_{3-6}$ cycloalkyl or 4- to 6-membered heterocycloalkyl containing 1-3 heteroatoms selected from N, S and O, wherein the cycloalkyl and heterocycloalkyl are optionally further substituted with 1-3 $R^A$ substituents;

$R^{61}$, $R^{62}$ and $R^{63}$ are each independently selected from H, deuterium, halogen, amino, cyano, hydroxyl, $C_{1-6}$ alkyl, halo $C_{1-6}$ alkyl, deuterated $C_{1-6}$ alkyl, $C_{1-6}$ alkoxy, halo $C_{1-6}$ alkoxy, -$C_{1-6}$ alkyl-$C_{3-6}$ cycloalkyl, $C_{3-6}$ cycloalkyl, 4- to 6-membered heterocycloalkyl containing 1-3 heteroatoms selected from N, S and O, deuterated $C_{1-6}$ alkoxy, or hydroxy $C_{1-6}$ alkyl;

or, $R^{31}$ and $R^{41}$, or $R^{41}$ and $R^{51}$ together with the carbon atoms to which they are each attached form $C_{3-6}$ cycloalkyl or 4- to 6-membered heterocycloalkyl containing 1-3 heteroatoms selected from N, S and O, wherein the cycloalkyl and heterocycloalkyl are optionally further substituted with 1-3 $R^A$ substituents;

or, $R^{41}$ and $R^{61}$, or $R^z$ and $R^{41}$ together with the atoms to which they are each attached form $C_{4-6}$ cycloalkyl or 4- to 6-membered heterocycloalkyl containing 1-3 heteroatoms selected from N, S and O, wherein the cycloalkyl and heterocycloalkyl are optionally further substituted with 1-3 $R^A$ substituents;

or, $R^{51}$ and $R^{61}$, or $R^{61}$ and $R^{62}$ together with the carbon atom(s) to which they are each attached form $C_{3-6}$ cycloalkyl, 4- to 6-membered heterocycloalkyl containing 1-3 heteroatoms selected from N, S and O, or a double bond, wherein the cycloalkyl and heterocycloalkyl are optionally further substituted with 1-3 $R^A$ substituents;

or, $R^{61}$, $R^{62}$ and $R^{63}$ together with the carbon atom to which they are attached form $C_{5-10}$ bridged ring or $C_{5-11}$ spiro ring, wherein the bridged ring and spiro ring are optionally further substituted with 1-3 $R^A$ substituents;

$R^A$ is selected from deuterium, halogen, amino, cyano, hydroxyl, $C_{1-6}$ alkyl, halo $C_{1-6}$ alkyl, deuterated $C_{1-6}$ alkyl, $C_{1-6}$ alkoxy, halo $C_{1-6}$ alkoxy, deuterated $C_{1-6}$ alkoxy, or hydroxy $C_{1-6}$ alkyl,

provided that when Z is selected from O,

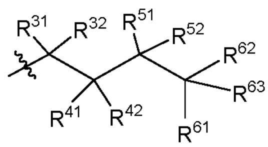

does not form the following structures:

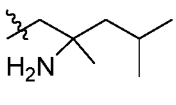 and 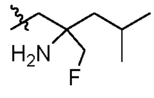 .

2. The compound, or the stereoisomer, deuterated compound, solvate, prodrug, metabolite, pharmaceutically acceptable salt or co-crystal thereof according to claim 1, having a structure of formula (Ia):

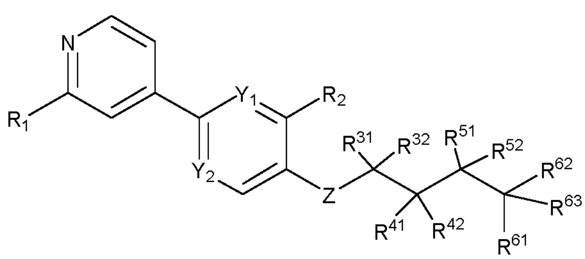 (Ia).

**3.** The compound, or the stereoisomer, deuterated compound, solvate, prodrug, metabolite, pharmaceutically acceptable salt or co-crystal thereof according to claims 1-2, **characterised in that**

$R^1$ is selected from sulphonyl, aminoacyl, halo $C_{1-3}$ alkyl, -NHC(O)$C_{1-4}$ alkyl, -NHC(O)$C_{3-6}$ cycloalkyl, -NHC(O)$C_{4-6}$ heterocycloalkyl, -NHC(O)NH$C_{1-4}$ alkyl, or -NHC(O)O$C_{1-4}$ alkyl, wherein the alkyl, cycloalkyl and heterocycloalkyl are optionally further substituted with 1-3 $R^A$ substituents;
$R^2$ is selected from cyano, $C_{1-3}$ alkyl, halo $C_{1-3}$ alkyl, or deuterated $C_{1-3}$ alkyl;
$R^A$ is selected from deuterium, F, Cl, amino, cyano, hydroxyl, $C_{1-3}$ alkyl, halo $C_{1-3}$ alkyl, deuterated $C_{1-3}$ alkyl, $C_{1-3}$ alkoxy, halo $C_{1-3}$ alkoxy, deuterated $C_{1-3}$ alkoxy, or hydroxy $C_{1-3}$ alkyl.

**4.** The compound, or the stereoisomer, deuterated compound, solvate, prodrug, metabolite, pharmaceutically acceptable salt or co-crystal thereof according to claim 1, having a structure of formula (II):

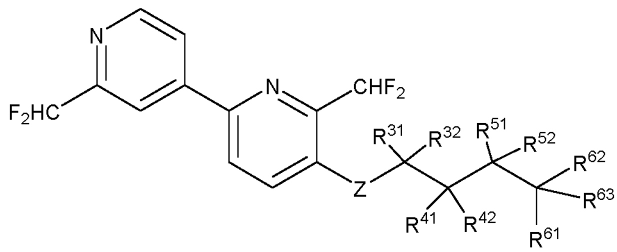
(II).

**5.** The compound, or the stereoisomer, deuterated compound, solvate, prodrug, metabolite, pharmaceutically acceptable salt or co-crystal thereof according to any one of claims 1-4, **characterised in that**

Z is selected from $NR^z$ or O;
$R^z$ is selected from H, deuterium or $C_{1-4}$ alkyl;
$R^{31}$ and $R^{32}$ are each independently selected from H, deuterium, F, Cl, cyano, hydroxyl, $C_{1-4}$ alkyl, halo $C_{1-4}$ alkyl, deuterated $C_{1-4}$ alkyl, $C_{1-4}$ alkoxy, halo $C_{1-4}$ alkoxy, deuterated $C_{1-4}$ alkoxy, or hydroxy $C_{1-4}$ alkyl; or $R^{31}$ and $R^{32}$ together with the carbon atom to which they are attached form 3-membered cycloalkyl, 4-membered cycloalkyl, 5-membered cycloalkyl, or 4- or 5-membered heterocycloalkyl containing 1-3 heteroatoms selected from N, S and O, wherein the cycloalkyl and heterocycloalkyl are optionally further substituted with 1, 2 or 3 substituents selected from $R^A$;
$R^{41}$ and $R^{42}$ are each independently selected from H, deuterium, amino, $C_{1-4}$ alkyl, halogen, cyano, hydroxyl, halo $C_{1-4}$ alkyl, deuterated $C_{1-4}$ alkyl, $C_{1-4}$ alkoxy, halo $C_{1-4}$ alkoxy, -$C_{1-4}$ alkyl-3-membered cycloalkyl, -$C_{1-4}$ alkyl-4-membered cycloalkyl, -$C_{1-4}$ alkyl-5-membered cycloalkyl, 3-membered cycloalkyl, 4-membered cycloalkyl, 5-membered cycloalkyl, 4-, 5- or 6-membered heterocycloalkyl containing 1-3 heteroatoms selected from N, S and O, deuterated $C_{1-4}$ alkoxy, or hydroxy $C_{1-4}$ alkyl; or $R^{41}$ and $R^{42}$ together form 3-membered cycloalkyl, 4-membered cycloalkyl, 5-membered cycloalkyl, 4- or 5-membered heterocycloalkyl containing 1-3 heteroatoms selected from N, S and O, wherein the cycloalkyl and heterocycloalkyl are optionally further substituted with 1, 2 or 3 substituents selected from $R^A$;
$R^{51}$ and $R^{52}$ are each independently selected from H, deuterium, amino, halogen, $C_{1-4}$ alkyl, cyano, hydroxyl, halo $C_{1-4}$ alkyl, deuterated $C_{1-4}$ alkyl, $C_{1-4}$ alkoxy, halo $C_{1-4}$ alkoxy, -$C_{1-4}$ alkyl-3-membered cycloalkyl, -$C_{1-4}$ alkyl-4-membered cycloalkyl, -$C_{1-4}$ alkyl-5-membered cycloalkyl, 3-membered cycloalkyl, 4-membered cycloalkyl, 5-membered cycloalkyl, 4-, 5- or 6-membered heterocycloalkyl containing 1-3 heteroatoms selected from N, S and O, deuterated $C_{1-4}$ alkoxy, or hydroxy $C_{1-4}$ alkyl; or $R^{51}$ and $R^{52}$ together with the carbon atom to which they are attached form 3-membered cycloalkyl, 4-membered cycloalkyl, 5-membered cycloalkyl, or 4-, 5- or 6-membered heterocycloalkyl containing 1-3 heteroatoms selected from N, S and O, wherein the cycloalkyl and heterocycloalkyl are optionally further substituted with 1, 2 or 3 substituents selected from $R^A$;
$R^{61}$, $R^{62}$ and $R^{63}$ are each independently selected from H, deuterium, halogen, amino, cyano, hydroxyl, $C_{1-4}$ alkyl, halo $C_{1-4}$ alkyl, deuterated $C_{1-4}$ alkyl, $C_{1-4}$ alkoxy, halo $C_{1-4}$ alkoxy, -$C_{1-4}$ alkyl-3-membered cycloalkyl, -$C_{1-4}$ alkyl-4-membered cycloalkyl, -$C_{1-4}$ alkyl-5-membered cycloalkyl, 3-membered cycloalkyl, 4-membered cycloalkyl, 5-membered cycloalkyl, 4-, 5- or 6-membered heterocycloalkyl containing 1-3 heteroatoms selected from N, S and O, deuterated $C_{1-4}$ alkoxy, or hydroxy $C_{1-4}$ alkyl;
or, $R^{31}$ and $R^{41}$, or $R^{41}$ and $R^{51}$ together with the carbon atoms to which they are each attached form 3-membered cycloalkyl, 4-membered cycloalkyl, 5-membered cycloalkyl, or 4-, 5- or 6-membered heterocycloalkyl containing 1-3 heteroatoms selected from N, S and O, wherein the cycloalkyl and heterocycloalkyl are optionally further

substituted with 1, 2 or 3 substituents selected from $R^A$;

or, $R^{41}$ and $R^{61}$ together with the carbon atom(s) to which they are each attached form 4-membered cycloalkyl, 5-membered cycloalkyl, 6-membered cycloalkyl, or 4-, 5- or 6-heterocycloalkyl containing 1-3 heteroatoms selected from N, S and O, wherein the cycloalkyl and heterocycloalkyl are optionally further substituted with 1, 2 or 3 substituents selected from $R^A$;

or, $R^z$ and $R^{41}$ together with the carbon atom(s) to which they are each attached form 4-, 5- or 6-membered heterocycloalkyl containing 1-3 heteroatoms selected from N, S and O, wherein the heterocycloalkyl is optionally further substituted with 1, 2 or 3 substituents selected from $R^A$;

or, $R^{51}$ and $R^{61}$, or $R^{61}$ and $R^{62}$ together with the carbon atom(s) to which they are each attached form 3-membered cycloalkyl, 4-membered cycloalkyl, 5-membered cycloalkyl, 4-, 5- or 6-membered heterocycloalkyl containing 1-3 heteroatoms selected from N, S and O, or a double bond, wherein the cycloalkyl and heterocycloalkyl are optionally further substituted with 1, 2 or 3 $R^A$ substituents;

or, $R^{61}$, $R^{62}$ and $R^{63}$ together with the carbon atom to which they are attached form 5-membered bicyclic bridged ring, 6-membered bicyclic bridged ring, 7-membered bicyclic bridged ring, 8-membered bicyclic bridged ring, 5-membered spiro ring, 6-membered spiro ring, 7-membered spiro ring, 8-membered spiro ring, 9-membered spiro ring, or 10-membered spiro ring, wherein the bridged ring and spiro ring are optionally further substituted with 1, 2 or 3 substituents selected from $R^A$;

$R^A$ is selected from deuterium, F, Cl, amino, cyano, hydroxyl, $C_{1-4}$ alkyl, halo $C_{1-4}$ alkyl, deuterated $C_{1-4}$ alkyl, $C_{1-4}$ alkoxy, halo $C_{1-4}$ alkoxy, deuterated $C_{1-4}$ alkoxy, or hydroxy $C_{1-4}$alkyl.

6. The compound, or the stereoisomer, deuterated compound, solvate, prodrug, metabolite, pharmaceutically acceptable salt or co-crystal thereof according to any one of claims 1-4, **characterised in that**

Z is selected from $NR^z$ or O;

$R^z$ is selected from H, deuterium, methyl, ethyl, n-propyl or isopropyl;

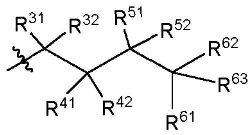

is selected from the following groups:

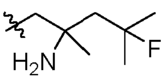 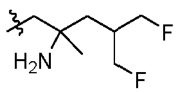 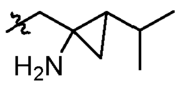 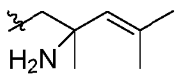 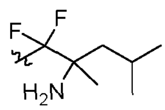

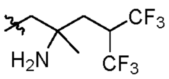 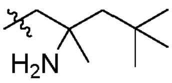 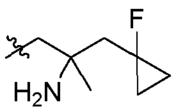 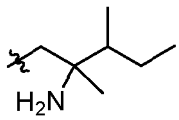 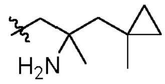

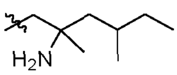 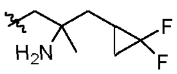 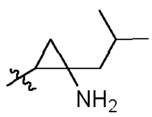 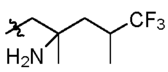 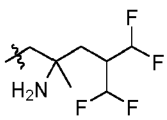

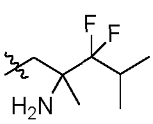 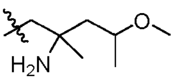 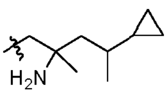 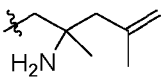 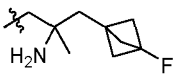

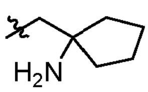 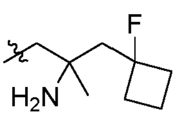 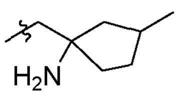 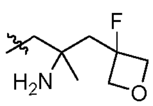 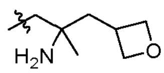

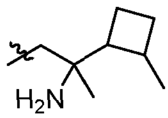 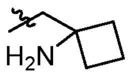 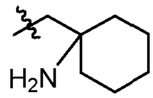 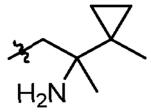 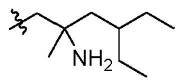

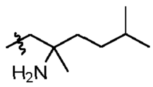 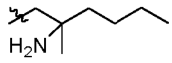 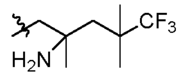 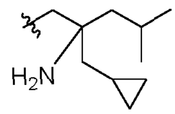 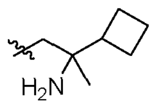

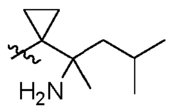 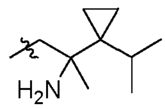 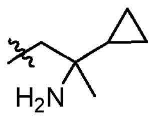 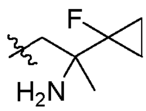 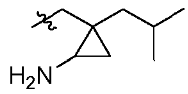

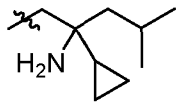 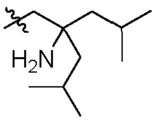 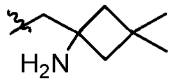 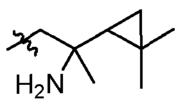 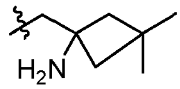

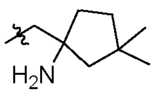 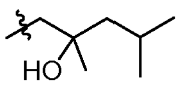 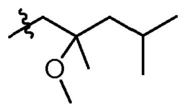 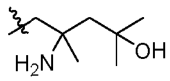 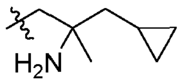

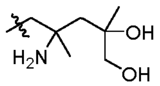

or

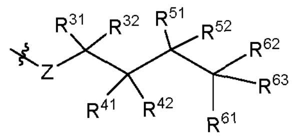,

is selected from

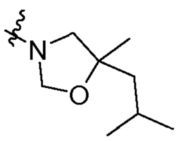.

**7.** The compound, or the stereoisomer, deuterated compound, solvate, prodrug, metabolite, pharmaceutically acceptable salt or co-crystal thereof according to claim 1, having a structure of formula (Ib):

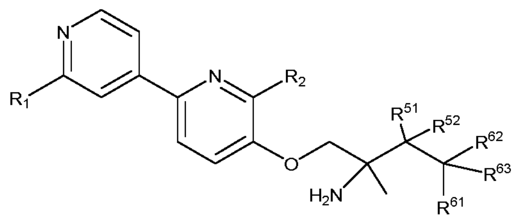 (Ib)

$R^1$ is selected from halo $C_{1-3}$ alkyl, -NHC(O)$C_{1-4}$ alkyl, -NHC(O)$C_{3-6}$ cycloalkyl, -NHC(O)$C_{4-6}$ heterocycloalkyl,

-NHC(O)NHC$_{1-4}$ alkyl, or - NHC(O)OC$_{1-4}$ alkyl, wherein the alkyl, cycloalkyl and heterocycloalkyl are optionally further substituted with 1-3 substituents selected from deuterium, F, Cl, amino, cyano, or hydroxyl;
$R^2$ is selected from cyano, halo C$_{1-3}$ alkyl or deuterated C$_{1-3}$ alkyl;
$R^{51}$ and $R^{52}$ are each independently selected from H or deuterium;
$R^{61}$ is independently selected from H, deuterium, halogen, amino, cyano, hydroxyl, C$_{1-6}$ alkyl, halo C$_{1-6}$ alkyl, deuterated C$_{1-6}$ alkyl, C$_{1-6}$ alkoxy, halo C$_{1-6}$ alkoxy, -C$_{1-6}$ alkyl-C$_{3-6}$ cycloalkyl, C$_{3-6}$ cycloalkyl, 4- to 6-membered heterocycloalkyl containing 1-3 heteroatoms selected from N, S and O, deuterated C$_{1-6}$ alkoxy or hydroxy C$_{1-6}$ alkyl;
$R^{62}$ and $R^{63}$ are each independently selected from halogen, amino, cyano, hydroxyl, C$_{1-6}$ alkyl, halo C$_{1-6}$ alkyl, deuterated C$_{1-6}$ alkyl, C$_{1-6}$ alkoxy, halo C$_{1-6}$ alkoxy, -C$_{1-6}$ alkyl-C$_{3-6}$ cycloalkyl, C$_{3-6}$ cycloalkyl, 4- to 6-membered heterocycloalkyl containing 1-3 heteroatoms selected from N, S and O, deuterated C$_{1-6}$ alkoxy, or hydroxy C$_{1-6}$ alkyl;
or, $R^{61}$ and $R^{62}$ together with the carbon atom(s) to which they are each attached form C$_{3-6}$ cycloalkyl, 4- to 6-membered heterocycloalkyl containing 1-3 heteroatoms selected from N, S and O, or a double bond, wherein the cycloalkyl and heterocycloalkyl are optionally further substituted with 1-3 substituents selected from deuterium, F, Cl, amino, cyano, or hydroxyl.

**8.** The compound, or the stereoisomer, deuterated compound, solvate, prodrug, metabolite, pharmaceutically acceptable salt or co-crystal thereof according to claim 7, **characterised in that**

$R^1$ is selected from halo C$_{1-3}$ alkyl, -NHC(O)C$_{1-4}$ alkyl, -NHC(O)C$_{3-6}$ cycloalkyl, or -NHC(O)OC$_{1-4}$ alkyl, wherein the alkyl, cycloalkyl and heterocycloalkyl are optionally further substituted with 1-3 substituents selected from deuterium, F, Cl, amino, cyano, or hydroxyl;
$R^2$ is selected from cyano or halo C$_{1-3}$ alkyl;
$R^{51}$ and $R^{52}$ are each independently selected from H or deuterium;
$R^{61}$ is independently selected from H, deuterium, halogen, amino, cyano, hydroxyl, C$_{1-3}$ alkyl, or hydroxy C$_{1-3}$ alkyl;
$R^{62}$ and $R^{63}$ are each independently selected from halogen, amino, cyano, hydroxyl, C$_{1-3}$ alkyl, halo C$_{1-3}$ alkyl, deuterated C$_{1-3}$ alkyl, or hydroxy C$_{1-3}$ alkyl;
or, $R^{61}$ and $R^{62}$ together with the carbon atom(s) to which they are each attached form C$_{3-6}$ cycloalkyl or a double bond, wherein the cycloalkyl is optionally further substituted with 1-3 substituents selected from deuterium, F, Cl, amino, cyano, or hydroxyl.

**9.** The compound, or the stereoisomer, deuterated compound, solvate, prodrug, metabolite, pharmaceutically acceptable salt or co-crystal thereof according to claim 1, **characterised in that** the compound is selected from the following structures:

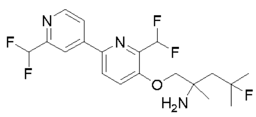 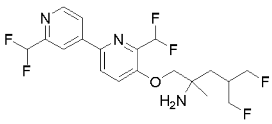 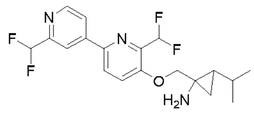 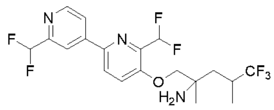

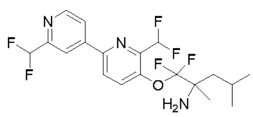 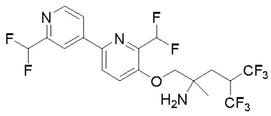 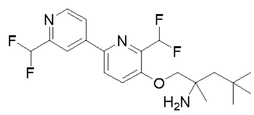 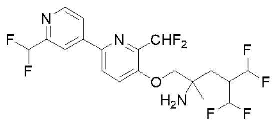

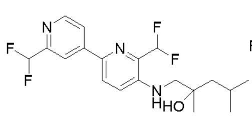 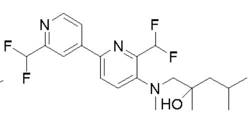 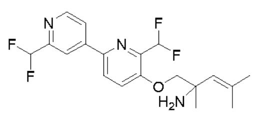 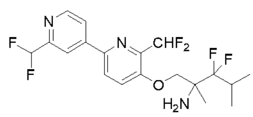

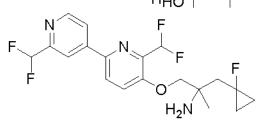 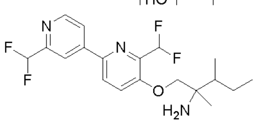 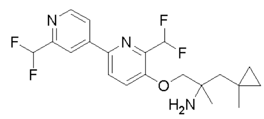 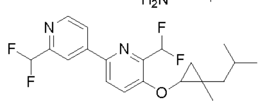

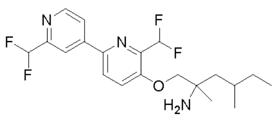
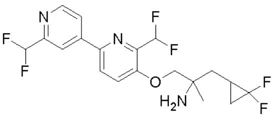
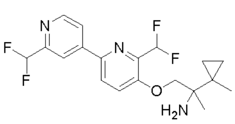
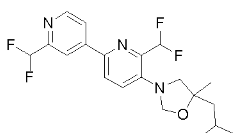
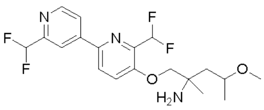
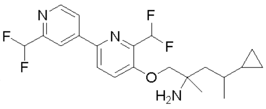
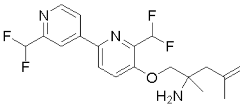
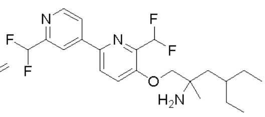
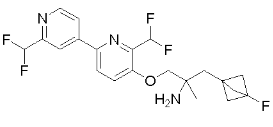
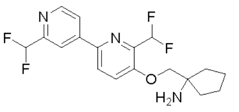
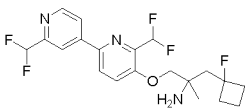
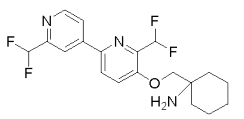
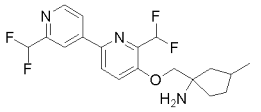
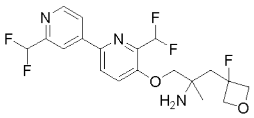
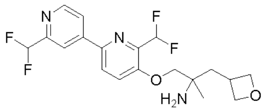
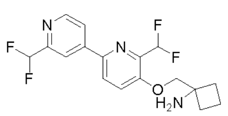
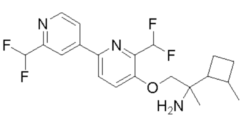
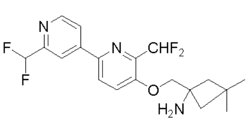
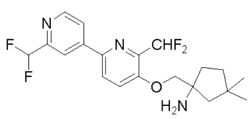
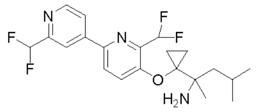
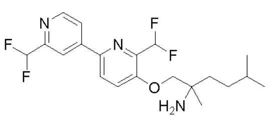
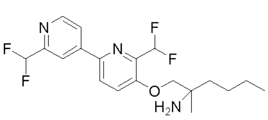
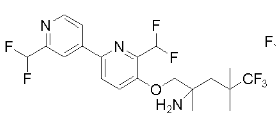
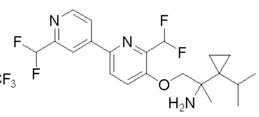
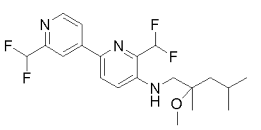
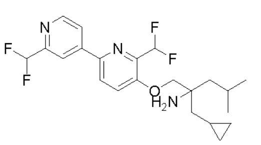
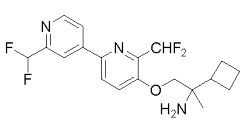
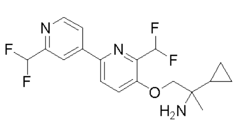
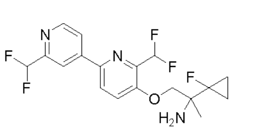
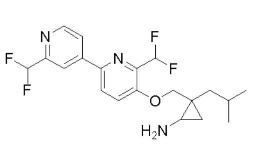
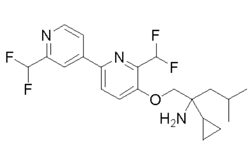
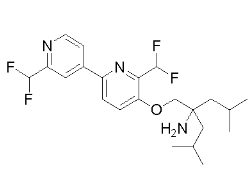
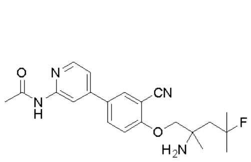
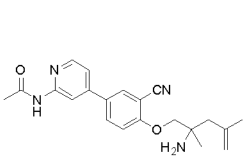
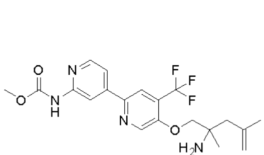
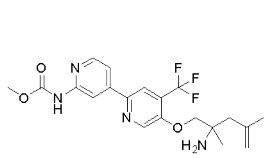
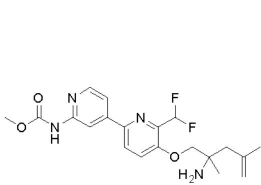
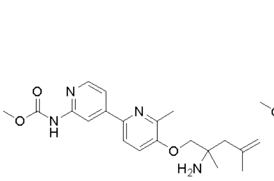
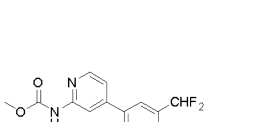
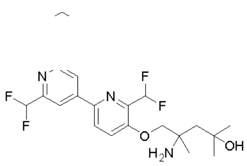

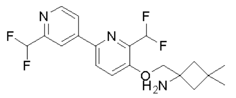
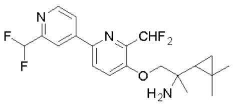

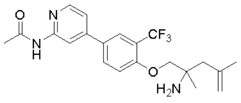
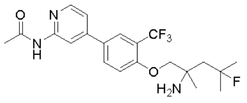
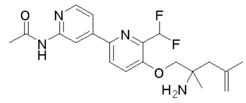
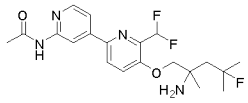

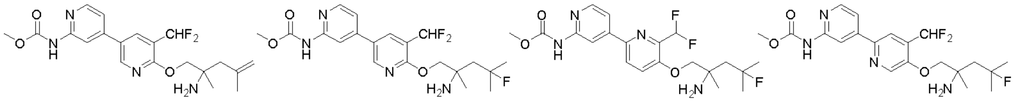

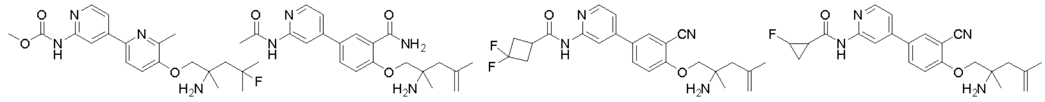

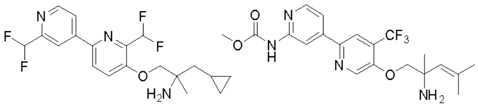
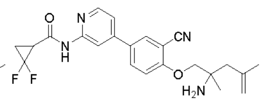
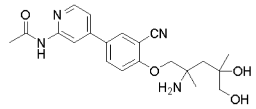

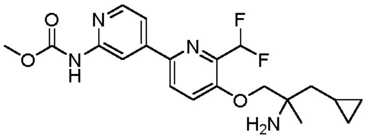.

10. The compound, or the stereoisomer, deuterated compound, solvate, prodrug, metabolite, pharmaceutically acceptable salt or co-crystal thereof according to claim 1, **characterised in that** the compound is selected from the following structures:

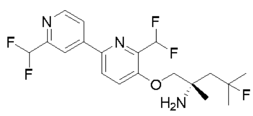
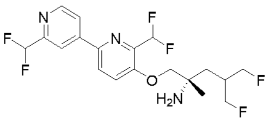
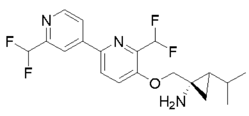
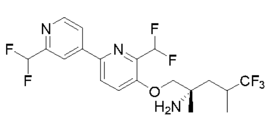

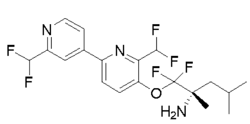
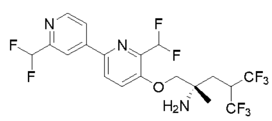
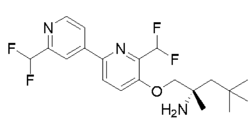
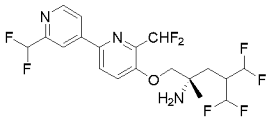

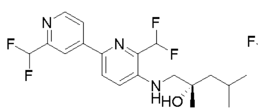
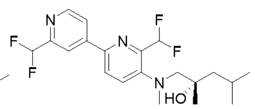
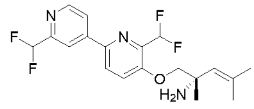
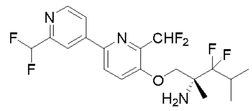

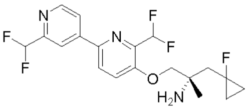
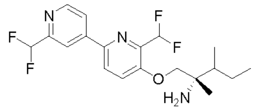
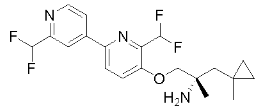
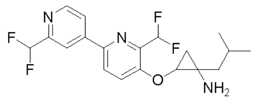

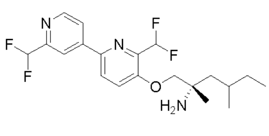
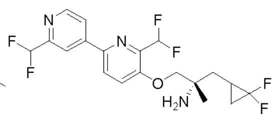
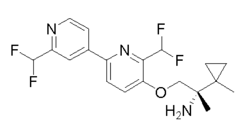
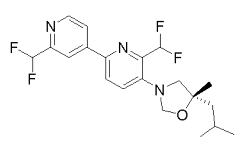

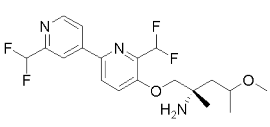 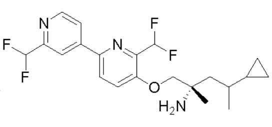 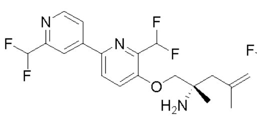 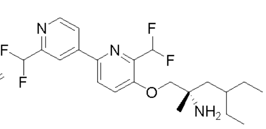

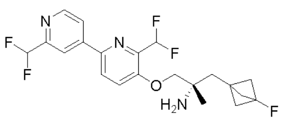 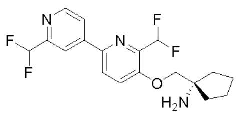 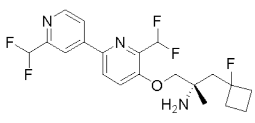 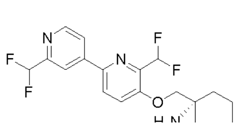

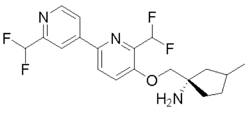 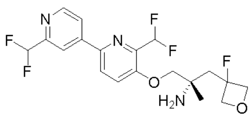 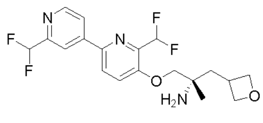 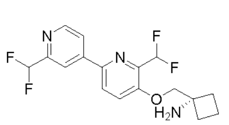

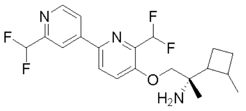 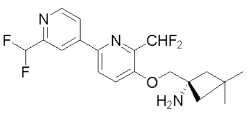 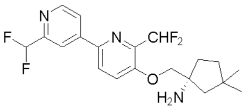 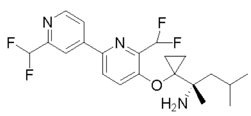

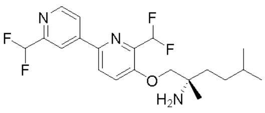 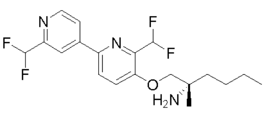 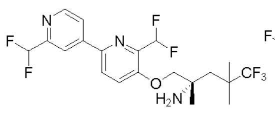 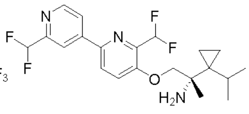

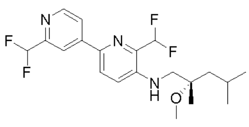 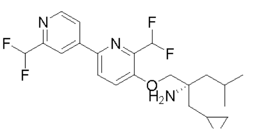 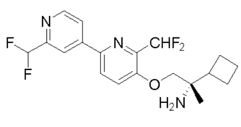 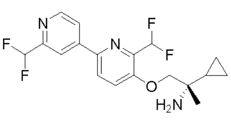

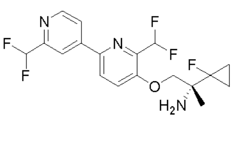 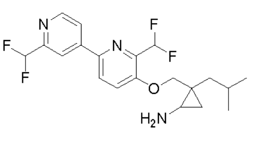 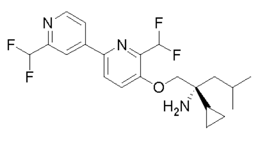 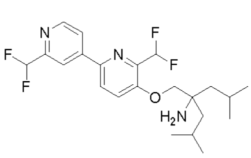

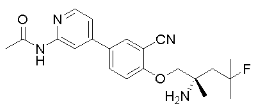 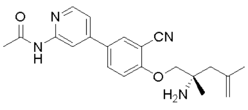 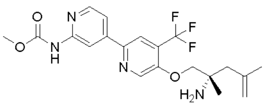 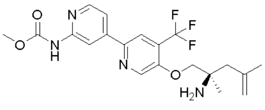

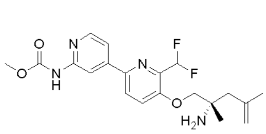 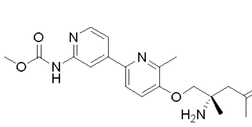 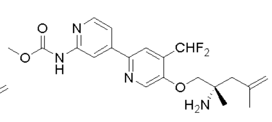 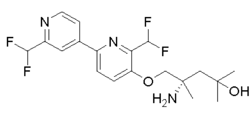

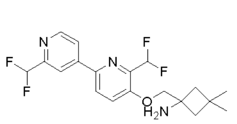 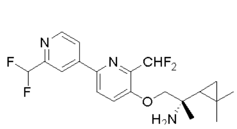

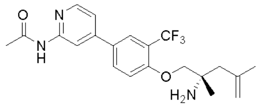 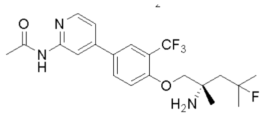 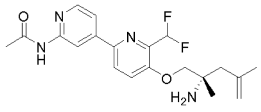 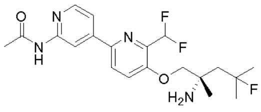

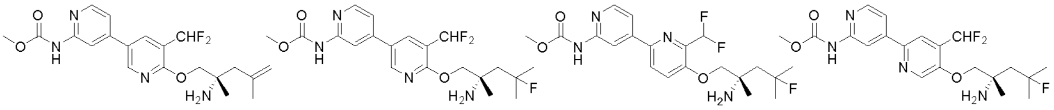

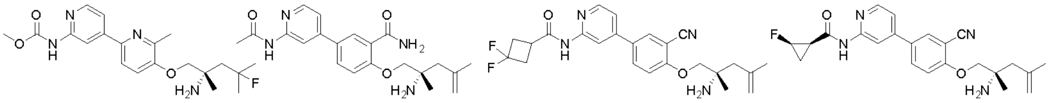

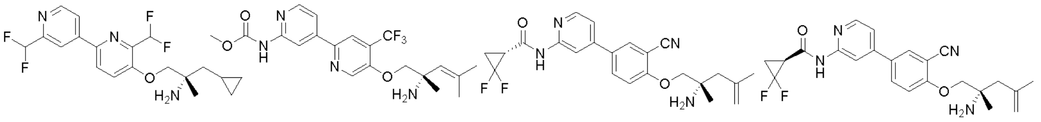

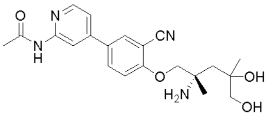 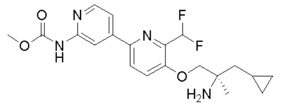 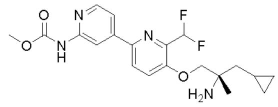.

**11.** A pharmaceutical composition, comprising the compound, or the stereoisomer, deuterated compound, solvate, prodrug, metabolite, pharmaceutically acceptable salt or co-crystal thereof according to any one of claims 1-10, and a pharmaceutically acceptable carrier and/or excipient.

**12.** The pharmaceutical composition according to claim 11, comprising 1-1500 mg of the compound, or the stereoisomer, deuterated compound, solvate, pharmaceutically acceptable salt or co-crystal thereof according to any one of claims 1-10, and a carrier and/or excipient.

**13.** Use of the compound, or the stereoisomer, deuterated compound, solvate, prodrug, metabolite, pharmaceutically acceptable salt or co-crystal thereof according to any one of claims 1-10, or the composition according to claims 11-12 in the preparation of a drug for treating an AAK1-mediated disease.

**14.** The use according to claim 13, **characterised in that** the AAK1-mediated disease is diabetic neuropathic pain or post-herpetic neuralgia.

**15.** A method for treating a disease in a mammal, **characterised in that** the method comprises administering to a subject a therapeutically effective amount of the compound, or the stereoisomer, deuterated compound, solvate, pharmaceutically acceptable salt or co-crystal thereof according to any one of claims 1-10, or the composition according to claim 11 or 12, wherein the therapeutically effective amount is preferably 1-1500 mg, and the disease is preferably diabetic neuropathic pain or post-herpetic neuralgia.

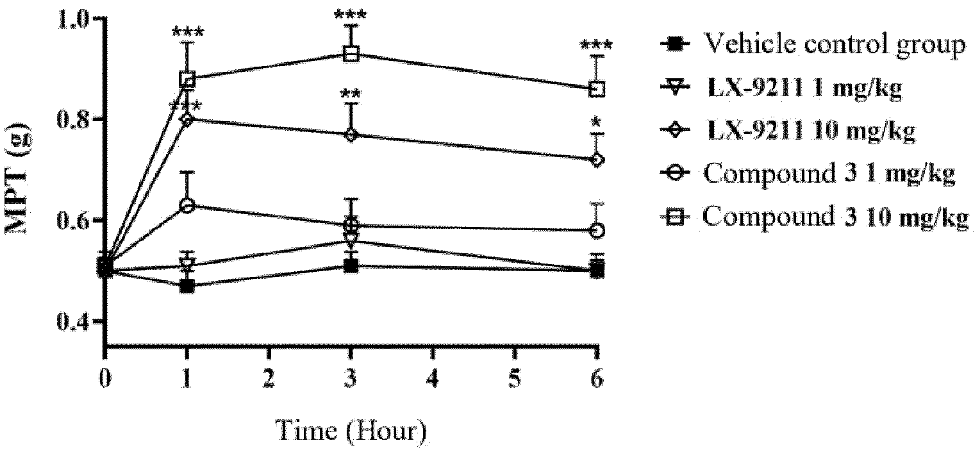
1.0
0.8
0.6
0.4
MPT (g)
***
***
***
***
**
*
0
1
2
3
4
5
6
Time (Hour)
Vehicle control group
LX-9211 1 mg/kg
LX-9211 10 mg/kg
Compound 3 1 mg/kg
Compound 3 10 mg/kg

*FIG. 1*

**INTERNATIONAL SEARCH REPORT**

International application No. **PCT/CN2022/105793**

**A. CLASSIFICATION OF SUBJECT MATTER**

C07D 401/04(2006.01)i； A61K 31/444(2006.01)i； A61P 25/00(2006.01)i

According to International Patent Classification (IPC) or to both national classification and IPC

**B. FIELDS SEARCHED**

Minimum documentation searched (classification system followed by classification symbols)

C07D401/-; A61K31/-; A61P25/-

Documentation searched other than minimum documentation to the extent that such documents are included in the fields searched

Electronic data base consulted during the international search (name of data base and, where practicable, search terms used)

CNPAT, CNKI, EPODOC, WPI, REGISTRY(STN), CAPLUS(STN): 连接蛋白相关激酶, 丝氨酸, 苏氨酸, 激酶, AAK1, STN 结构式检索, STN structural formula search

**C. DOCUMENTS CONSIDERED TO BE RELEVANT**

| Category* | Citation of document, with indication, where appropriate, of the relevant passages | Relevant to claim No. |
|---|---|---|
| X | CN 108368084 A (BRISTOL-MYERS SQUIBB CO.) 03 August 2018 (2018-08-03) description, paragraphs 0007-0030, 0052, and 0054, and embodiments 3, 5, 7, 11, 177, and 273 | 1-15 |
| X | CN 108290843 A (BRISTOL-MYERS SQUIBB CO.) 17 July 2018 (2018-07-17) claims 1, 7, and 9-12, and description, paragraphs 0006-0037, and embodiment 277 | 1-15 |
| X | CN 106458994 A (BRISTOL-MYERS SQUIBB CO.) 22 February 2017 (2017-02-22) claims 1 and 10-15, and description, embodiments 3, 5, 7, 11, and 177 | 1-15 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **02 September 2022** | **23 September 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/105793**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **15**
because they relate to subject matter not required to be searched by this Authority, namely:

[1] Claim 15 relates to a method for treating a living human or animal body as defined in PCT Rule 39.1(4). The present search report was conducted on the basis that claim 15 is a pharmaceutical use claim.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2022/105793**

| Patent document cited in search report | Publication date (day/month/year) | Patent family member(s) | Publication date (day/month/year) |
|---|---|---|---|
| CN 108368084 A | 03 August 2018 | EP 3356340 A1 | 08 August 2018 |
| | | ES 2765730 T3 | 10 June 2020 |
| | | KR 20180056765 A | 29 May 2018 |
| | | WO 2017059085 A1 | 06 April 2017 |
| | | JP 2018537408 A | 20 December 2018 |
| | | US 2018346440 A1 | 06 December 2018 |
| | | CN 112142655 A | 29 December 2020 |
| CN 108290843 A | 17 July 2018 | US 2019040080 A1 | 07 February 2019 |
| | | EP 3356330 A1 | 08 August 2018 |
| | | ES 2767776 T3 | 18 June 2020 |
| | | JP 2018529731 A | 11 October 2018 |
| | | KR 20180056764 A | 29 May 2018 |
| | | WO 2017059080 A1 | 06 April 2017 |
| CN 106458994 A | 22 February 2017 | EA 201691916 A1 | 28 February 2017 |
| | | CA 2944466 A1 | 08 October 2015 |
| | | HU E041457 T2 | 28 May 2019 |
| | | AU 2015240869 A1 | 17 November 2016 |
| | | IL 248086 D0 | 30 November 2016 |
| | | WO 2015153720 A1 | 08 October 2015 |
| | | CY 1120988 T1 | 11 December 2019 |
| | | ES 2700549 T3 | 18 February 2019 |
| | | PT 3126351 T | 04 December 2018 |
| | | NZ 725814 A | 25 June 2021 |
| | | SG 11201608195 Q A | 28 October 2016 |
| | | US 2019367508 A1 | 05 December 2019 |
| | | HR P20181899 T1 | 11 January 2019 |
| | | DK 3126351 T3 | 14 January 2019 |
| | | PL 3126351 T3 | 29 March 2019 |
| | | US 2018134704 A1 | 17 May 2018 |
| | | RS 58062 B1 | 28 February 2019 |
| | | US 2017183340 A1 | 29 June 2017 |
| | | PE 20170004 A1 | 04 March 2017 |
| | | CL 2016002502 A1 | 23 June 2017 |
| | | SI 3126351 T1 | 30 November 2018 |
| | | MX 2016012829 A | 05 January 2017 |
| | | US 2019048006 A1 | 14 February 2019 |
| | | US 2021277001 A1 | 09 September 2021 |
| | | EP 3126351 A1 | 08 February 2017 |
| | | KR 20160132491 A | 18 November 2016 |
| | | JP 2017511329 A | 20 April 2017 |
| | | LT 3126351 T | 26 November 2018 |
| | | ZA 201606767 B | 30 May 2018 |
| | | US 2020317671 A1 | 08 October 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017059085 A
- WO 2017059080 A
- WO 2015153720 A
- WO 2010038465 A


### Non-patent literature cited in the description


- **HENDERSON ; CONNER.** *Mol. Biol. Cell,* 2007, vol. 18, 2698-2706
- **CONNER et al.** *Traffic,* 2003, vol. 4, 885-890
- **JACKSON et al.** *J. Cell. Biol.,* 2003, vol. 163, 231-236
- **RICOTTA et al.** *J. Cell Bio.,* 2002, vol. 156, 791-795
- **CONNER ; SCHMID, J.** *Cell Bio.,* 2002, vol. 156, 921-929
- **MOTELY et al.** *Mol. Biol. Cell.,* 2006, vol. 17, 5298-5308
- **KUAI et al.** *Chemistry and Biology,* 2011, vol. 18, 891-906
- **BUONANNO.** *Brain Res. Bull.,* 2010, vol. 83, 122-131
- **GREENWOOD et al.** *Am. J. Psychiatry,* 2011, vol. 168, 930-946
- **JAARO-PELED et al.** *Schizophrenia Bulletin,* 2010, vol. 36, 301-313
- **WEN et al.** *Proc. Natl. Acad. Sci. USA.,* 2010, vol. 107, 1211-1216
- **LATOURELLE et al.** *BMC Med. Genet.,* 2009, vol. 10, 98
- Synthetic Organic Chemistry. John Wiley & Sons, Inc,
- **S. R. SANDLER et al.** Organic Functional Group Preparations. Academic Press, 1983
- **H. O. HOUSE ; W. A. BENJAMIN.** Modern Synthetic Reactions. Inc. Menlo Park, 1972
- **T. L. GILCHRIST.** Heterocyclic Chemistry. John Wiley & Sons, 1992
- **J. MARCH.** Advanced Organic Chemistry: Reactions, Mechanisms and Structure. Wiley-Interscience, 1992
- **FUHRHOP, J. ; PENZLIN G.** Organic Synthesis: Concepts, Methods, Starting Materials. John Wiley & Sons, 1994
- **HOFFMAN, R.V.** Organic Chemistry, An Intermediate Text. Oxford University Press, 1996
- **LAROCK, R. C.** Comprehensive Organic Transformations: A Guide to Functional Group Preparations. Wiley-VCH, 1999
- **MARCH, J.** Advanced Organic Chemistry: Reactions, Mechanisms, and Structure. John Wiley & Sons, 1992
- Modern Carbonyl Chemistry. Wiley-VCH, 2000
- **PATAI, S.** Patai's 1992 Guide to the Chemistry of Functional Groups. Interscience, 1992
- **SOLOMONS, T. W. G.** Organic Chemistry. John Wiley & Sons, 2000
- **STOWELL, J.C.** Intermediate Organic Chemistry. Wiley-Interscience, 1993
- Industrial Organic Chemicals: Starting Materials and Intermediates: An Ullmann's Encyclopedia. John Wiley & Sons, 1999, vol. 8
- Organic Reactions. John Wiley & Sons, 1942, vol. 55
- Chemistry of Functional Groups. John Wiley & Sons, vol. 73
- **P. H. STAHL ; C. G. WERMUTH.** Handbook of Pharmaceutical Salts. Verlag Helvetica Chimica Acta, 2002
- *CHEMICAL ABSTRACTS,* 1310584-14-5
- *CHEMICAL ABSTRACTS,* 564483-18-7